# EUROPEAN PATENT APPLICATION

(11) **EP 1 990 055 A2**
(43) Date of publication of application: **12.11.2008**
(21) Application number: 08075679.4
(22) Date of filing: 29.11.2002
(51) Int. Cl.: A61K 35/26, A61K 35/28, A61K 35/14, A61K 31/37, A61K 41/00

(54) **Methods for pretreating a subject with extracorporeal photopheresis and/or apoptotic cells**

(30) Priority: 29.11.2001 US 333746 P
(62) Divisional of application: 02786824.9
(71) Applicant: THERAKOS, INC., Exton, PA 19311 (US)
(72) Inventor: Peritt, David, Bal Cynwyd, PA 19004 (US); Harriman, Gregory, Paoli, PA 19301 (US)
(74) Representative: Mercer, Christopher Paul

(57) **Abstract**

The present invention relates to methods for treating a subject predisposed to an autoimmune disease with extracorporeal photopheresis or an effective amount of apoptotic cells before the clinical manifestation of a symptom associated with the autoimmune disease. The present invention alsorelates to methods for treating a subject predisposed to an atopic disease with extracorporeal photopheresis or an effective amount of apoptotic cells before the clinical manitfestation of a symptom associated with the atopic disease. The present invention further relates to methods for treating a transplant donor and/or a transplant recipient, or an implant recipient with extracorporeal photopheresis or an effective amount of apoptotic cells prior to the transplant or implantation procedure.

## Description

### CROSS REFERENCE TO RELATED APPLICATIONS

The present application is related to and claims, under 35 U.S.C. § 119(e), the benefit of U.S. Provisional Patent Application Serial No. 60/333,746, filed 29 November 2001, which is entirely expressly incorporated herein by reference.

### FIELD OF THE INVENTION

The present invention relates to methods for treating a subject predisposed to an autoimmune disease with extracorporeal photopheresis or an effective amount of apoptotic cells before the clinical manitfestation of a symptom associated with the autoimmune disease. The present invention alsorelates to methods for treating a subject predisposed to an atopic disease with extracorporeal photopheresis or an effective amount of apoptotic cells before the clinical manitfestation of a symptom associated with the atopic disease. The present invention further relates to methods for treating a transplant donor and/or a transplant recipient, or an implant recipient with extracorporeal photopheresis or an effective amount of apoptotic cells prior to the transplant or implant procedure.

### BACKGROUND OF THE INVENTION

Light irradiation or phototherapy has been widely used in the chemical and biological sciences for many years. Ultraviolet (UV) light irradiation of blood was used in the 1930's, 40's, and 50's for the treatment of many conditions. These conditions included bacterial diseases such as septicemias, pneumonias, peritonitis, wound infection, viral infections including acute and chronic hepatitis, poliomyelitis, measles, mumps, and mononucleosis. Phototherapy or light irradiation also includes the processes of exposing photoactivatable or photosensitizable targets, such as cells, blood products, bodily fluids, chemical molecules, tissues, viruses, and drug compounds, to light energy, which induces an alteration in or to the targets. In recent years, the applications of phototherapy are increasing in the medical field. These applications include the inactivation of viruses contaminating blood or blood products, the preventive treatment of platelet-concentrate infusion-induced all immunization reactions, and the treatment of both autoimmune and T-cell mediated diseases because numerous human disease states, particularly those relating to biological fluids such as blood, respond favorably to treatment by visible or UV light irradiation.

Irradiation applications may also include the irradiation sterilization of fluids that contain undesirable microorganisms, such as bacteria or viruses. Light irradiation may also be effective to eliminate immunogenicity in cells, inactivate or kill selected cells, inactivate viruses or bacteria, or activate desirable immune responses. Phototherapy may be used as an antiviral treatment for certain blood components or whole blood. For example, a pathogenic virus in a donated platelet concentrate may be inactivated by UV light exposure. See PCT Publication No. WO 97/36634.

Although light irradiation may be effective by itself, without the introduction of outside agents or compounds, it may also involve the introduction of specific agents or catalysts, such as, for example, photoactivatable drugs. In a particular application, it is well known that a number of human disease states may be characterized by the overproduction of certain types of leukocytes, including lymphocytes, in comparison to other population of cells which normally comprise whole blood. Excessive abnormal lymphocyte populations result in numerous adverse effects in patients including the functional impairment of bodily organs, leukocyte mediated autoimmune diseases and leukemia related disorders many of which often ultimately result in fatality. Indeed, uses of these photoactivatable drugs may involve treating the blood of a diseased patient where specific blood cells have become pathogenic as a consequence of the disease state. The methods generally may involve treating the pathogenic blood cells, such as lymphocytes, with a photoactivatable drug, such as a psoralen, which is capable of forming photoadducts with lymphocyte DNA when exposed to UV radiation.

Photopheresis using a psoralen such as methoxsalen may cause an immunization against the abnormal (cancerous, in the case of CTCL) T-cells. During photopheresis, methoxsalen enters the white blood cell nuclei and intercalates in the double-strand DNA helix. In an extracorporeal circuit, long wave ultraviolet light is directed at the leukocyte-enriched blood volume. The methoxsalen, responding to the ultraviolet energy, links to the thymidine base in the DNA helix. This results in the cross-linking of thymidine bases which prevent the unwinding of the DNA during transcription. Ultraviolet A light (UVA) damages abnormal T-cells rendering them more immunogenic. Other psoralens or psoralen derivatives may act via another pathway. Nevertheless, after cells are photoactivated, reinfusion of these altered T-cells causes an immunological reaction that targets T-cells carrying the same surface antigens. *See* Edelson, 636 ANN. N.Y. ACAD. SCI. 154-64 (1991). This results in the production of a highly specific immune response against the abnormal cells (either a cancer clone or perhaps T-cells which express viral antigens on their surface). It is estimated that approximately 25-50% of the total peripheral blood mononuclear cell 5 compartment is treated per photopheresis session (2 consecutive days schedule).

Work by Vowels demonstrated monocytes treated in an extracorporeal circuit of plasma containing 8-methoxypsoralen and exposure to ultraviolet-A light (photopheresis) releases tumor necrosis factor-alpha, IL-1, IL-6, and possibly IL-8. Vowels et al., 98 J. INVEST. DERMATOL. 686-92 (1998). It is believed that photopheresis modulates the activity of peripheral blood monocytes/macrophages.

Photopheresis also has been shown to be an effective therapy in a number of autoimmune diseases such as progressive systemic sclerosis (*see* Rook et al., 128 ARCH. DERMATOL. 337 (1992)), inflammatory bowel disease, rheumatoid arthritis (*see* Malawista et al., 34 ARTHMTIS RHEUM. 646 (1991)), and juvenile onset diabetes mellitus (*see* Ludvigsson, 9(4) DIABETES METAB. REV. 329 (1993)), as well as other T-cell mediated phenomena including graft-versus-host disease ("GVHD") (*see* Rosseti et al., 59(1) TRANSPLANT 149 (1995)), and organ allograft rejection after transplantation (*see* Rook et al., 9(1) J. CLIN. APIMRESIS 28 (1994)).

### SUMMARY OF THE INVENTION

In one aspect, the present invention relates to methods for treating a subject predisposed to an autoimmune disease with an effective amount of apoptotic cells before the clinical manitfestation of a symptom associated with the autoimmune disease. In another aspect, the present invention further relates to methods for treating a subject predisposed to an atopic disease with an effective amount of apoptotic cells before the clinical manitfestation of a symptom associated with the atopic disease. In another aspect, the present invention relates to methods for treating a subject predisposed predisposed to an autoimmune reaction manifested by a T-cell response with an effective amount of apoptotic cells before the clinical manifestation of a symptom associated with the autoimmune reaction.

In one embodiment, the effective amount of apoptotic cells may be administered to the subject after the identification of a disease marker for the autoimmune disease in the subject. In another embodiment, the predisposition may be determined by the identification of a disease marker for the autoimmune disease in the subject. In either or both embodiments, the disease marker may include, but is not limited to, genetic marker, serological marker, immunological marker, gene expression profile, protein expression profile, and polymorphism. In context of treating a subject predisposed to an autoimmune disease or an atopic disease, the effective amount of apoptotic cells may be administered according to a dosage schedule that includes, but not limited to, weekly, monthly, twice a month, three times a month, every other month, every three months, every six months, every nine months, and yearly.

In another aspect, the present invention relates to methods for treating a transplant donor and/or a transplant recipient, or an implant recipient with an effective amount of apoptotic cells prior to the transplant. For example, in one embodiment, the transplant donor and transplant recipient may be treated with an effective amount of apoptotic cells prior to harvesting and receving the transplant, respectively. In an alternative embodiment, the transplant recipient may be treated with an effective amount of apoptotic cells prior to receiving the transplant. In another embodiment, the transplant donor may be treated with an effective amount of apoptotic cells prior to harvesting the transplant. In yet another embodiment, the transplant recipient may further be treated after receiving the transplant. In one embodiment, the implant recipient may be treated with an effective amount of apoptotic cells prior to receiving the implant. Moreover, the implant recipient may further be treated after receiving the implant.

In the context of treating prior to transplant, the effective amount of apoptotic cells may be administered according to a dosage schedule that includes, but is not limited to, two days, one week prior to harvesting and/or receving the transplant, or receiving the implant; three days, one week prior to harvesting and/or receving the transplant, or receiving the implant; two days a week for two weeks prior to harvesting and/or receving the transplant, or receiving the implant; and three days a week for three weeks prior to harvesting and/or receving the transplant, or receiving the implant.

In the context of treating after the transplant or implant, the effective amount of apoptotic cells may be administered to the transplant recipient or implant recipient according to a dosage schedule that includes, but is not limited to, weekly, monthly, twice a month, three times a month, every other month, every three months, every six months, every nine months, and yearly.

In a specific embodiment, the apoptotic cells are in a liquid suspension. Specifically, the apoptotic cells may comprise from about 10% to about 90% of the total number of cells in the liquid suspension. More specifically, the apoptotic cells may comprise from about 30% to about 70% of the total number of cells in the liquid suspension.

In another specific embodiment, the effective amount of apoptotic cells comprises a dosage of apoptotic cells comprising from about 10,000 to about 10,000,000 apoptotic cells per kilogram body weight of the subject, transplant recipient and/or transplant donor, or implant recipient. Specifically, the dosage may contain from about 500,000 to about 5,000,000 apoptotic cells per kilogram body weight of the subject, transplant recipient and/or transplant donor, or implant recipient. More specifically, the dosage may contain from about 1,500,000 to about 4,000,000 apoptotic cells per kilogram body weight of the subject, transplant recipient and/or transplant donor, or implant recipient.

In one embodiment, the apoptotic cells may comprise apoptotic cells derived from a cultured cell line. Specifically, the apoptotic cells may comprise cultured cell line cells subjected to extracorporeal treatment. In an alternative embodiment, the apoptotic cells may comprise blood cells compatible with those of the subject, transplant recipient and/or transplant donor, or implant recipient. Specifically, the apoptotic cells may comprise blood cells subjected to extracorporeal treatment. In either context, the extracorporeal treatment may include, but is not limited to, antibodies, chemotherapeutic agents, radiation, extracorporeal photopheresis, ultrasound, proteins, and oxidizing agents.

In one embodiment, the blood cells that are compatible with the subject, transplant recipient and/or transplant donor, or implant recipient may comprise white blood cells, specifically, T-cells. In another embodiment, the blood cells that are compatible with the subject, transplant recipient and/or transplant donor, or implant recipient may comprise the subject's or transplant recipient's and/or transplant donor's own blood cells. Specifically, the subject's, transplant recipient's and/or transplant donor's, or implant recipient's own blood cells may comprise white blood, more specifically, T-cells.

In one aspect, the present invention relates to methods for treating a subject predisposed to an autoimmune disease with extracorporeal photopheresis before the clinical manitfestation of a symptom associated with the autoimmune disease. In another aspect, the present invention relates to methods for treating a subject predisposed to an atopic disease with extracorporeal photopheresis before the clinical manitfestation of a symptom associated with the atopic disease. In another aspect, the present invention relates to methods for treating a subject predisposed predisposed to an autoimmune reaction manifested by a T-cell response with extracorporeal photopheresis before the clinical manifestation of a symptom associated with the autoimmune reaction. In yet another aspect, the present invention relates to methods for treating a transplant donor and/or a transplant recipient, or an implant recipient with extracorporeal photopheresis prior to the transplant or implantation procedure.

In context of treating a subject predisposed to an autoimmune disease or an atopic disease, extracorporeal photopheresis may comprise administering to at least a portion of the blood of a subject predisposed to an autoimmune or an atopic disease, a photoactivatable compound before the clinical manifestation of a symptom associated with the autoimmune disease or the atopic disease; and treating the portion of the blood of the subject with light of a wavelength that activates the photoactivatable compound, before the clinical manifestation of a symptom associated with the autoimmune disease or the atopic disease.

In context of treating a subject predisposed to an autoimmune disease or an atopic disease, extracorporeal photopheresis may be administered to the subject after the identification of a disease marker for the autoimmune disease in the subject. In another embodiment, the predisposition may be determined by the identification of a disease marker for the autoimmune disease in the subject. In either or both embodiments, the disease marker may include, but is not limited to, genetic marker, serological marker, immunological marker, gene expression profile, protein expression profile, and polymorphism.

In context of treating a subject predisposed to an autoimmune disease or an atopic disease, extracorporeal photopheresis may be administered to the subject according to a dosage schedule that includes, but is not limited to, weekly, monthly, twice a month, three times a month, every other month, every three months, every six months, every nine months, and yearly.

In another aspect, the present invention relates to methods for treating a transplant donor and/or a transplant recipient, or an implant recipient with extracorporeal photopheresis prior to the transplant. For example, in one embodiment, the transplant donor and transplant recipient may be treated with extracorporeal photopheresis prior to harvesting and receving the transplant, respectively. In an alternative embodiment, the transplant recipient may be treated with extracorporeal photopheresis prior to receiving the transplant. In another embodiment, the transplant donor may be treated with extracorporeal photopheresis prior to harvesting the transplant. In yet another embodiment, the transplant recipient may further be treated after receiving the transplant. In one embodiment, the implant recipient may be treated with extracorporeal photopheresis prior to receiving the implant. Moreover, the implant recipient may further be treated after receiving the implant.

In context of treating a transplant donor and/or transplant recipient, or an implant recipient, extracorporeal photopheresis may comprise administering to at least a portion of the blood of a transplant donor and/or transplant recipient, or an implant recipient, a photoactivatable compound prior to harvesting the transplant and/or receiving the transplant, or receiving the implant; and treating the portion of the blood of the transplant donor and/or transplant recipient, or implant recipient with light of a wavelength that activates the photoactivatable compound, prior to harvesting the transplant and/or receiving the transplant, or receiving the implant.

In one embodiment, the photoactivatable compound comprises a psoralen or psoralen derivative. Specifically, psoralen or psoralen derivative comprises 8-methoxypsoralen. Alternatively, the psoralen or psoralen derivative comprises amotosalen.

In the context of treating prior to transplant or implant, extracorporeal photopheresis may be administered to the transplant recipient and/or transplant donor, or implant recipient according to a dosage schedule that includes, but is not limited to, two days, one week prior to harvesting and/or receving the transplant, or receiving the implant; three days, one week prior to harvesting and/or receving the transplant, or receiving the implant; two days a week for two weeks prior to harvesting and/or receving the transplant, or receiving the implant; and three days a week for three weeks prior to harvesting and/or receving the transplant, or receiving the implant.

In the context of treating after the transplant or implant, extracorporeal photopheresis may be administered according to to the transplant recipient or implant recipient a dosage schedule that includes, but is not limited to, weekly, monthly, twice a month, three times a month, every other month, every three months, every six months, every nine months, and yearly.

In at least one embodiment of the methods for the present invention, the autoimmune disease may include, but is not limited to, alopecia areata, ankylosing spondylitis, antiphospholipid syndrome, autoimmune Addison's disease, autoimmune hemolytic anemia, autoimmune hepatitis, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatricial pemphigoid, CREST syndrome, cold agglutinin disease, Crohn's disease, discoid lupus, essential mixed cryoglobulinemia, fibromyalgia-fibromyositis, Graves' disease, Guillain-Barré, Hashimoto's thyroiditis, idiopathic pulmonary fibrosis, idiopathic thrombocytopenia purpura (ITP), IgA nephropathy, insulin dependent diabetes, juvenile arthritis, lichen planus, ménière's disease, mixed connective tissue disease, multiple sclerosis, myasthenia gravis, pemphigus vulgaris, pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, Raynaud's phenomenon, Reiter's syndrome, rheumatic fever, rheumatoid arthritis, sarcoidosis, scleroderma, Sjögren's syndrome, stiff-man syndrome, systematic lupus erythematosus, Takayasu arteritis, temporal arteritis/giant cell arteritis, ulcerative colitis, uveitis, vasculitis, vitiligo, and Wegener's granulomatosis.

Alternatively, the atopic disease may include, but is not limited to, atopic dermatitis, extrinsic bronchial asthma, urticaria, allergic rhinitis, and allergic enterogastritis. In yet another embodiment, the autoimmune reaction may be associated with a transplant including, but not limited to, organ transplant, tissue transplant, bone marrow transplant, and stem cell transplant. Alternatively, the autoimmune reaction may be associated with a disease including, but not limited to, autoimmune disease and atopic disease.

In one embodiment, the transplant may include, but is not limited to, organ, tissue, stem cell, and bone marrow. Specifically, the organ transplant may be an organ graft including, but not limited to, syngeneic graft, allograft, and xenograft. The organ may be selected from the group consisting heart, liver, pancreas, pancreatic islets, kidney, lung, larynx, stem cells, eyes, cornea, muscle, and skin. Moreoever, the organ may include, but is not limited to, human, artificial, clonal, and mammalian.

In another specific embodiment, the tissue transplant may be a tissue graft including, but not limited to, autograft, syngeneic graft, allograft, and xenograft. More specifically, the tissue may include, but is not limited to, cartilage, bone, liver, small-bowel, neuronal, adrenal medullary tissue, fetal thymus tissue, and parathyroid tissue. Moreoever, the tissue may include, but is not limited to, human, artificial, clonal, and mammalian.

In yet another specific embodiment, the stem cell transplant may include, but is not limited to, allogeneic and xenogeneic. More specifically, the stem cell may include, but is not limited to, ectoderm, endoderm, mesenchymal, or any cells derived therefrom.

In another embodiment, the bone marrow transplant may include, but is not limited to, allogeneic and xenogeneic. In yet another embodiment, the implant may include, but is not limited to, spinal, vertebral, bone repair, bone replacement, joint replacement, metal plate, facial, hair, collagen, prostate seed, breast, hormonal, pacemaker, defibrillator, cochlear, muscle, and cortical.

### DETAILED DESCRIPTION OF THE INVENTION

Before the present invention is described, it is to be understood that the terminology used herein is for the purpose of describing particular embodiments only, and is not intended to limit the scope of the present invention which will be limited only by the appended claims.

It must be noted that as used herein and in the appended claims, the singular forms "a," "and," and "the" include plural reference unless the context clearly dictates otherwise. Thus, for example, reference to "a drug" is a reference to one or more drugs and includes equivalents thereof known to those skilled in the art, and so forth.

Unless defined otherwise, all technical and scientific terms used herein have the same meaning as commonly understood to one of ordinary skill in the art to which this invention belongs. Although any methods, devices, and materials similar or equivalent to those described herein may be used in the practice or testing of the invention, the preferred methods, devices and materials are now described.

All publications and patents mentioned herein are hereby incorporated by reference for the purpose of describing and disclosing, for example, the methodologies that are described in the publications which might be used in connection with the presently described invention. The publications are provided solely for their disclosure prior to the filing date of the present application. Nothing herein is to be construed as an admission that the inventors are not entitled to antedate such disclosure by virtue of prior invention.

The present invention relates to methods for treating a subject predisposed to an autoimmune disease with extracorporeal photopheresis or an effective amount of apoptotic cells before the clinical manitfestation of a symptom associated with the autoimmune disease. The present invention alsorelates to methods for treating a subject predisposed to an atopic disease with extracorporeal photopheresis or an effective amount of apoptotic cells before the clinical manitfestation of a symptom associated with the atopic disease. The present invention further relates to methods for treating a transplant donor and/or a transplant recipient, or an implant recipient with extracorporeal photopheresis or an effective amount of apoptotic cells prior to the transplant or implant procedure.

Thus, the present invention relates to the administration of extracorporeal photopheresis ("ECP") and/or apoptotic cells to prevent, or reduce the potential severity of, GVHD and/or transplant or implant rejection in patients about to undergo a transplant or implant procedure. The present invention further relates to the administration of ECP and/or apoptotic cells to prevent, delay, or reduce the potential severity of, autoimmune diseases and atopic diseases in subjects predisposed to such diseases.

The administration of apoptotic cells to subjects, in the context of preventing, or reducing the potential severity of, GVHD, for example, provides the same immunosuppressive effects as the use of ECP with GVHD. Indeed, the link between ECP and the induction of apoptosis has been established in the context of GVHD. *See* Bladon et al., 146(1) BR. J. DERMATOL. 59-68 (2002); Knobler et al., 941 ANN. N.Y. ACAD. Sci. 123-38 (2001); and Bladon et al., 107(4) BR. J. HAEMATOL. 707-11 (1999). Moreover, ECP has been used to treat a variety of active disease states, i.e., patients who are currently suffering from particular disease states. Those include atopic dermatitis (Krutmann, 25(7) CLIN. EXP. DERMATOL. 552-8 (2000); Richter et al., 38(4) J. AM. ACAD. DERMATOL. 585-8 (1998)); scleroderma (Simon et al., 10(8) EUR. J. DERMATOL. 642-5 (2000)); urticaria (Mang et al., 18(4) PHOTODERMATOL. PHOTOIMMUNOL. PHOTOMED. 196-8 (2002)); type 1 diabetes (Ludvigsson et al., 85(2) ARCH. DIS. CHILD. 149-54 (2001)); pemphigus (Efferth et al., 21(4A) ANTICANCER RES. 2777-83 (2001)); and lupus (Richard et al., 129(8-9) ANN. DERMATOL. VENEROL. (2002)). Thus, the administration of ECP and/or apoptotic cells before transplant may prevent, or reduce the potential severity of, GVHD by impacting, for example, an associated autoimmune response. Such methods may similarly be applied in the context of transplant or implant rejection. Moreover, the administration of ECP and/or apoptotic cells to subjects is equally applicable to the prevention of, delay of, or reduction of the potential severity of, disease states that involves the mechanism of GVHD or transplant rejection, and specifically by impacting an associated autoimmune response. For example, the administration of ECP and/or apoptotic cells may prevent, delay, or reduce the potential severity of, the T-cell mediated response associated with an autoimmune disease or an atopic disease. Furthermore, the administration of ECP and/or apoptotic cells may be applicable in the context of the autoimmune diseases and atopic diseases described herein and, more particularly, to any other disease state that may be further identified as being associated with a similar T-cell mediated reaction.

### I. Treatment of a Subject with Extracorporeal Photopheresis

As used herein, "ECP" refers to extracorporeal photopheresis, also known as extracorporeal phototherapy. The present invention relates to the use of ECP to prevent or reduce the potential severity of graft-versus-host disease in a transplant recipient about to undergo a transplant. "Subject," as used in the present invention, includes human or animal subjects. The terms "transplant recipient" and "transplant donor" includes human or other animal transplant recipients and donors, respectively. The term "transplant recipient" has the same meaning as "recipient of a transplant." Similarly, the term "implant recipient" has the same meaning as "recipient of an implant." In the context of "transplant donors," the word "harvesting" has the same meaning as applied by those of ordinary skill in the art, which may specifically include, for example, the removal of the transplant from the donor. Similarly, in the context of "transplant recipients," the words "receiving the transplant" has the same meaning as applied by those of ordinary skill in the art, which may specifically include, for example, the the implantation of the transplant into the transplant recipient.

The present invention also relates to the use of ECP to prevent or reduce the potential severity of organ or tissue transplant rejection in a transplant recipient about to undergo the transplant. The present invention further relates to the use of ECP to prevent the onset of, delay the onset of or reduce the potential severity of an autoimmune disease or an atopic disease. While it is not intended that the scope of the present invention be limited by any specific theory of operation, it is believed that autoimmune disease, atopic disease, graft-versus-host disease, and transplant rejection may be prevented, delayed, or the potential severity reduced by using an ECP treatment according to the present invention.

### A. Extracorporeal Photopheresis

In one embodiment, a photoactivatable or photosensitive compound is first administered to at least a portion of the blood of a recipient of a transplant prior to the recipient receiving the transplant. Alternatively, in the context of transplantation, the photoactivatable compound may be administered to at least a portion of the blood of a transplant donor prior to harvesting the transplant from the donor. The photoactivatable or photosensitive compound may be administered *in vivo* (*e.g.,* orally or intravenously). The photosensitive compound, when administered to the subject's blood, recipient's blood, or the donor's blood, as the case may be, *in vivo* may be administered orally, but also may be administered intravenously and/or by other conventional administration routes. The oral dosage of the photosensitive compound may be in the range of about 0.3 to about 0.7 mg/kg., more specifically, about 0.6 mg/kg.

When administered orally, the photosensitive compound may be administered at least about one hour prior to the photopheresis treatment and no more than about three hours prior to the photopheresis treatment. If administered intravenously, the times would be shorter.

Alternatively, the photosensitive compound may be administered to the subject's blood, recipient's blood, or the donor's blood following its withdrawal from the subject, recipient, or donor, respectively, and prior to or contemporaneously with exposure to ultraviolet light. The photosensitive compound may be administered to whole blood or a fraction thereof provided that the target blood cells or blood components receive the photosensitive compound. A portion of the subject's blood, recipient's blood, or the donor's blood could first be processed using known methods to substantially remove the erythrocytes and the photoactive compound may then be administered to the resulting enriched leukocyte fraction. In one embodiment, the blood cells comprise white blood cells, specifically, T-cells.

In accordance with the present invention, the photoactivatable or photosensitive compound may, in the case of some psoralens, be capable of binding to nucleic acids upon activation by exposure to electromagnetic radiation of a prescribed spectrum, *e.g.*, ultraviolet light.

Photoactive compounds for use in accordance with the present invention may include, but are not limited to, compounds known as psoralens (or furocoumarins) as well as psoralen derivatives such as those described in, for example, U.S. Pat. No. 4,321,919 and U.S. Pat. No. 5,399,719. The photoactivatable or photosensitive compounds that may be used in accordance with the present invention include, but are not limited to, psoralen and psoralen derivatives; 8-methoxypsoralen; 4,5'8-trimethylpsoralen; 5-methoxypsoralen; 4-methylpsoralen; 4,4-dimethylpsoralen; 4-5'-diinethylpsoralen; 4'-aminomethyl-4,5',8-trimethylpsoralen; 4'-hydroxymethyl-4,5',8-trimethylpsoralen; 4',8-methoxypsoralen; and a 4'-(omega-amino-2-oxa) allcyl-4,5',8-trimethylpsoralen, including but not limited to 4'-(4-amino-2-oxa)butyl-4,5',8-trimethylpsoralen. In one embodiment, the photosensitive compound that may be used comprises the psoralen derivative, amotosalen (S-59) (Cerus, Corp., Concord, CA). *See, e.g.*, U.S. Patent Nos. 6,552,286; 6,469,052; and 6,420,570. In another embodiment, the photosensitive compound that may be used in accordance with the invention comprises 8-methoxypsoralen.

Methoxsalen is a naturally occurring photoactive substance found in the seed of the Ammi majus (umbelliferae plant). It belongs to a class of compounds known as psoralens or furocoumarins. The chemical name is 9-methoxy-7H-furo[3,2-g][1]-benzopyran-7-one. The formulation of the drug is a sterile liquid at a concentration of 20 mcg/mL in a 10 mL vial. See http://www.therakos.com/TherakosUS/pdf/uvadexpi.pdf. Toxicology studies of extracorporeal photopheresis and different dosages of UVADEX® and ultraviolet light in beagle dogs is located in the investigator's brochure.

Next, the portion of the subject's blood, recipient's blood, or the donor's blood to which the photoactive compound has been administered is treated by subjecting the portion of the blood to photopheresis using ultraviolet light. The photopheresis treatment in the treatment methods according to the present invention may be carried out using long wavelength ultraviolet light (UVA) at a wavelength within the range of 320 to 400 nm. Such a range is not limiting, however, but is merely provided as an example. The exposure to ultraviolet light during the photopheresis treatment may have a duration of sufficient length to deliver, for example, about 1-2 J/cm² to the blood.

The photopheresis step in accordance with the present invention may be carried out *in vivo.* When the photopheresis treatment according to the present invention is carried out *in vivo*, careful attention should be paid to controlling the maximum radiant exposure so as to avoid unnecessary injury to the patient. Methods for calculating maximum radiant exposure to ultraviolet light are known to those of ordinary skill in the art.

In an alternative embodiment, the photopheresis step may be carried out *in vitro* using an extracorporeal photopheresis apparatus. An extracorporeal photopheresis apparatus that may be used in the methods according to the invention is currently manufactured by Therakos, Inc., (Exton, PA) under the name UVAR®. A description of such an apparatus may be found, for example, in U.S. Pat. No. 4,683,889. The exposure of blood to ultraviolet light in a photopheresis apparatus is within the ability of persons having ordinary skill in the art.

In one embodiment, when the photopheresis step is carried out *in vitro,* at least a fraction of the treated blood is returned to the subject, recipient, or donor. The treated blood or the treated enriched leukocyte fraction (as the case may be) may then be administered back to the subject, recipient, or donor. Alternatively, the subject's blood, recipient's blood, or the donor's blood may be separated on a standard apheresis-type device and photoactivated on a separate device.

A specific but non-limiting example of a photopheresis system is the UVAR® System, which uses a photospheres treatment system and consists of three phases including: 1) the collection of a buffy-coat fraction (leukocyte-enriched), 2) irradiation of the collected buffy coat fraction, and 3) reinfusion of the treated white blood cells. The collection phase has six cycles of blood withdrawal, centrifugation, and reinfusion steps. During each cycle, whole blood is centrifuged and separated in a pediatric pheresis bowl. From this separation, plasma (volume in each cycle is determined by the ITVAR®. Instrument operator) and 40 ml buffy coat are saved in each collection cycle. The red cells and all additional plasma are reinfused to the patient before beginning the next collection cycle. Finally, a total of 240 ml of buffy coat and 300 ml of plasma are separated and saved for UVA irradiation.

The irradiation of the leukocyte-enriched blood within the irradiation circuit begins during the buffy coat collection of the first collection cycle. The collected plasma and buffy coat are mixed with 200 ml of heparinized normal saline and 200 mg of UVADEX®. (water soluble 8-methoxypsoralin). This mixture flows in a 1.4 mm thick layer through the PHOTOCEPTOR®. Photoactivation Chamber, which is inserted between two banks of UVA lamps of the PHOTOSETTE®. PHOTOSETTE® UVA lamps irradiate both sides of this UVA-transparent PHOTOCEPTOR®. chamber, permitting a 180-minute exposure to ultraviolet A light, yielding an average exposure per lymphocyte of 1-2 J/cm². The final buffy coat preparation contains an estimated 20% to 25% of the total peripheral blood mononuclear cell component and has a hematocrit from 2.5% to 7%. Following the photoactivation period, the volume is reinfused to the patient over a 30 to 45 minute period.

For a description of similar photopheresis systems useful in the methods of the present invention, see U.S. Patent Application No. 09/480,893, which is entirely expressly incorporated herein by reference. Also useful herein are the methods and systems described in U.S. Patent Nos. 5,951,509; 5,985,914; 5,984,887, 4,464,166; 4,428,744; 4,398,906; 4,321,919; PCT Publication Nos. WO 97/36634; and WO 97/36581, all of which are entirely expressly incorporated herein by reference.

Another system that may be useful in the methods of the present invention is described in U.S. Patent Application No. 09/556,832, which is entirely expressly incorporated herein by reference. The system described therein relates to systems and apparatus by which the net fluid volume collected or removed from a patient may be reduced during a medical treatment process such as ECP. By way of example, an ECP process such as the IIVAR® process (Therakos, Inc., Exton, PA) removes blood from a patient, separates the buffy coat from the plasma and red blood cells and replaces the biological fluids in a batch process. When blood is removed from the patient, however, a volume deficit is created within the patient. This volume deficit is particularly detrimental in small children and the elderly or in patients that suffer from certain illnesses or diseases because their blood has a higher percentage of plasma relative to the cellular components. This volume imbalance requires that a greater volume of blood be drawn from the patient to obtain the required amount of red blood cells. This especially impacts infants and sick children who may have low body weight and hemocrit percentages of 25-30% which is significantly lower than the normal average of 45%. The need thus arose to be able to detect small incremental changes in natural fluid ratios within the body and to use these measurements to create a process by which the net fluid volume collected or removed from a patient may be reduced during a medical treatment process.

The effective amount of light energy that is delivered to the biological fluids may be determined using the methods and systems described in U.S. Patent No. 6,219,584, which is entirely expressly incorporated herein by reference. Indeed, the application of ECP to the various diseases described herein may require an adjustment of the amount of light energy to optimize the treatment process.

Furthermore, the photosensitizing agent used in the ECP process may be removed prior to returning the treated biological fluid to the patient. For example, the UVAR® System utilizes Methoxsalen (UVADEX®) in the ECP process. Methoxsalen belong to a group of compounds known as psoralens. The exposure to methoxsalen or other psoralens may cause undesirable effects on the subject, recipient, or donor such as phototoxicity or other toxic effects associated with psoralen and their decomposition products. Therefore, the psoralen, psoralen derivatives, or psoralen decomposition products that may remain in the biological fluid may be removed after UV exposure. A process for the removal of psoralen biological fluids is described in U.S. Patent No. 6,228,995, which is entirely expressly incorporated herein by reference.

The ECP system useful in the methods of the present invention may incorporate one or more components described in U.S. Patent Nos. 6,069,687 (contaminant detector), 5,921,951 (steady flow rate pump), 5,569,928 (photoactivation light array), 5,459,322 (ultraviolet light chamber), 5,330,420 (hemolysis detector), 5,308,309 (securing system for centrifuge chamber), 4,921,473 (multicomponent fluid separation and irradiation system); and U.S. Application No. 09/389,463 (uninterrupted flow pump apparatus), all of which are entirely expressly incorporated herein by reference.

### B. Treatment Methods

Accordingly, the present invention relates to methods for preventing, or reducing the potential severity of, organ transplant rejection in subjects by treating the subjects with ECP prior to transplantation. The methods of the present invention are also applicable in preventing, or reducing the potential severity of, tissue transplant rejection using ECP. The methods of the present invention further relate to preventing or reducing the potential severity of GVHD in subjects about to undergo a transplant. The methods of the present invention further relate to preventing, delaying, or reducing the potential severity of one or more diseases including autoimmune diseases and atopic diseases in subjects predisposed to such diseases.

More specifically, in the context of disease states, ECP may be administered, for example, before the clinical manifestation of a symptom associated with an autoimmune disease, before the clinical manifestation of a symptom associated with an autoimmune reaction, or before the clinical manifestation of a symptom associated with an atopic disease. In one embodiment, ECP may be administered to a subject predisposed to, for example, an autoimmune disease, before the subject manifests a clinical symptom associated with the autoimmune disease. More specifically, ECP may be administered to a subject predisposed to an autoimmune disease before the clinical manifestation of a symptom associated with the autoimmune disease that is treatable by known therapies. In another embodiment, ECP may be administered to a subject predisposed to, for example, an autoimmune disease, after the identification of a disease marker associated with the autoimmune disease. Indeed, ECP may be administered, for example, to a subject predisposed to a particular disease at any point between the identification of a disease marker associated with the disease and the clinical manifestation of a symptom associated with the disease. Such symptoms include symptoms described herein and known to those of ordinary skill in the art. *See generally* THE MERCK MANUAL (Mark H. Beers & Robert Berkow eds., 17th ed. 1999), which is entirely expressly incorporated herein by reference; *see also generally* THE MERCK MANUAL (Mark H. Beers & Robert Berkow eds., 17th ed. 1999), *available at* http://www.merck.com/pubs/mmanual. Symptoms and the clinical manifestation thereof may also be identified using guidelines issued, for example, from medical institutions such as The National Institutes of Health. As described herein and otherwise known to those of ordinary skill in the art, a disease marker may include, but is not limited to, genetic marker, serological marker, immunological marker, gene expression profile, protein expression profile, and polymorphism. Such disease markers may be used to identify whether a subject is predisposed to a particular disease.

In the context of prevention, as a result of ECP, the subject may not exhibit any symptoms of GVHD, transplant rejection, autoimmune disease, or atopic disease. In the context of reducing the potential severity of, for example, GVHD, the subject may exhibit one or more symptoms of GVHD but such symptoms of GVHD are less severe, or the subject may not exhibit all of the symptoms thereof, than subjects that do not receive ECP prior to transplant. In another embodiment, ECP may delay the onset of a disease such as an autoimmune disease or an atopic disease. Indeed, the subject may exhibit one or more symptoms of a particular disease but the manifestation of such symptoms occur, if at all, at a time later and/or at a reduced severity, than the manifestation of symptoms in a subject that does not receive ECP.

In a specific embodiment, ECP may be given prophylacticly and on a one-time or on a periodic basis, specifically in the context of the treatment of subjects predisposed to a T-cell response associated with an autoimmune reaction, and specifically in the context of an autoimmune disease. In an alternative embodiment, ECP may be given prophylacticly and on a one-time or on a periodic basis, specifically in the context of the treatment of subjects predisposed to an atopic disease. In another specific embodiment, ECP may be given prophylacticly and on a one-time or on a periodic basis, specifically in the context of preventing, or reducing the potential severity of, graft-versus-host disease in transplant subjects. In yet another specific embodiment, ECP may be given prophylacticly and on a one-time or on a periodic basis, specifically in the context of preventing, or reducing the potential severity of, transplant rejection in transplant recipients.

In all of the applications discussed herein, ECP may be given to a subject, recipient, and/or donor at an appropriate time and on an appropriate schedule, as determined by one skilled in the art. One skilled in the art would be able to determine such appropriate times and periods based on, for example, the sex, age, race, general health, disease state, etc., of the subject, as well as desired outcomes based on, for example, the desired length or amount of prevention, delay, or reduction of, for example, an autoimmune disease, an atopic disease, a T-cell response, GVHD, organ transplant rejection, or tissue transplant rejection.

In the context of subjects in whom it may be determined whether they are predisposed to autoimmune or atopic diseases that, for example, may be assessed by genetic testing procedures, *i.e.*, genetic testing of HLA haplotype in cord blood stem cells obtained from newborn infants, through SNPs or other genetic markers associated with autoimmune disease, or by other known blood or serum tests indicative of autoimmune disease, the methods of the present invention may include ECP performed on the subject at an appropriate time and for an appropriate period after the determination of said predisposition.

Indeed, the methods of the present invention include ECP performed on a subject in the present context one or more times and over one or more periods of time. The one or more times include one to at least one hundred or more times, one to at least fifty times, one to at least twenty five times, one to at least ten times, one to at least five times, one to four, one to three and two times and any combination of said times with one or more of periods of time, which include daily, weekly, monthly, yearly and any combination thereof.

In one embodiment, ECP may be administered at least one time at least 12 months, at least 11 months, at least 10 months, at least 9 months, at least 8 months, at least 7 months, at least 6 months, at least 6 months, at least 5 months, at least 4 months, at least 3 months, at least 2 months, or at least 1 month before transplant, before the clinical manifestation of a symptom associated with an autoimmune disease, before the clinical manifestation of a symptom associated with an autoimmune reaction, or before the clinical manifestation of a symptom associated with an atopic disease.

In another embodiment, ECP may be administered at least one time at least 30 days, at least 29 days, at least 28 days, at least 27 days, at least 26 days, at least 25 days, at least 24 days, at least 23 days, at least 22 days, at least 21 days, at least 20 days; at least 19 days, at least 18 days, at least 17 days, at least 16 days, at least 15 days, at least 14 days, at least 13 days, at least 12 days, at least 11 days, at least 10 days, at least 9 days, at least 8 days, at least 7 days, at least 6 days, at least 5 days, at least 4 days, at least 3 days, at least 2 days, or at least 1 day before transplant, before the clinical manifestation of a symptom associated with an autoimmune disease, before the clinical manifestation of a symptom associated with an autoimmune reaction, or before the clinical manifestation of a symptom associated with an atopic disease.

In the context of bone marrow, organ, and tissue transplantation, ECP may be administered according to a schedule that includes, but is not limited to, two days, one week prior to transplant; three days, one week prior to transplant; two days a week for two weeks before transplant; and three days a week for three weeks before transplant.

Moreover, in the context of preventing and/or reducing the potential severity of GVHD and/or rejection in the bone marrow, stem cell, organ, and tissue transplants described herein and otherwise known to those of ordinary skill in the art, ECP may be administered after the transplant similar to the schedules described above and more particularly, weekly, monthly, twice a month, three times a month, every other month, every six months, every nine months, and yearly. Such an exemplary schedule may also be utilized in the context of preventing, delaying, or reducing the potential severity of one or more diseases including, for example, autoimmune diseases and atopic diseases.

For example, ECP may be administered at least once a week over the course of several weeks. In one embodiment, ECP may be administered at least once a week over several weeks to several months. In another embodiment, ECP may be administered once a week over four to eight weeks. In yet another embodiment, ECP may be administered once a week over four weeks.

More specifically, ECP may be administered at least once a day for about 2 days, at least once a day for about 3 days, at least once a day for about 4 days, at least once a day for about 5 days, at least once a day for about 6 days, at least once a day for about 7 days, at least once a day for about 8 days, at least once a day for about 9 days, at least once a day for about 10 days, at least once a day for about 11 days, at least once a day for about 12 days, at least once a day for about 13 days, at least once a day for about 14 days, at least once a day for about 15 days, at least once a day for about 16 days, at least once a day for about 17 days, at least once a day for about 18 days, at least once a day for about 19 days, at least once a day for about 20 days, at least once a day for about 21 days, at least once a day for about 22 days, at least once a day for about 23 days, at least once a day for about 24 days, at least once a day for about 25 days, at least once a day for about 26 days, at least once a day for about 27 days, at least once a day for about 28 days, at least once a day for about 29 days, at least once a day for about 30 days, or at least once a day for about 31 days.

Alternatively, ECP may be administered about once every day, about once every 2 days, about once every 3 days, about once every 4 days, about once every 5 days, about once every 6 days, about once every 7 days, about once every 8 days, about once every 9 days, about once every 10 days, about once every I 1 days, about once every 12 days, about once every 13 days, about once every 14 days, about once every 15 days, about once every 16 days, about once every 17 days, about once every 18 days, about once every 19 days, about once every 20 days, about once every 21 days, about once every 22 days, about once every 23 days, about once every 24 days, about once every 25 days, about once every 26 days, about once every 27 days, about once every 28 days, about once every 29 days, about once every 30 days, or about once every 31 days.

ECP may alternatively be administered about once every week, about once every 2 weeks, about once every 3 weeks, about once every 4 weeks, about once every 5 weeks, about once every 6 weeks, about once every 7 weeks, about once every 8 weeks, about once every 9 weeks, about once every 10 weeks, about once every 11 weeks, about once every 12 weeks, about once every 13 weeks, about once every 14 weeks, about once every 15 weeks, about once every 16 weeks, about once every 17 weeks, about once every 18 weeks, about once every 19 weeks, about once every 20 weeks.

Alternatively, ECP may be administered about once every month, about once every 2 months, about once every 3 months, about once every 4 months, about once every 5 months, about once every 6 months, about once every 7 months, about once every 8 months, about once every 9 months, about once every 10 months, about once every 11 months, or about once every 12 months.

Alternatively, ECP may be administered at least once a week for about 2 weeks, at least once a week for about 3 weeks, at least once a week for about 4 weeks, at least once a week for about 5 weeks, at least once a week for about 6 weeks, at least once a week for about 7 weeks, at least once a week for about 8 weeks, at least once a week for about 9 weeks, at least once a week for about 10 weeks, at least once a week for about 11 weeks, at least once a week for about 12 weeks, at least once a week for about 13 weeks, at least once a week for about 14 weeks, at least once a week for about 15 weeks, at least once a week for about 16 weeks, at least once a week for about 17 weeks, at least once a week for about 18 weeks, at least once a week for about 19 weeks, or at least once a week for about 20 weeks.

Alternatively ECP may be administered at least once a week for about 1 month, at least once a week for about 2 months, at least once a week for about 3 months, at least once a week for about 4 months, at least once a week for about 5 months, at least once a week for about 6 months, at least once a week for about 7 months, at least once a week for about 8 months, at least once a week for about 9 months, at least once a week for about 10 months, at least once a week for about 11 months, or at least once a week for about 12 months.

### II. Treatment of a Subject with Apoptotic Cells

Two mechanisms of cell death in the body are recognized, necrosis and apoptosis. Apoptosis is the process of programmed cell death by which steady-state levels of the various organ systems and tissues in the body are maintained as continuous cell division and differentiation takes place. *See* Kerr et al. 26 BR J CANCER 239-57 (1992). Cells undergoing apoptosis often exhibit distinctive morphological changes such as a pronounced decrease in cell volume, modification of the cytoskeleton resulting in pronounced membrane blebbing, a condensation of the chromatin, and degradation of the DNA into oligonucleosomal fragments. Following these morphological changes, an apoptotic cell may break up into a number of small fragments known as apoptotic bodies, consisting essentially of membrane-bound bodies containing intact organelles, chromatin etc. Apoptotic bodies are normally rapidly removed from the body by phagocytosis by macrophages, dendritic cells and other antigen presenting cells, before they may become lysed and release their potentially pro-inflammatory intracellular contents.

Three phases are identified in the apoptotic mechanism of programmed cell death: Induction phase, Effector phase, and Degradation phase.

The induction phase is dependent, in part, on specific interactions of death-inducing signals at the cell surface membrane. One common signal is initiated by the binding of specific ligands to receptors of the TNF receptor family present on the cell membrane. One important such receptor is Fas (APO-1, CD95), which interacts with Fas-ligand to initiate apoptosis.

The effector phase, activated by the binding of receptors and ligands of the induction phase, leads to the activation of caspases, cystinyl-aspartate-requiring proteinases (proteolytic enzymes), including caspases 1 and 8. This activation may be associated with a change in the permeability of mitochondria, allowing the release of cytochrome-c which is involved in caspase activation. Activated caspases initiate a chain of lethal proteolytic events culminating in the changes in chromatin and cytoskeletal components seen in apoptosis.

Many cells undergoing apoptosis may be identified by a characteristic 'laddering' of DNA seen on agarose gel electrophoresis, resulting from cleavage of DNA into a series of fragments. These changes occur a few hours before death of the cell as defined by the ability of a cell to exclude vital dyes. The appearance of DNA laddering on agarose gel electrophoresis following extraction of DNA from cells is one recognized method of identification of apoptosis in cells, although it is not always sensitive enough to detect apoptosis. *See* Loo et al., 57 METHODS CELL BIOL. 251-64 (1998). In situ labeling of nuclear DNA fragmentation, for example, using commercially available terminal dUTP nick end labeling (TUNEL) assays, are an alternative and more reproducible measure for the determination of fragmented DNA in apoptotic cells and cells undergoing apoptosis. *See* Gavrieli et al., 119 J. CELL BIOL. 493-501 (1992).

During apoptosis, phosphatidylserine becomes exposed externally on the cell membrane. *See* Fadok et al., 148 J. TMMUNOL. 2207-16 (1992). This exposed phosphatidylserine binds to specific receptors to mediate the uptake and clearance of apoptotic cells in mammals. *See* Fadok et al., 405 NATURE 85-90 (2000). The surface expression of phosphatidylserine on cells is another recognized method of identification of apoptotic cells.

Changes in mitochondrial integrity are intimately associated with apoptosis, resulting in alterations in mitochondrial membrane permeability and the release of cytochrome-c from the mitochondria into the cell cytoplasm. *See* Susin et al., 189 J. EXP. MED. 381-94 (1999). Measurement of changes in mitochondrial membrane potential, reflecting changes in mitochondrial membrane permeability, is another recognized method of identification of apoptotic cells.

A number of other methods of identification of cells undergoing apoptosis and of apoptotic cells, many using monoclonal antibodies against specific markers for apoptotic cells, have also been described in the scientific literature.

Necrosis, in contrast, is cell death of a pathological nature, resulting from injury, bacterial toxin effects, inflammatory mediators, etc., and involving membrane rupture and release of intracellular contents to the surrounding tissue, often with harmful inflammatory consequences. Accordingly, one of the ways in which necrotic cells may be detected and
characterized is by detection of compromised cell membranes, *e.g.* by methods of staining with propidium iodide followed by flow cytometry or microscopy.

### A. Apoptotic Cells

The term "apoptotic cells" as used herein, includes cells and cell bodies ("apoptotic bodies") that exhibit, or will exhibit, one or more apoptosis-characterizing features described herein and known to those of ordinary skill in the art. The term "apoptotic cells" includes cells that have been treated with an apoptosis-inducing agent. Thus, apoptotic cells include cells that are in any of the various stages of apoptosis. For example, an apoptotic cell includes any cell that is in the Induction phase, Effector phase, or the Degradation phase. Moreover, the term "apoptotic cells" include cells that have been treated with an apoptotis-inducing agent yet are still viable. Such cells may exhibit apoptosis-characterizing features at some point, for example, after administration to the subject.

Apoptosis-characterizing features may include, but are not limited to, surface exposure of phosphatidylserine, as detected by standard, accepted methods of detection such as Annexin V staining; alterations in mitochondrial membrane permeability measured by standard, accepted methods (*e.g.*, Salvioli et al., 411 FEBS LETTERS 77-82 (1997)); evidence of DNA fragmentation such as the appearance of DNA laddering on agarose gel electrophoresis following extraction of DNA from the cells (Teiger et al., 97 J. CLIN. INVEST. 2891-97 (1996)), or by in situ labeling (Gavrieli et al., 1992, referenced above).

### B. Methods of Preparation

The apoptotic cells for use in the present invention may be prepared *ex vivo, i.e.,* extracorporeally, from cells, specifically including mammalian, that are compatible with those of the subject, donor, or recipient. Apoptotic cells may be prepared from substantially any type of mammalian cell including cultured cell lines. For example, apoptotic cells may be prepared from a cell type derived from the mammalian subject's own body or from an established cell line. Specifically, apoptotic cells may be prepared from white blood cells of blood compatible with that of the mammalian subject, more specifically, from the subject's own white blood cell and even more specifically, from the subject's own T-cells. Even more specifically, apoptotic cells may be prepared from an established cell line. A cell line that may be useful in the methods of the present invention includes, for example, Jurkat cells (ATCC No. TIB-152). Other cells lines appropriate for use in accordance with the methods of the present invention may be identified and/or determined by those of ordinary skill in the art. The apoptotic cells may be prepared extracorporeally prior to administration to the subject, donor, or recipient. Thus, in one embodiment, an aliquot of the subject's blood, recipient's blood, or the donor's blood may be withdrawn, *e.g.* by venipuncture, and at least a portion of the white cells thereof subjected extracorporeally to apoptosis-inducing conditions.

A variety of methods of inducing apoptosis in mammalian cells, so as to create apoptotic cells, are known in the art and essentially any of these may be adopted in preparing apoptotic cells for use in the present invention. One such method is the subjection of the cells to ionizing radiation (gamma-rays, x-rays, etc.) and/or non-ionizing electromagnetic radiation including ultraviolet light. Apoptosis may be induced by subjecting cells to ultrasound.

Another method is the treatment of the cells with drugs such as non-specific protein kinase inhibitors as exemplified by staurosporine. Bombeli et al. 89 BLOOD 2429-42 (1997). Also, certain chemotherapeutic agents used for the treatment of malignant tumours induce apoptosis, for example adriamycin, as can statin drugs (3-hydroxy-3methylglutaryl coenzyme A reductase inhibitors) (Guijarro et al., 83 J. CIRC. RES. 490-500 (1998)), and colcicine (Suzuki 855 ANN. N.Y. ACAD. SCI. 252-54 (1998)). The use of ligands for death receptors on cells, such as Fas-ligand, will be apparent for inducing apoptosis from the discussion of apoptosis above.

Yet another method is the application of oxidative stress to cells extracorporeally. Buttke et al., 15 IMMUNOL. TODAY 7-10 (1994). This may be achieved by treating the cells, in suspension, with chemical oxidizing agents such as hydrogen peroxide, other peroxides and hydroperoxides, ozone, permanganates, periodates, and the like. Biologically acceptable such oxidizing agents may be used, so as to reduce potential problems associated with residues and contaminations of the apoptotic cells and apoptotic bodies so formed.

The present invention is not restricted to any particular method of producing apoptotic cells, for use herein, and any suitable, known process may be used.

Methods for the detection and quantitation of apoptosis may be used to determine the presence and level of apoptosis in the preparation to be administered to the subject in the present invention. At least one of the methods from those described herein may be used to confirm the level of apoptosis achieved prior to administration. The apoptotic cells may be purified prior to use by methods known in the art, such as differential centrifugation.

In preparing the apoptotic cells, care should be taken not to apply excessive levels of oxidative stress, radiation, drug treatment, etc., since otherwise there is a significant risk of causing necrosis of at least some of the cells under treatment. Necrosis causes cell membrane rupture and the release of cellular contents often with biologically harmful results, particularly inflammatory events, so that the presence of necrotic cells and their components along with the apoptotic bodies is best avoided. Appropriate levels of treatment of the cells to create apoptotic cells composition, and the type of treatment chosen to induce apoptosis are readily determinable by those skilled in the art, having regard to the available scientific literature on the subject including the above-referenced articles.

One process according to the present invention involves the culture of cells from the subject, or a compatible mammalian cell line. The cultured cells may then be treated extracorporeally to induce apoptosis and to create apoptotic cells therein. The extracorporeal treatment may be selected from the group consisting of antibodies, chemotherapeutic agents, radiation, extracorporeal photopheresis, ultrasound, proteins, and oxidizing agents. The cells, suspended in the subject's plasma or another suitable suspension medium, such as saline or a balanced mammalian cell culture medium, may then be administered as indicated below.

The numbers of apoptotic cells and/or bodies may be determined by published methods available in the scientific literature on the subject including the above-referenced articles. The numbers of such apoptotic cells required for administration to the subject to obtain the required clinical benefit may vary depending on the source of cells, the subject's condition, the age and weight of the subject and other relevant factors which are readily determinable by the those of ordinary skill in the art.

Thus, a non-limiting example of a process according to the present invention accordingly involves extraction of an aliquot of blood from the subject to be treated, separation of the white cells therefrom, and treatment of the white cells under apoptosis-causing conditions, so as to create a cellular composition in which significant numbers of the white cells therein have been apoptosed so as to create therein substantial numbers of apoptotic cells and/or apoptotic bodies. Then the treated composition is administered to the subject. More specifically, T-cells, isolated from the blood by known means, and suspended as above, may be used as a source of apoptotic cells.

### C. Treatment Methods

Accordingly, the present invention relates to methods for preventing, or reducing the potential severity of, organ transplant rejection in subjects by treating the transplant recipients and/or transplant donors with an effective amount of apoptotic cells prior to transplantation or prior to and after transplantation. The methods of the present invention are also applicable in preventing, or reducing the potential severity of, tissue transplant rejection using an effective amount of apoptotic cells. The methods of the present invention further relate to preventing, or reducing the potential severity of, GVHD in transplant recipients about to receive a transplant. The methods of the present invention further relate to preventing, delaying, or reducing the potential severity of one or more diseases including autoimmune diseases and atopic diseases.

More specifically, in the context of disease states, an effective amount of apoptotic cells may be administered, for example, before the clinical manifestation of a symptom associated with an autoimmune disease, before the clinical manifestation of a symptom associated with an autoimmune reaction, or before the clinical manifestation of a symptom associated with an atopic disease. In one embodiment, an effective amount of apoptotic cells may be administered to a subject predisposed to, for example, an autoimmune disease, before the subject manifests a clinical symptom associated with the autoimmune disease. More specifically, an effective amount of apoptotic cells may be administered to a subject predisposed to an autoimmune disease before the clinical manifestation of a symptom associated with the autoimmune disease that is treatable by known therapies. In another embodiment, an effective amount of apoptotic cells may be administered to a subject predisposed to, for example, an autoimmune disease, after the identification of a disease marker associated with the autoimmune disease. Indeed, an effective amount of apoptotic cells may be administered, for example, to a subject predisposed to a particular disease at any point between the identification of a disease marker associated with the disease and the clinical manifestation of a symptom associated with the disease. Such symptoms include symptoms described herein and known to those of ordinary skill in the art. *See generally* THE MERCK MANUAL (Mark H. Beers & Robert Berkow eds., 17th ed. 1999), which is entirely expressly incorporated herein by reference; *see also generally* THE MERCK MANUAL (Mark H. Beers & Robert Berkow eds., 17th ed. 1999), *available at* http://www.merck.com/pubs/mmanual. Symptoms and the clinical manifestation thereof may also be identified using guidelines issued, for example, from medical institutions such as The National Institutes of Health. As described herein and otherwise known to those of ordinary skill in the art, a disease marker may include, but is not limited to, genetic marker, serological marker, immunological marker, gene expression profile, protein expression profile, and polymorphism. Such disease markers may be used to identify whether a subject is predisposed to a particular disease

In the context of prevention, as a result of the administration of apoptotic cells, the subject, transplant recipient, or implant recipient, as the case may be, may not exhibit any symptoms of GVHD, transplant rejection, autoimmune disease, or atopic disease. In the context of reducing the potential severity of, for example, GVHD, the transplant recipient may exhibit one or more symptoms of GVHD but such symptoms of GVHD are less severe, or the transplant recipient may not exhibit all of the symptoms thereof, than transplant recipient that do not receive an effective amount of apoptotic cells prior to transplant. In another embodiment, the administration of apoptotic cells may delay the onset of a disease such as an autoimmune disease or an atopic disease. Indeed, the subject may exhibit one or more symptoms of a particular disease but the manifestation of such symptoms occur, if at all, at a time later and/or at a reduced severity, than the manifestation of symptoms in a subject that does not receive an effective amount of apoptotic cells.

Accordingly, the present invention relates to methods for preventing, or reducing the potential severity of, organ transplant rejection in transplant recipients. The methods of the present invention are also applicable in preventing, or reducing the potential severity of, tissue transplant rejection using an effective amount of apoptotic cells. The methods of the present invention further relate to preventing, delaying, or reducing the potential severity of one or more diseases including autoimmune diseases and atopic diseases.

The amount of apoptotic cells effective to prevent, delay, or reduce the potential severity of, for example, an autoimmune disease, may be selected in accordance with a variety of factors including type, species, age, weight, sex, medical condition of the subject; the severity of the condition to be treated; the route of administration; the renal and hepatic function of the subject; and the particular origin of the cell employed. A physician or veterinarian of ordinary skill can readily determine and prescribe the effective amount of the apoptotic cells required to prevent, counter, or arrest the progress of the condition.

In one embodiment, the apoptotic cells for use in the present invention may comprise not more than about 35 weight percent of necrotic cells based on the total weight of the apoptotic cells and necrotic cells; more specifically, not more than about 20 weight percent; and even more specifically, not more than about 10 weight percent. At these levels, the presence of such necrotic cells may not to significantly alter *in vivo* processes. In one embodiment, the apoptotic cells may be substantially free of necrotic cells and or bodies, for example, but not limited to, less than about 2 weight percent of necrotic cells/bodies.

In one embodiment, the number of viable cells selected for treatment to create apoptotic cells may be up to, but is not limited to, about 4x10⁹, specifically from about 1,000,000 to about 1,000,000,000 and more specifically from about 50,000,000 to about 150,000,000, for each administration to a human subject, *i.e.,* subject. From about 10% to about 90%, and specifically from about 30% to 70% of the total number of cells in the liquid suspension for administration may comprise apoptotic cells, the balance including, for example, necrotic cells. Accordingly, the amounts of apoptotic cells for administration may be those produced by subjecting these numbers of cells to the apoptosis-inducing conditions. When whole blood is used as the source of the cells to be subjected to the apoptosis-inducing conditions, these numbers of white cells may be obtainable in blood aliquots of volume up to, for example, about 400 ml, specifically up to about 100 ml. More specifically, about 50,000,000 to about 150,000,000 white cells may be present in blood aliquots of, for example, volume 10-30 ml.

In one embodiment, the volume of the aliquot of blood withdrawn from the subject, recipient, or donor for treatment to create apoptotic cells may be up to, but is not limited to, about 400 ml, specifically from about 0.1 to about 100 ml and more specifically from about 5 to about 15 ml. Accordingly, the amounts of apoptotic cells for administration may be those corresponding to the numbers derivable from the white blood cells, or isolated T-cells, contained in such quantities of whole blood, following subjection to apoptosis-inducing conditions.

The suspension of treated apoptotic cells and/or bodies for administration to the subject may be prepared in a biologically acceptable liquid suspending medium, such as the subject's serum or plasma, saline or balanced mammalian cell culture medium. The addition of other factors, such as cytokines, hormones, and products of stressed cells or other appropriate biologically active material may enhance the benefit of the administered apoptotic cellular materials. The aliquot may be introduced into the subject's, recipient's, or donor's body by any suitable method including, but not limited to, intramuscular injection, subcutaneous injection, mini-grafting, intra peritoneal injection, intra-arterial injection, intravenous injection and oral administration. The apoptotic cells may be delivered to the specific body organ and/or site by using any appropriate, known delivery system.

The effective amount of apoptotic cells of this invention may include a pharmaceutically acceptable excipient. Some examples of suitable excipients include sterile water, sterile saline, phosphate buffered saline, and the like.

When administered, the pharmaceutical compositions comprise an effective amount of apoptotic cells to induce a suitable prophylactic response in the subject predisposed to an autoimmune disease, atopic disease, or under going a transplant. The composition administered to a mammalian subject may comprise, for example, from about 10,000 to about 10,000,000 apoptotic cells per kilogram of body weight, from about 500,000 to 5,000,000, or from about 1,500,000 to about 4,000,000 apoptotic cells per kg body weight. The specific dose employed may be dependent upon the age, weight, and severity of, onset of, or type of disease in the treated subject, all of which are within the skill of art.

Moreover, the administration of apoptotic cells may be optimized using a pharmacokinetic/pharmacodynamic modeling system. For example, one or more dosage regimens may be chosen and a pharmacokinetic/pharmacodynamic model may be used to determine the pharmacokinetic/pharmacodynamic profile of one or more dosage regimens. Next, one of the dosage regimens for administration may be selected which achieves the desired phannacokinetic/pharmacodynamic response based on the particular pharmacokinetic/pharmacodynamic profile. *See* WO 00/67776, which is entirely expressly incorporated herein by reference.

In one embodiment, for prophylaxis of GVHD, transplant rejection, autoimmune diseases, and/or atopic disease, the subject, recipient, and/or donor may be given a course of treatments with apoptotic cells. For example, each course of treatment may involve administration to the subject, recipient, and/or donor about 1 to about 6 aliquots of suspended cellular material, as described above. Booster treatments as described herein may advantageously be used. To maintain the desired effects, the subject, recipient, and/or donor may undergo booster treatments, with a further course of administration of aliquots of suspended apoptotic cells as described above, at intervals of, for example, three to four months.

In a specific embodiment, an effective amount of apoptotic cells may be administered prophylacticly and on a one-time or on a periodic basis, specifically in the context of the treatment of subjects predisposed to a T-cell response associated with an autoimmune reaction, and specifically in the context of an autoimmune disease. In an alternative embodiment, an effective amount of apoptotic cells may be administered prophylacticly and on a one-time or on a periodic basis, specifically in the context of the treatment of subjects predisposed to an atopic disease. In another specific embodiment, an effective amount of apoptotic cells may be administered prophylacticly and on a one-time or on a periodic basis, specifically in the context of preventing, or reducing the potential severity of, graft-versus-host disease in transplant recipients. In yet another specific embodiment, an effective amount of apoptotic cells may be administered prophylacticly and on a one-time or on a periodic basis, specifically in the context of preventing, or reducing the potential severity of, organ or tissue transplant rejection in transplant recipients.

In all of the applications discussed herein, an effective amount of apoptotic cells may be administered to a subject, recipient, and/or donor at an appropriate time and on an appropriate schedule, as determined by one of ordinary skill in the art. One skilled in the art would be able to determine such appropriate times and periods based on, for example, the sex, age, race, general health, disease state, etc., of the subject, as well as desired outcomes based on, for example, the desired length or amount of prevention, delay, or reduction of, for example, an autoimmune disease, an atopic disease, a T-cell response, GVHD, organ transplant rejection, or tissue transplant rejection.

In the context of subjects in whom it may be determined whether they are predisposed to autoimmune or atopic diseases that, for example, may be assessed by genetic testing procedures, *i.e.,* genetic testing of HLA haplotype in cord blood stem cells obtained from newborn infants, through SNPs or other genetic markers associated with autoimmune disease, or by other known blood or serum tests indicative of autoimmune disease, the methods of the present invention may include the administration of an effective amount of apoptotic cells to the subject at an appropriate time and for an appropriate period after the determination of said predisposition.

Indeed, the methods of the present invention include the administration of an effective amount of apoptotic cells to a subject, recipient, and/or donor in the present context one or more times and over one or more periods of time. The one or more times include one to at least one hundred or more times, one to at least fifty times, one to at least twenty five times, one to at least ten times, one to at least five times, one to four, one to three and two times and any combination of said times with one or more of periods of time, which include daily, weekly, monthly, yearly and any combination thereof.

In one embodiment, an effective amount of apoptotic cells may be administered at least one time at least 12 months, at least 11 months, at least 10 months, at least 9 months, at least 8 months, at least 7 months, at least 6 months, at least 6 months, at least 5 months, at least 4 months, at least 3 months, at least 2 months, or at least 1 month before transplant, before the clinical manifestation of a symptom associated with an autoimmune disease, before the clinical manifestation of a symptom associated with an autoimmune reaction, or before the clinical manifestation of a symptom associated with an atopic disease.

In another embodiment, an effective amount of apoptotic cells may be administered at least one time at least 30 days, at least 29 days, at least 28 days, at least 27 days, at least 26 days, at least 25 days, at least 24 days, at least 23 days, at least 22 days, at least 21 days, at least 20 days, at least 19 days, at least 18 days, at least 17 days, at least 16 days, at least 15 days, at least 14 days, at least 13 days, at least 12 days, at least 11 days, at least 10 days, at least 9 days, at least 8 days, at least 7 days, at least 6 days, at least 5 days, at least 4 days, at least 3 days, at least 2 days, or at least 1 day before transplant, before the clinical manifestation of a symptom associated with an autoimmune disease, before the clinical manifestation of a symptom associated with an autoimmune reaction, or before the clinical manifestation of a symptom associated with an atopic disease.

In the context of bone marrow, organ, and tissue transplantation, an effective amount of apoptotic cells may be administered according to a schedule that includes, but is not limited to, two consecutive days, one week prior to transplant; two days, one week prior to transplant; three consecutive days, one week prior to transplant; three days, one week prior to transplant; two days a week for two weeks before transplant; and three days a week for three weeks before transplant.

Moreover, in the context of preventing and/or reducing the potential severity of GVHD and/or rejection in the bone marrow, stem cell, organ, and tissue transplants described herein and otherwise known to those of ordinary skill in the art, an effective amount of apoptotic cells may be administered after the transplant similar to the schedules described above and more particularly, weekly, monthly, twice a month, three times a month, every other month, every six months, every nine months, and yearly. Such an exemplary schedule may also be utilized in the context of preventing, delaying, or reducing the potential severity of one or more diseases including, for example, autoimmune diseases and atopic diseases.

For example, an effective amount of apoptotic cells may be administered at least once a week over the course of several weeks. In one embodiment, an effective amount of apoptotic cells may be administered at least once a week over several weeks to several months. In another embodiment, an effective amount of apoptotic cells may be administered once a week over four to eight weeks. In yet another embodiment, an effective amount of apoptotic cells may be administered once a week over four weeks.

More specifically, an effective amount of apoptotic cells may be administered at least once a day for about 2 days, at least once a day for about 3 days, at least once a day for about 4 days, at least once a day for about 5 days, at least once a day for about 6 days, at least once a day for about 7 days, at least once a day for about 8 days, at least once a day for about 9 days, at least once a day for about 10 days, at least once a day for about 11 days, at least once a day for about 12 days, at least once a day for about 13 days, at least once a day for about 14 days, at least once a day for about 15 days, at least once a day for about 16 days, at least once a day for about 17 days, at least once a day for about 18 days, at least once a day for about 19 days, at least once a day for about 20 days, at least once a day for about 21 days, at least once a day for about 22 days, at least once a day for about 23 days, at least once a day for about 24 days, at least once a day for about 25 days, at least once a day for about 26 days, at least once a day for about 27 days, at least once a day for about 28 days, at least once a day for about 29 days, at least once a day for about 30 days, or at least once a day for about 31 days.

Alternatively, an effective amount of apoptotic cells may be administered about once every day, about once every 2 days, about once every 3 days, about once every 4 days, about once every 5 days, about once every 6 days, about once every 7 days, about once every 8 days, about once every 9 days, about once every 10 days, about once every 11 days, about once every 12 days, about once every 13 days, about once every 14 days, about once every 15 days, about once every 16 days, about once every 17 days, about once every 18 days, about once every 19 days, about once every 20 days, about once every 21 days, about once every 22 days, about once every 23 days, about once every 24 days, about once every 25 days, about once every 26 days, about once every 27 days, about once every 28 days, about once every 29 days, about once every 30 days, or about once every 31 days.

An effective amount of apoptotic cells may alternatively be administered about once every week, about once every 2 weeks, about once every 3 weeks, about once every 4 weeks, about once every 5 weeks, about once every 6 weeks, about once every 7 weeks, about once every 8 weeks, about once every 9 weeks, about once every 10 weeks, about once every 11 weeks, about once every 12 weeks, about once every 13 weeks, about once every 14 weeks, about once every 15 weeks, about once every 16 weeks, about once every 17 weeks, about once every 18 weeks, about once every 19 weeks, about once every 20 weeks.

Alternatively, an effective amount of apoptotic cells may be administered about once every month, about once every 2 months, about once every 3 months, about once every 4 months, about once every 5 months, about once every 6 months, about once every 7 months, about once every 8 months, about once every 9 months, about once every 10 months, about once every 11 months, or about once every 12 months.

Alternatively, an effective amount of apoptotic cells may be administered at least once a week for about 2 weeks, at least once a week for about 3 weeks, at least once a week for about 4 weeks, at least once a week for about 5 weeks, at least once a week for about 6 weeks, at least once a week for about 7 weeks, at least once a week for about 8 weeks, at least once a week for about 9 weeks, at least once a week for about 10 weeks, at least once a week for about 11 weeks, at least once a week for about 12 weeks, at least once a week for about 13 weeks, at least once a week for about 14 weeks, at least once a week for about 15 weeks, at least once a week for about 16 weeks, at least once a week for about 17 weeks, at least once a week for about 18 weeks, at least once a week for about 19 weeks, or at least once a week for about 20 weeks.

Alternatively an effective amount of apoptotic cells may be administered at least once a week for about 1 month, at least once a week for about 2 months, at least once a week for about 3 months, at least once a week for about 4 months, at least once a week for about 5 months, at least once a week for about 6 months, at least once a week for about 7 months, at least once a week for about 8 months, at least once a week for about 9 months, at least once a week for about 10 months, at least once a week for about 11 months, or at least once a week for about 12 months.

### III. Transplantation and Rejection

Modem immunosuppressive regimens, consisting of cyclosporine-based triple-drug therapy, with or without monoclonal or polyclonal antibodies, have dramatically increased survival among organ-transplant recipients. However, these regimens improve graft survival by nonspecific immunosuppression, leaving the host at increased risk for opportunistic infection and the development of malignant tumors and vulnerable to the adverse effects of these drugs. Moreover, considerable morbidity and mortality persist as a result of acute episodes of organ rejection, particularly in the first few months after transplantation, and or chronic forms of rejection such as graft vasculopathy. treatments directed at suppressing donor-specific T-cell clones in the recipient have the potential to decrease graft rejection without increasing the toxicity of immunosuppressive drugs.

Rose et al. used photopheresis to treat four patients undergoing cardiac transplantation who had elevated levels of non-donor-specific anti-HLA antibodies and who were at high risk for rejection. Rose et al., 11 J. HEART LUNG TRANSPLANT. 746 (1992). They found a decrease in the levels of non-donor-specific anti-HLA antibodies and a relatively low incidence of rejection. Constanzo-Nordin et al. used a single photopheresis treatment in patients with hemodynamically stable cardiac rejection and compared the results with those in a control group that received high-dose corticosteriods for three days. Constanzo-Nordin et al., 86(Suppl. II) CIRCULATION II-242 (1992). Although less successful than corticosteroids in resolving rejection episodes, photopheresis alone was capable of reversing acute cellular ejection in most cases and was associated with fewer post-treatment infections.

Chronic graft-versus-host disease (cGVHD) occurs in 30% to 60% of patients after allogeneic bone marrow transplantation (BMT). Minor antigen mismatches cause donor T-cell activation against recipient tissues, and antigen-presenting cells (APCs) are essential in initiating this process. Natural killer (NK) cells have been shown to be suppressed in most patients with active cGVHD. Clinical management of cGVHD includes the use of steroids, cyclosporine, and FK506 (tacrolimus). Extracorporeal photopheresis (ECP) has been shown to be effective in the treatment of cutaneous T-cell lymphoma (CTCL), some autoimmune diseases, and rejection of solid-organ grafts. Several small studies found improvement in the skin and visceral manifestations of cGVHD after ECP. One proposed reason for the effects of ECP in CTCL and autoimmune diseases is that exposure to ultraviolet light and 8-methoxypsoralen induces apoptosis in a small subset of circulating clonal tumor or autoreactive T-lymphocytes, thus stimulating a cytotoxic T-cell (CTL) response against the clone. We analyzed the clinical and immunologic effects of ECP in patients with steroid-refractory cGVHD and found that response correlated with normalization of the ratio of CD4 to CD8 cells, an increase in CD3-CD561 natural killer (NK) cells, and a decrease in circulating CD801 and CD1231 APCs. These results suggest that in patients with ongoing alloreactivity and cGVHD, ECP may interfere with the presentation of alloantigens by altering both effectors and APCs, resulting in establishment of immune tolerance.

### A. Generally

Since the first successful kidney transplantation more than 40 years ago, great expansion of transplantation in treating end-stage organ failure has occurred. Projected survival rates are improved, and many organs are now transplanted. This expansion is attributed to new, more selective immunosuppressants; improved measures for detecting preexisting immunity to given donors; better patient selection; earlier intervention; improved surgical technique; earlier and more accurate detection of rejection episodes; and a better understanding of rejection.

However, transplantation is still somewhat limited, mainly because of rejection, which can destroy the tissue soon after transplantation except in special circumstances (*e.g.*, most grafts of cornea and cartilage and transplants between identical twins). Slower, chronic rejection has also emerged as a significant factor in the long-term survival and functional status of transplanted organs. Limitations in the availability of human donor organs also continue to be important.

Transplants are categorized by site and genetic relationship between the donor and recipient. An orthotopic tissue or organ graft is transferred to an anatomically normal recipient site (*e.g.*, in a heart transplant). Transfer to an anatomically abnormal site is called heterotopic (*e.g.*, transplantation of a kidney into the iliac fossa of the recipient). An autograft is the transfer of one's own tissue from one location to another (*e.g.*, a bone graft to stabilize a fracture). A syngeneic graft (isograft) is a graft between identical twins; an allograft (homograft) is a graft between genetically dissimilar members of the same species. Xenografts (heterografts) are transplants between members of different species. Xenografts in general are confined to fixed, nonviable material, *e.g.*, porcine heart valves. Improved immunosuppression may allow for successful organ xenografts to help overcome the current critical shortage of donors.

With rare exceptions, transplants are allografts from either living relatives (and occasionally unrelated individuals) or cadaveric donors. Living donors are used mainly in kidney and bone marrow transplantation, but segmental liver, pancreas, and lung transplants are increasingly being donated by the recipients' living relatives. Acceptance of the concept of brain death has increased the use and demand for cadaveric organs, making it common to procure many organs from a single donor. Nevertheless, the need for organs far exceeds the number available from relatives of patients, and the number of patients waiting for organ transplants continues to grow.

### 1. Immunobiology of Rejection

Allografts may be rejected through either a cell-mediated or a humoral immune reaction of the recipient against transplantation (histocompatibility) antigens present on the membranes of the donor's cells. The strongest antigens are governed by a complex of genetic loci termed human leukocyte group A (HLA) antigens; together with the ABO blood group antigens, they are the chief transplantation antigens detectable in humans. Because transplantation antigens may be identified by their effects *in vitro,* tissue typing is possible.

The lymphocyte (cell)-mediated immune reaction against transplantation antigens, *i.e.,* GVHD, is the principal mechanism of acute rejection. A delayed hypersensitivity reaction similar to the tuberculin reaction, GVHD causes graft destruction days to months after transplantation and is characterized histologically by mononuclear cellular infiltration of the allograft, with varying degrees of hemorrhage and edema. Usually, vascular integrity is maintained, although the arterial endothelium appears to be a primary target of GVHD. Cell-mediated rejection may be reversed in many cases by intensifying immunosuppressive therapy. After successful reversal of an acute rejection episode, severely damaged elements of the graft heal by fibrosis and the remainder of the graft appears normal. After resolution of acute rejection, the allograft commonly survives for prolonged periods, even though immunosuppressive drug dosages are reduced to very low levels. This process of graft adaptation is most likely explained by the loss of highly immunogenic passenger leukocytes, including dendritic cells, and maybe by the development of donor-specific suppression of the recipient's immune response.

Late graft deterioration occurs occasionally, and this chronic type of rejection often progresses insidiously despite increased immunosuppressive therapy. It is thought to be due, in large part, to antibody-mediated damage. The pathologic picture differs from that of acute rejection. The arterial endothelium is primarily involved, with extensive proliferation that may gradually occlude the vessel lumen, resulting in ischemia and fibrosis of the graft.

The role of humoral antibody in graft rejection is evident when the recipient has been presensitized (by pregnancy, blood transfusion, or previous transplant) to HLA in the graft. Transplantation in these circumstances almost invariably leads to antibody-mediated hyperacute rejection, causing destruction of the graft within hours or even minutes after revascularization. This rejection reaction is characterized by small vessel thrombosis, and graft infarction is unresponsive to known immunosuppressive therapies. Liver grafts seem to be less susceptible to such antibody-mediated hyperacute rejection. The role of humoral antibody in more delayed graft destruction is probably also important but is still unclear.

A result similar to antibody-mediated rejection usually occurs if a graft is transplanted in defiance of the blood group barriers normally observed in blood transfusions. Therefore, pretransplant evaluation generally includes verification of ABO compatibility between donor and recipient and the existence of a negative cross-match for tissue antibodies (lack of significant reactivity between donor WBCs and recipient serum *in vitro*), as well as tissue typing for HLA compatibility.

### 2. Tissue Compatibility

Histocompatibility (or tissue) typing of peripheral blood or lymph node lymphocytes is performed before transplantation in many centers to identify HLA serologically and, by appropriate donor selection, to minimize the antigenic differences between donor and recipient. HLA matching has significantly improved functional survival of transplants between related individuals. Results between unrelated individuals also show a correlation with the degree of HLA compatibility, although much less clearly, since the complex histocompatibility differences in an outbred population introduce many more variables.

In large multicenter studies of kidney transplantation, the degree of HLA compatibility is one of many factors correlating with the survival of cadaveric grafts, especially when survival is evaluated at long intervals after transplantation.

In contrast, in several single-center series, the role of HLA compatibility is not great. Thus, practice in the USA is to share cadaveric kidneys throughout the country only if there is a complete HLA match with a prospective donor. Otherwise, the organs are transplanted into a recipient in the local region of the donor. The role of HLA matching in heart, liver, pancreas, and lung transplantation has not been evaluated extensively because these organs must be transplanted quickly, before tissue typing may be completed. In heart and pancreas transplantation, graft survival appears to correlate with HLA compatibility. In particular, class n antigen matching may lead to better long-term graft survival.

Detecting specific presensitization of the potential recipient against donor antigens is very important in determining whether transplantation should be performed. Presensitization most commonly results from prior exposure to donor antigens by means of blood transfusions, prior transplantations, or pregnancies; it is evaluated by a lymphocytotoxic test between recipient serum and donor lymphocytes in the presence of complement. Other techniques are also available. A positive cross-match usually indicates antibodies in the recipient's serum directed against donor class I antigens. This is generally considered a contraindication to transplantation because hyperacute rejection is common.

The risk/benefit of blood transfusions in dialysis patients looking toward kidney transplantation is controversial. Transfusions to patients with end-stage renal failure can sensitize them to a potential kidney transplant. However, allograft survival improved in recipients who received transfusions and did not become sensitized. Some altered form (*e.g.*, suppression) of immune responsiveness seemed to be induced by the transfusions. With the use of cyclosporine, the beneficial effect of pretransplant transfusions seems to be greatly reduced. Because of the risk of transmission of infectious diseases (*e.g.*, hepatitis and HIV) and the availability of biosynthetic erythropoietin, many centers do not routinely insist on pretransplant transfusion of organ recipients.

### 3. Immunosuppression

Except with isografts, immunosuppressive therapy can rarely be stopped completely after transplantation. However, intensive immunosuppressive therapy is usually required only during the first few weeks after transplantation or during a rejection crisis. Subsequently, the graft often seems to become accommodated and may be maintained with relatively small doses of immunosuppressants and fewer adverse effects.

### 4. Immunosuppressive Drugs

Immunosuppressants are used to control the rejection reaction and are primarily responsible for the success of transplantation. However, these drugs suppress all immunologic reactions, thus making overwhelming infection the leading cause of death in transplant recipients.

Prednisolone (IV), a corticosteroid, usually is given in a high dose (2 to 20 mg/kg) at the time of transplantation and then reduced gradually to a maintenance dose of 0.2 mg/kg/day indefinitely. Several months after transplantation, the drug may be given on alternate days to reduce adverse effects, which is particularly important in growing children. Stopping prednisolone may become possible in some multidrug regimens, but this somewhat increases the risk of rejection. If rejection occurs, the dose is sharply increased despite increased adverse effects.

Azathioprine, an antimetabolite, is usually given beginning at the time of transplantation. Oral or IV dosages of 1 to 2.5 mg/kg/day generally are tolerated indefinitely. The primary toxic effects are bone marrow depression and (rarely) hepatitis. Since the advent of cyclosporine, many transplant centers use azathioprine and low doses of cyclosporine together.

Cyclophosphamide, an alkylating agent, is used in patients who do not tolerate azathioprine. Equivalent doses are apparently equal in immunosuppressive activity. Cyclophosphamide also is used in much larger doses as one of the primary immunosuppressive drugs in bone marrow transplantation. Severe toxicity is common, with hemorrhagic cystitis, alopecia, and infertility.

Cyclosporine, a fungal metabolite, has been used as the primary immunosuppressant in place of antimetabolites in transplantation during the last two decades. Unlike antimetabolites, cyclosporine spares the bone marrow, acting instead more selectively to inhibit T-cell proliferation and activation. The exact molecular mechanism of action is unknown.

Although cyclosporine may be given alone, it usually is given with other drugs, such as azathioprine and prednisone, allowing a rapid reduction in corticosteroid dosage. Initial dosages of cyclosporine are 6 to 12 mg/kg/day po, reduced to a maintenance level of 3 to 5 mg/kg/day soon after transplantation.

Counterbalancing cyclosporine's efficacy is its considerable toxicity. Nephrotoxicity, hepatotoxicity, refractory hypertension, increased incidence of neoplasms, and several less serious adverse effects (*e.g.,* gum hypertrophy and hirsutism) can occur. B-cell lymphomas and polyclonal B-cell lymphoproliferative disorders are related to Epstein-Barr virus (EBV) activation and have been observed more often in patients receiving high doses of cyclosporine or combinations of cyclosporine and other immunosuppressants directed at T cells. Nephrotoxicity is a special concern. Cyclosporine appears to cause vasoconstriction of the afferent preglomerular arterioles, leading ultimately to myonecrosis and refractory glomerular hypoperfusion. Long-term use of cyclosporine may cause chronic irreversible kidney failure. Although the level of cyclosporine in the blood may be measured easily, there is no adequate means of determining the therapeutically effective amount of cyclosporine necessary for a given patient. Moreover, blood levels of cyclosporine do not correlate reliably with its toxic effects.

Tacrolimus is an immunosuppressive drug for liver transplant recipients. It is a byproduct released in the growth of a cultured organism *(Streptomyces tsukubaensis).* Its adverse effects are similar to cyclosporine, although gum hypertrophy and hirsutism are less prominent. It also may induce diabetes. Treatment may be started at the time of transplantation or later, and it may be given either IV or orally. Dosage usually begins at 0.15 to 0.30 mg/kg/day when administered orally and 0.05 to 0.1 mg/kg/day if given IV. Regulation of dosage is aided by periodic tests of blood levels, and knowledge of undesirable drug interactions is essential. Tacrolimus may be useful for patients in whom cyclosporine has proven unduly toxic or ineffective.

### 5. Other Immunosuppressive Therapies

Attempts to obtain more selective immunosuppression include the use of antisera to human lymphocytes or thymus cells in an effort to suppress cellular immunity while leaving the recipient's humoral immunologic response intact. Monoclonal antibodies and irradiation are also used. Immunosuppressive therapies under development include chemical agents of various types and biologically produced substances, such as antibodies selected for their special properties.

Antilymphocyte globulin (ALG) and antithymocyte globulin (ATG) are useful adjuncts, allowing other immunosuppressants to be used in lower, less toxic doses. The use of ALG and ATG at the time of transplantation may be beneficial because of decreased incidence of rejection; moreover, their use allows a delay in starting cyclosporine therapy and its toxicity. The use of ALG or ATG to control established rejection episodes has clearly led to improved graft survival rates. Possible adverse reactions to heterologous sera include anaphylactic reactions, serum sickness, or antigen-antibody-induced glomerulonephritis. Using highly purified serum fractions, giving them IV, and combining them with other immunosuppressive agents have greatly reduced the incidence of these reactions.

Monoclonal antibodies against T cells offer a much greater concentration of specifically reactive antibody molecules and fewer irrelevant serum proteins compared with polyclonal antiglobulin fractions. The murine monoclonal antibody, OKT3, can reverse rejection. OKT3 binds to the T-cell antigen-receptor complex (TCR/CD3), leading initially to nonspecific T-cell activation and a prominent clinical syndrome caused by the ensuing cytokine release characterized by fevers, rigors, myalgia, arthralgia, and CNS and GI irritation. Subsequently, OKT3 blocks binding of the TCR to antigen and results in modulation of the entire TCR/CD3 complex from the T-cell surface. OKT3 5 mg/day IV for 10 to 14 days is given at the time of an acute rejection episode. OKT3 has also been used at the time of transplantation; like ALG, it appears to both delay the onset and reduce the incidence of rejection episodes. However, the benefits gained from prophylaxis with this agent must be weighed against its toxic adverse effects, the risk of overimmunosuppression, and the risk of a patient's developing neutralizing antibodies against the heterologous monoclonal antibody that would render it ineffective if needed later to treat a rejection episode. As with the use of high-dose cyclosporine, an increased incidence of EBV-induced B-cell lymphoproliferative disorders has been seen with repeated use of OKT3.

As the roles of distinct T-cell subpopulations in the rejection reaction are better understood, the use of monoclonal antibodies that react with specific subpopulations will allow even greater selectivity in immune suppression. For example, clinical trials with monoclonal antibodies that react with antigens present only on activated T cells (sparing T cells not participating in the rejection reaction) are in progress.

Irradiation for immunosuppression is of limited use in transplantation. The graft and local recipient tissues are sometimes irradiated, either as an adjunct prophylactic immunosuppressive measure or during treatment of established rejection. The total dose (usually 4 to 6 Gy) is below the threshold that might cause serious radiation injury of the graft itself. In treating refractory leukemia, whole-body irradiation in doses of 12 Gy combined with chemotherapy destroys the host's immunologic capability (and residual leukemic cells). Such irradiation is followed by a bone marrow allograft.

Interest in irradiation has been restimulated by the following observation: Treatment directed (with suitable shielding, such as that used for Hodgkin's disease) toward all lymphoid centers (total lymphatic irradiation) appears to provide a profound but relatively safe suppression of cellular immunity. This may be mediated at first by suppressor T cells, which may be detected after total lymphatic irradiation. In a few patients, later clonal deletion of specific antigen-reactive cells has been seen. Application of total lymphatic irradiation to transplantation is promising, but experimental.

### 6. Immunologic Tolerance

Some degree of tolerance appears to be achieved with the currently used nonspecific immunosuppressive regimens. However, transplant biologists hope to provide specific, selective suppression of the recipient's response only to the foreign antigens on the graft, allowing discontinuation of nonspecific immunosuppression. In animals, tolerance to antigens encountered in the neonatal period when the immune system is still maturing has been relatively easy to achieve; however, adult animals have been for the most part refractory to the induction of antigen-specific tolerance. Tolerance to foreign antigens in adult animals has required careful selection of conditions (*e.g.*, antigen dose, route of injection, and short-term use of other immunosuppressants in toxic doses). Increasingly reliable methods for producing antigen-specific unresponsiveness are being devised for clinical transplantation and may reach clinical trials in the near future.

### B. Kidney Transplantation

All patients with terminal renal failure should be considered for transplantation except those at risk from another life-threatening condition. Kidney transplantation is now common: For all children older than 6 months old with renal failure, kidney transplantation is the treatment of choice. A successful transplant not only frees the patient from lengthy dialyses, but also provides the kidney's other metabolic functions (*e.g.*, erythropoietic stimulation and calcium homeostasis).

Patient survival one year after a transplant from a living related donor is greater than 95%, with about 90% of the allografts functioning. Subsequently, an annual graft loss of 3 to 5% is observed, including that due to patient death. The 1-year patient survival rate after a transplant from a cadaver is about 90%, and graft survival ranges between 70 and 90% at various centers. In subsequent years, some 5 to 8% of grafts are lost annually. Several kidney transplant recipients now have grafts that have functioned for > 30 years. Although transplantation in patients > 55 years was thought to carry an unacceptable risk, careful use of immunosuppressive drugs and close immunologic monitoring allows for allografting in selected patients in the 7th decade of life and even beyond.

Donor selection and kidney preservation: Kidney allografts are obtained from living relatives or cadavers, excluding donors with a history of hypertension, diabetes, or malignant disease (except possibly those with neoplasms originating in the CNS). Potential living donors are also evaluated for emotional stability, normal bilateral renal function, freedom from other systemic disease, and histocompatibility. A living donor relinquishes reserve renal capacity, may have complex psychologic conflicts, and faces some morbidity from nephrectomy; yet the significantly improved long-term prognosis for a recipient of a well-matched allograft usually justifies consideration of the related donor.

Over 2/3 of kidney transplants are from cadavers of previously healthy persons who sustained brain death but maintained stable cardiovascular and renal function. After brain death, the kidneys are removed as soon as is practical and cooled by perfusion. For simple hypothermic storage, special cooling solutions containing relatively large concentrations of poorly permeating substances (*e.g*., mannitol or hetastarch) and electrolyte concentrations approximating intracellular levels are used to flush the kidney, which is then stored in an iced solution. Kidneys preserved this way usually function well if transplanted within 48 hours. By using the more complex technique of continuous pulsatile hypothermic perfusion with an oxygenated, plasma-based perfusate, kidneys have been successfully transplanted after *ex vivo* perfusion of as long as 72 hours.

Pretransplantation preparation and transplantation procedure: Pretransplantation preparation includes hemodialysis to ensure a relatively normal metabolic state and provision of a functional, infection-free lower urinary tract. Bladder reconstruction, nephrectomy of infected kidneys, or construction of an ileal loop for draining the allograft may be required. The transplanted kidney usually is placed retroperitoneally in the iliac fossa. Vascular anastomoses are made to the iliac vessels, and ureteral continuity is established.

Rejection management: Despite prophylaxis with immunosuppressants begun just before or at the time of transplantation, most recipients undergo one or more acute rejection episodes in the early post-transplant period. Rejection is suggested by deterioration of renal function, hypertension, weight gain, tenderness and swelling of the graft, fever, and appearance in the urine sediment of protein, lymphocytes, and renal tubular cells. If the diagnosis is unclear, percutaneous needle biopsy is performed for histopathologic evaluation of tissue. In cyclosporine-treated recipients, drug-induced nephrotoxicity is sometimes difficult to differentiate from rejection, even with biopsy. Intensified immunosuppressive therapy usually reverses rejection. If it cannot be reversed, immunosuppressive therapy is tapered, and the patient returns to hemodialysis to await a subsequent transplant. Nephrectomy of the transplanted kidney is necessary if hematuria, graft tenderness, or fever results from the rejection response with withdrawal of immunosuppressants.

Most rejection episodes and other complications occur within 3 to 4 months after transplantation; most patients then return to more normal health and activity. However, unless toxicity or severe infection occurs, immunosuppressants must be maintained, since even brief cessation may precipitate rejection.

Complications: Some patients suffer irreversible chronic graft rejection. Other late complications include drug toxicity, recurrent underlying renal disease, adverse effects of prednisone, and infection. In addition, the incidence of malignancy in renal allograft recipients is increased. The risk of epithelial carcinoma is 10 to 15 times higher than normal; of lymphoma, about 30 times. Management of these neoplasms is similar to that of cancer in nonimmunosuppressed patients. Reduction or interruption of immunosuppression is not generally required in treating squamous cell epitheliomas, but is recommended for more aggressive tumors and lymphomas. B-cell lymphomas associated with EBV have become much more frequent in transplant recipients in recent years. Although individual associations with the use of cyclosporine and with protocols using ALG or OKT3 have been postulated, the more likely correlation is with the overall degree of immunosuppression achieved with more potent immunosuppressants.

### C. Liver Transplantation

Liver transplantation is required for end-stage hepatic dysfunction. Survival rates have improved markedly with advances in surgical technique, the use of cyclosporine, and better patient selection. One-year survival rates have climbed from 30% to 80 or 85% depending on the preoperation status of patients. Late deaths have been rare and often have been attributed to recurrent disease (*e.g.*, cancer, hepatitis) rather than to post-transplant difficulties. Increasing numbers of patients have now had functioning allografts for more than 2 decades. Successful transplant recipients can return to normal social and work activities.

The advent of cyclosporine has permitted early reduction of corticosteroid dosage, resulting in better postoperative healing and greater resistance to overwhelming infection. With better results, more patients are being accepted for transplantation before reaching a terminal debilitated state. If a graft fails, liver retransplantation is possible. At present, 5 to 15% of liver transplant patients who would have died receive second transplants, with a success rate of > 60%. Improved success rates are due not only to cyclosporine exclusively, but also to many details of patient management.

Indications for liver transplantation have been mainly diseases causing *chronic* liver failure. In *acute* failure, prognosis is difficult to assess, time to obtain a suitable donor is often inadequate, and the risk of recurrent viral infection in the transplanted liver is substantial. This is encountered with certain intoxications, *e.g.,* with acetaminophen. Nonetheless, if a liver may be procured expeditiously, transplantation can save patients with acute, fulminant liver failure even after the onset of hepatic coma.

End-stage chronic hepatitis and biliary cirrhosis are the most frequent indications for liver transplantation in adults, as are biliary atresia and inborn metabolic deficiencies in children. Patients with a primary hepatic malignancy have a relatively poor prognosis; the tumor often recurs after transplantation in the immunosuppressed patient, leading to a 1-year survival rate of about 20%. However, if hepatic carcinoma, especially the fibrolamellar type, is confined to the liver, long-term tumor-free survival has resulted.

Donor selection: Cadaveric donors of livers must be of previously healthy persons who are size- and ABO-matched to the recipient. A history of hepatic dysfunction, hypotension requiring prolonged vasopressor support, systemic infection, or evidence of liver ischemia or damage suggested by elevation of hepatic enzymes precludes organ use.

Liver preservation and transplantation procedures: Long-term extracorporeal hepatic preservation methods are not available; livers are stored in cold solutions generally for 8 to 16 h after removal. Some grafts stored for > 24 h are successfully transplanted, but the incidence of graft nonfunction increases with prolonged storage. Tissue typing and cross-matching usually are performed retrospectively. Recipient hepatectomy, which can occasionally result in intraoperative blood loss of > 20 U, is the most demanding part of the transplant procedure and is often performed in the face of portal hypertension and after previous hepatobiliary surgery. Transfusion requirements in adult liver transplant recipients are often less than 8 to 10 units. To complete the transplant, five anastomoses are required: suprahepatic vena cava, intrahepatic vena cava, portal vein, hepatic artery, and biliary duct. Heterotopic placement of the liver, which provides an auxiliary liver, obviates several technical difficulties. However, because results have been discouraging, this technique has only been used experimentally. Because appropriately size-matched donors for children are in short supply, reduced-size grafts consisting of a segment of an adult liver are being used; results appear to be equivalent to the full-size pediatric grafts. Transplantation of the left-lateral segment of a parent's liver to the child has also been performed successfully, but the ultimate role of living relatives as donors awaits further evaluation.

Rejection management: Surprisingly, liver allografts are less aggressively rejected than other organ allografts. For example, hyperacute rejection of a liver transplant does not occur invariably in patients who were presensitized to HLA antigens or ABO incompatibilities. The reasons for this are unknown. However, when either fulminant acute rejection or chronic rejection is refractory to immunosuppressive therapy, retransplantation is the treatment. The vanishing bile duct syndrome, characterized by intrahepatic cholestasis with preserved hepatocellular function, is a pattern of chronic rejection.

Typical immunosuppressive therapy in an adult is cyclosporine usually given IV at 4 to 6 mg/kg/day starting at the time of transplantation and then 8 to 14 mg/kg/day po when feeding is tolerated. Doses are adjusted downward if renal dysfunction occurs, and blood levels are used as approximate measures of adequate dosage. Children often require higher doses to maintain adequate blood levels. If biliary drainage via a T tube is used postoperatively, then higher doses may be required because of the loss of cyclosporine via the bile. Typically, methylprednisolone IV or prednisone po is started at about 10 mg/kg/day and reduced in a stepwise fashion to a maintenance dosage of 0.2 mg/kg/day. Azathioprine at 1 to 2 mg/kg/day po or IV is also sometimes used.

Mild acute rejection episodes may be self-limited. Rejection is suspected by development of hepatomegaly, light-colored bile (seen in T-tube drainage) or stools, and complaints of anorexia, right-sided pain, and fever. Jaundice and elevated serum levels of hepatic enzymes are corroborative findings. Needle biopsy can provide pathologic confirmation. Suspected rejection episodes are treated with IV corticosteroids, antithymocyte globulin (ATG), or monoclonal antibodies. A variety of complications must be expected, including those attributable to the complex operation itself, in addition to rejection and the consequences of attempts to control it.

### D. Heart Transplantation

Recent results with heart transplants have shown long-term survival and rehabilitation rates equal to those of patients receiving cadaveric donor renal allografts, leading to an increased use of transplants to treat end-stage heart disease. Rehabilitation of recipients surviving > 1 year is excellent; > 95% of patients achieve a New York Heart Association class I cardiac status, and > 70% return to full-time employment.

Common indications are cardiomyopathy, end-stage coronary artery disease, and inability to be weaned from temporary cardiac-assist devices after MI or nontransplant cardiac surgery. Recipient selection criteria have been stringent; 1/4 of patients found suitable for transplants die of cardiac disease before a suitable donor organ becomes available. Left ventricular-assist devices and artificial hearts may be used as interim support. Donor evaluation includes assessment of cardiac function, pulmonary status, size match, and ABO blood group compatibility. Donor hearts are preserved by simple hypothermic storage. Total ischemic time is held to < 4 to 6 h, thus excluding procurement from distant hospitals.

Transplantation procedure: The heart is transplanted in an orthotopic position with aortic, pulmonary artery, and pulmonary vein anastomoses. Venous return is provided by a single anastomosis joining the retained posterior wall of the recipient's right atrium to that of the donor organ.

Rejection management: Immunosuppressive regimens are similar to those for kidney or liver transplantation. One-year actuarial survival is about 80% for patients receiving cyclosporine, up from 60% for those using azathioprine. Rejection in the initial postoperative period has also been reduced; about 40% of patients experience no rejection compared with < 10% with azathioprine.

Rejection onset may be heralded by fever, malaise, tachycardia, hypotension, and heart failure that is predominantly right-sided. Arrhythmias may occur in more severe rejection episodes. In milder cases, rejection may be suggested by biopsy findings only. With the use of cyclosporine, routine protocol transvenous endomyocardial biopsy has been used increasingly to diagnose rejection, because other signs and symptoms are often absent and rejection may be detected before function of the graft deteriorates. Rejection is treated with corticosteroid and ATG or OKT3, if necessary. Mild rejection by histologic criteria without detectable clinical sequelae requires no treatment.

Complications: Infection causes > 1/2 of all deaths after heart transplantation; other major causes are rejection, graft coronary artery arteriosclerosis, and malignancy, each accounting for 5 to 20% of deaths. Accelerated graft arteriosclerosis occurs as a sequela in about 25% of all successful heart transplants. This may well be a result of indolent, humorally mediated chronic rejection. It appears that cyclosporine, which greatly increases the incidence of posttransplant hypertension, may also exacerbate coronary atherosclerosis in the graft, perhaps by direct toxicity to the coronary vasculature similar to that seen in the kidney. An increased incidence of graft coronary atherosclerosis has been suspected in patients with cytomegalovirus infections after transplantation, suggesting that an immune response to viral antigens may be involved in the development of this devastating late complication.

### E. Lung and Heart-Lung Transplantation

Lung transplantation presents special problems because of the risk of devastating infection in a transplanted organ that is continually exposed to nonsterile ambient air and dependent on the cough mechanism, which transplantation disrupts. Current 1-year survival of lung transplant recipients is about 70% in a patient population that has essentially no chance of survival without transplantation. The long-term survival rate after lung transplantation is not fully established, but the incidence of graft loss late after transplantation appears to be lower than that of other organ allografts. Functional rehabilitation is good; most recipients resume everyday activities. Exercise capacity is slightly limited due to a hyperventilatory response.

Options for lung transplantation are single lung, double lung, or combined heart-lung transplantation. The former has been performed most often. Advantages of double lung and heart-lung transplantation are the removal of all potentially diseased tissue from the thorax and, for heart-lung transplantation, a more dependable healing of the tracheal anastomosis because coronary-bronchial collaterals are present within the heart-lung block. Disadvantages are the more extensive nature of the operations, with heart-lung replacement requiring cardiopulmonary bypass, the close match necessary for thoracic size, the use of two or three donor organs for one recipient, and, in some cases, the replacement of a normal heart with one that may develop posttransplant dysfunction. Indications for heart-lung transplantation are pulmonary vascular disease or diffuse parenchymal lung disease in which removal of all lung tissue is indicated (*e.g*., certain cases of cystic fibrosis). When there is no intrinsic or secondary cardiac abnormality, the native heart of the heart-lung transplant recipient may be a donor organ for cardiac transplantation.

A single lung transplant is most clearly indicated for patients with restrictive lung disease. Advantages are the relative simplicity of the surgical procedure, which avoids systemic anticoagulation and cardiopulmonary bypass; the greater range acceptable for donor/recipient size match; and the optimal use of organs with the heart (and the contralateral lung) available for other recipients. Disadvantages include the possibility of ventilation/perfusion mismatch between the native and transplant lungs and poor healing of the bronchial anastomosis. Wrapping of the bronchial anastomosis with omentum has reduced but not eliminated the latter problem.

A double lung transplant removes all diseased lung tissue and theoretically is applicable in all patients who have no irreversible cardiac abnormality. However, division of the donor bronchial arteries and bronchocoronary collaterals makes tracheal healing problematic.

Donor selection and preservation: Cadaveric lung donors should be previous nonsmokers < 40 years old. There should be minimal evidence of consolidation on chest x-ray, and ventilator-assisted oxygenation should be normal. Lung preservation is not well developed; a lung transplant must be performed swiftly. Most often, cold crystalloid solution containing prostacyclin is infused into the donor pulmonary arteries in situ before excision. Alternatively, the donor lung may be cooled systemically using cardiopulmonary bypass, avoiding the introduction of crystalloid into the pulmonary vasculature.

Transplantation procedure: For a single lung transplant, a lateral thoracotomy is used in the recipient. Cuffs of the pulmonary artery, pulmonary vein, and bronchus are used for anastomosis. If pulmonary artery clamping is not tolerated, bypass is required. A heart-lung transplant is performed on bypass through a median stemotomy with aortic and right atrial anastomoses. The tracheal anastomosis is carried out at a point immediately above the bifurcation. Double lung transplants require more elaborate surgical reconstruction of vessels and airways but have met with increasing success recently for patients whose hearts are normal.

Rejection management: Treatment is with corticosteroids given rapidly IV in high dosage, ATG, or OKT3. Prophylactic ALG or OKT3 is also frequently given during the first two posttransplant weeks. Acute rejection occurs in > 80% of patients but may be successfully managed in a very high percentage of cases. Lung rejection occurs more often than heart rejection in combined heart-lung transplant recipients, so that endomyocardial biopsies are not always helpful. Rejection is characterized by fever, dyspnea, and decreased Sa_{O2} and forced expiratory volume in 1 sec (FEV₁). The interstitial infiltrate seen on x-ray is hard to distinguish from that of an infection. Bronchoscopy with lavage and transbronchial biopsy are often used for diagnosis.

Complications: The most troublesome early complications are related to poor healing of the bronchial or tracheal anastomosis. Up to 20% of single-lung recipients develop bronchial stenosis, which can often be treated with dilation or stent placement. To prevent interference with healing of the bronchial anastomosis, corticosteroids are omitted from the immunosuppression regimen in the early postoperative period. Relatively high doses of cyclosporine (10 to 14 mg/kg/day po) and azathioprine (1.5 to 2.5 mg/kg/day po or IV) are used.

A late complication of lung transplantation is obliterative bronchiolitis causing slowly progressive airway obstruction. It may be a manifestation of chronic rejection. There is a decrease in FEV₁ without evidence of any pulmonic process.

### F. Pancreas Transplantation

Pancreas transplantation is unique among the vascularized organ transplants: Instead of being used to save life, it attempts to stabilize or prevent the devastating target organ complications of type I diabetes. If the complications of diabetes (*e.g.*, nephropathy, retinopathy, neuropathy, accelerated atherosclerosis) are a direct result of poor glucose homeostasis, then returning the patient to normoglycemia may stabilize the progression of these secondary processes. There are insufficient data to know whether such results will be achieved.

Success of pancreas transplantation is measured by the recipient's ability to remain normoglycemic without exogenous insulin. Within the last decade, overall success rates have improved from < 40% to > 80%, with several centers reporting that > 85% of recipients remain insulin-independent. Improvement in success rates has been due primarily to improved immunosuppression regimens and to technical advances.

Recipient and donor selection: Pancreas transplantation is not appropriate for all diabetic patients. Because the recipient exchanges the risks of insulin injection with the risks of immunosuppression, pancreas transplantation has been generally limited primarily to patients who already need to receive immunosuppressive drugs (*i.e*., diabetics with renal failure who are receiving a kidney transplant). However, a few centers are now performing isolated pancreas transplantations in diabetic patients without end-stage diabetic nephrosclerosis but who have other severe complications of diabetes. Simultaneous pancreas-kidney transplants from a single cadaver have been used increasingly with excellent metabolic results. The recipient is thus exposed to high-dose induction immunosuppression only once, and because both organs come from the same donor, rejection may be monitored in the kidney, which appears more prone to this process than the pancreas, where detection of rejection is difficult. Although postoperative morbidity is increased after the combined transplant, kidney allograft survival is not jeopardized.

Donors are usually between 10 and 55 years old with no history of glucose intolerance or chronic alcohol abuse. (Serum glucose and amylase at the time of death are not helpful, because these values are often elevated in the setting of head injury and trauma resuscitation even when the pancreas is normal.)

Transplantation procedure: Technical advances include transplantation of the entire pancreas, which provides more insulin-secreting cells, rather than a segment. Also, the incidence of posttransplant graft thrombosis has been greatly reduced. Drainage of the pancreatic exocrine secretions into the urinary bladder via a conduit of a small segment of donor duodenum has also proved superior to the formerly used procedure. However, with bladder drainage of the pancreas, there is an obligate bicarbonate loss and an increase in UTIs. Attachment of the donor duodenal segment directly to the recipient small intestine has been used increasingly in recent years.

The allograft is positioned laterally in the lower abdomen. Vascular anastomoses in a pancreas transplant are the donor celiac and superior mesenteric artery and portal vein to the recipient iliac artery and vein, respectively. This provides systemic rather than portal delivery of insulin with a resulting baseline fasting hyperinsulinemia.

Rejection management: Immunosuppression is the same as that used for patients with kidney transplants. Induction therapy and rejection treatment usually involve use of ALG or OKT3.

Complications: Major complications in addition to those mentioned above are rejection, infection, and graft pancreatitis. In patients who have an isolated pancreas transplant, detection of rejection is difficult, because most of the graft may be destroyed by the rejection reaction before abnormalities in glucose metabolism become evident. However, with excellent HLA matching, a graft survival rate of 80%, comparable with the overall success rate of combined kidney-pancreas transplantation, is achieved.

### G. Alternatives to Pancreas Transplantation

There are a number of alternative approaches to pancreas transplantation. For example, artificial insulin-producing cells or glucose responsive insulin-secreting like cells may be used for transplantation, more specifically, pancreatic islet cells and/or beta cells. Such cells may comprise, for example, an engineered beta cell line, or another type of engineered cell line. For example, U.S. Patent Application NO. 20010024824, which is entirely expressly incorporated herein by reference, describes the use of a molecular marker to isolate stem cells or pseudo-islet like structures that are useful in transplantation. The stem cells may be isolated, expanded, and transplanted into a subject either allogeneically, isogeneically, or xenogeneically, to provide replacement for lost or damaged insulin-secreting cells.

Moreover, U.S. Patent Nos. 6,399,341, which is entirely expressly incorporated herein by reference, describes an artificial pancreas comprising pancreatic islets cells capable of producing insulin, encapsulated within a semipermeable spheroidal membrance comprising an agar gel. *See also* U.S. Patent Application No. 20020151055, which is entirely expressly incorporated herein by reference. U.S. Patent No. 5,702,444, which is entirely expressly incorporated herein by reference, describes an implantable artificial endocrine pancreas comprising a body of soft, plastic, biocompatible, porous hydratable material supporting a multiplicity of endocrine pancreatic islets.

Transplantation of islet cells alone has been limited in humans because of problems in obtaining and delivering sufficient islet cells. Recently, insulin independence has been achieved in diabetic recipients of islet allografts by using cells obtained from several cadaver pancreata. Whether long-term normoglycemia may be maintained is uncertain. Islet cell transplantation has several advantages: The cells may be delivered easily into the recipient's portal circulation by umbilical vein cannulation without a major operation, and islets may be cryopreserved. A potential for treating islet cells to reduce their immunogenicity also exists.

### H. Bone Marrow Transplantation

Over the past two decades, allogeneic bone marrow transplantation (BMT) has evolved from an experimental procedure reserved for patients with refractory leukemia into a rapidly expanding area of clinical investigation that offers potential cure for patients with aplastic anemia, acute and chronic leukemia, breast cancer, and selected types of lymphoma. The objective of BMT is to provide a healthy stem cell population that will differentiate into blood cells to replace deficient or pathologic cells of the host. Intensive preparative regimens, effective graft-vs.-host disease (GVHD) prophylaxis, treatment with cyclosporine-based regimens, and improvements in supportive care (*e.g.*, antibiotics, herpesvirus and cytomegalovirus prophylaxis) have brought significant improvements in long-term disease-free survival in patients undergoing BMT. Cytokine treatment after BMT (*e.g.,* with colony-stimulating factor) is being tested to see if engraftment may be improved or accelerated.

Indications: Patients with acute myeloid or lymphoblastic leukemia may benefit from BMT. Patients with acute myeloid leukemia transplanted in first remission can now expect an approximately 50 to 60% likelihood of long-term disease-free survival. Similar probabilities are also achievable after transplantation of adults with acute lymphoblastic leukemia in first remissions. Probability of relapse correlates with remission status at the time of the transplant, ranging from 20% in first remission to 60% with more advanced disease. Long-term survival for patients with chronic myelocytic leukemia who receive BMT in the phase of remission is 60 to 70%.

Pediatric BMT has expanded because of its potential for curing children with genetic diseases (*e.g.*, thalassemia, sickle cell anemia, immunodeficiencies, inborn errors of metabolism).

Donor limitations: The key limiting factor in the use of BMT is the lack of donors. Because only 25 to 30% of patients have an HLA-identical sibling, alternative donors are often required. Two possibilities exist: (1) Marrow may be procured from unrelated living donors; marrow donation is a simple, safe procedure. National and international registries of prospective volunteer donors are being expanded to increase the likelihood of finding an exact HLA match for any given recipient. (2) Related donors who are not HLA-identical have been used with increasing frequency. Results with either procedure suggest long-term disease-free survival probabilities of 30 to 50% in patients with acute and chronic leukemia or aplastic anemia; *i.e.*, in most situations the results are somewhat inferior to those with marrow from HLA-identical siblings.

Another option for BMT is autologous transplantation (removal of a patient's own marrow when a complete remission has been induced, followed by ablative treatment of the patient with the hope of destruction of any residual tumor and rescue with the patient's own bone marrow). Since an autograft is used, no immunosuppression is necessary other than the short-term high-dose chemotherapy used for tumor eradication and bone marrow ablation; posttransplant problems with GVHD are minimal. Indications for autologous BMT are relapsed, chemotherapy-sensitive lymphoma, in which a 30 to 40% success rate has been achieved, and acute leukemia in remission, in which 20 to 50% success rates have been observed. Success rates are inferior with more advanced disease and with responsive solid cancers (*e.g.*, breast or germ cell tumors). Two major obstacles remain for successful application of autologous BMT: the possibility of contamination of the marrow inoculum with tumor cells, and the absence of graft-vs.-tumor activity (in contrast with that seen in allogeneic BMT), both of which contribute to the observed higher rates of tumor recurrence. Thus, developing schemes for *ex vivo* marrow purging and for recipient immune modulation posttransplant is an active area of research.

Recipient preparation: The development of aggressive preparative regimens has improved outcome by reducing the incidence of rejection and relapse. These regimens have increased antitumor or antileukemic potential as well as delivered superior myeloablation, necessary to destroy the host marrow and make room for the donor graft without compromising the marrow stromal elements essential for engraftment. Preparative regimens also suppress the patient's immune system to allow for acceptance of the graft.

Patients are given high dosages of cyclophosphamide and/or total body irradiation in standard preparation regimens. The rejection rate is < 5% in transplants for leukemia patients from HLA-identical donors. For multiply transfused patients with aplastic anemia, the rejection rate has also been significantly decreased because of increased immunosuppression during transplant induction. The two most common preparative regimens are high-dosage cyclophosphamide (*e.g.,* 60 mg/kg/day for 2 days) and total body irradiation or a regimen of busulfan (*e.g.*, 4 mg/kg/day for 4 days) and cyclophosphamide without total body irradiation. Other drugs (*e.g.*, etoposide and cytarabine) are sometimes added to these transplant regimens to maximize antitumor properties, myeloablation, and immunosuppression.

Transplantation procedure: The transplantation procedure is relatively straightforward. Patients are given high doses of chemotherapy and/or total body irradiation. The marrow is then aspirated from the iliac crests of an HLA-compatible donor and infused IV into the patient. Patients are severely pancytopenic until engraftment, usually within 2 to 3 wk after reinfusion of the marrow.

Complications: Early complications include rejection by the host of the marrow graft, acute GVHD, and infections. Later complications include chronic GVHD, prolonged immunodeficiency, and disease recurrence.

### 1. Cutaneous T-cell lymphoma

Cutaneous T-cell lymphoma is a disease in which certain cells of the lymph system (called T-lymphocytes) become cancer (malignant) and affect the skin. Lymphocytes are infection-fighting white blood cells that are made in the bone marrow and by other organs of the lymph system. T-cells are special lymphocytes that help the body's immune system kill bacteria and other harmful things in the body.

The lymph system is part of the immune system and is made up of thin tubes that branch, like blood vessels, into all parts of the body, including the skin. Lymph vessels carry lymph, a colorless, watery fluid that contains lymphocytes. Along the network of vessels are groups of small, bean-shaped organs called lymph nodes. Clusters of lymph nodes are found in the underarm, pelvis, neck, and abdomen. The spleen (an organ in the upper abdomen that makes lymphocytes and filters old blood cells from the blood), the thymus (a small organ beneath the breastbone), and the tonsils (an organ in the throat) are also part of the lymph system.

There are several types of lymphoma. The most common types of lymphoma are called Hodgkin's disease and non-Hodgkin's lymphoma. These types of lymphoma usually start in the lymph nodes and the spleen. See the patient information summaries on adult or childhood non-Hodgkin's lymphoma or adult or childhood Hodgkin's disease for treatment of these cancers.

Cutaneous T-cell lymphoma usually develops slowly over many years. In the early stages, the skin may itch, and dry, dark patches may develop on the skin. As the disease gets worse, tumors may form on the skin, a condition called mycosis fungoides. As more and more of the skin becomes involved, the skin may become infected. The disease can spread to lymph nodes or to other organs in the body, such as the spleen, lungs, or liver. When large numbers of the tumor cells are found in the blood, the condition is called the Sezary syndrome.

If there are symptoms of cutaneous lymphoma, a doctor may remove a growth from the skin and look at it under a microscope. This is called a biopsy.
The chance of recovery (prognosis) and choice of treatment depend on the stage of the cancer (whether it is just in the skin or has spread to other places in the body) and the patient's general state of health.

There are several other types of cancer that start in the skin. The most common are basal cell cancer and squamous cell cancer, which are covered in the PDQ patient information summary on skin cancer. Another type of skin cancer called melanoma is covered in the patient information summary on melanoma. Kaposi's sarcoma, a rare type of cancer that occurs most commonly in patients with the Acquired Immunodeficiency Syndrome (AIDS), also affects the skin. See the PDQ patient information summary on Kaposi's sarcoma for treatment of this cancer. Cancers that start in other parts of the body may also spread (metastasize) to the skin.

### a. Stages of cutaneous T-cell lymphoma

Once cutaneous T-cell lymphoma is found, more tests will be done to find out if cancer cells have spread to other parts of the body. This is called staging. A doctor needs to know the stage of the disease to plan treatment. The following stages are used for cutaneous T-cell lymphoma:

In Stage I, the cancer only affects parts of the skin, which has red, dry, scaly patches, but no tumors. The lymph nodes are not larger than normal. In Stage II, either of the following may be true. The skin has red, dry, scaly patches, but no tumors. Lymph nodes are larger than normal, but do not contain cancer cells. There are tumors on the skin. The lymph nodes are either normal or are larger than normal, but do not contain cancer cells. In Stage III, nearly all of the skin is red, dry, and scaly. The lymph nodes are either normal or are larger than normal, but do not contain cancer cells. In Stage IV, the skin is involved, in addition to either of the following: cancer cells are found in the lymph nodes or cancer has spread to other organs, such as the liver or lung. Recurrent disease means that the cancer has come back after it has been heated. It may come back where it started or in another part of the body.

### b. Treatment

There are treatments for all patients with cutaneous T-cell lymphoma. Three kinds of treatment are commonly used: radiation therapy (using high-energy rays to kill cancer cells); chemotherapy (using drugs to kill cancer cells); and photopheresis (using light plus special drugs to make the cancer cells more sensitive to the light). Biological therapy (using the body's immune system to fight cancer) is being tested in clinical trials.

Radiation therapy uses high-energy rays to kill cancer cells and shrink tumors. In cutaneous T-cell lymphoma, special rays of tiny particles called electrons are commonly used to treat all of the skin. This is called total skin electron beam radiation therapy, or TSEB radiation therapy. Electron beam radiation may also be given to smaller areas of the skin. This kind of radiation only goes into the outer layers of the skin. Another type of radiation uses x-rays to kill cancer cells. The x-rays are usually directed to only certain areas of the body, but there are studies using x-rays directed at the whole body (total body irradiation).

Chemotherapy uses drugs to kill cancer cells. Chemotherapy may be taken by pill, or it may be put into the body by a needle in a vein or muscle. Chemotherapy given in this way is called a systemic treatment because the drug enters the bloodstream, travels through the body, and can kill cancer cells throughout the body. In cutaneous T-cell lymphoma, chemotherapy drugs may be given in a cream or lotion put on the skin. This is-called topical chemotherapy.

Biological therapy tries to get the body to fight cancer. It uses materials made by the body or made in a laboratory to boost, direct, or restore the body's natural defenses against disease. Biological therapy is sometimes called biological response modifier (BRM) therapy or immunotherapy.

Bone marrow transplantation is used to replace the bone marrow with healthy bone marrow. First, all of the bone marrow in the body is destroyed with high doses of chemotherapy with or without radiation therapy. Healthy marrow is then taken from another person (a donor) whose tissue is the same as or almost the same as the patient's. The donor may be a twin (the best match), a brother or sister, or another person not related. The healthy marrow from the donor is given to the patient through a needle in the vein, and the marrow replaces the marrow that was destroyed. A bone marrow transplant using marrow from a relative or unrelated person is called an allogeneic bone marrow transplant.

Another type of bone marrow transplant, called autologous bone marrow transplant, is being studied in clinical trials. To do this type of transplant, bone marrow is taken from the patient and treated with drugs to kill any cancer cells. The marrow is then frozen to save it. Next, the patient is given high-dose chemotherapy with or without radiation therapy to destroy all of the remaining marrow. The frozen marrow that was saved is then thawed and given back to the patient through a needle in a vein to replace the marrow that was destroyed. Another type of autologous transplant is called a peripheral blood stem cell transplant. The patient's blood is passed through a machine that removes the stem cells (immature cells from which all blood cells develop), then returns the blood back to the patient. This procedure is called leukapheresis and usually takes 3 or 4 hours to complete. The stem cells are treated with drugs to kill any cancer cells and then frozen until they are transplanted back to the patient. This procedure may be done alone or with an autologous bone marrow transplant. A greater chance for recovery occurs if the doctor chooses a hospital which does more than 5 bone marrow transplantations per year.

### c. Treatment by stage

Treatment of cutaneous T-cell lymphoma depends on the stage of the disease, and the patient's age and overall health. Standard treatment may be considered because of its effectiveness in patients in past studies, or participation in a clinical trial may be considered. Most patients with cutaneous T-cell lymphoma are not cured with standard therapy and some standard treatments may have more side effects than are desired. For these reasons, clinical trials are designed to find better ways to treat cancer patients and are based on the most up-to-date information.

Stage I Treatment may be one of the following: ECP with or without biological therapy; total skin electron beam radiation therapy (TSEB radiation therapy); topical chemotherapy; local electron beam or x-ray therapy to reduce the size of the tumor or to relieve symptoms; clinical trials of photopheresis; or interferon-α (biological therapy) alone or in combination with topical therapy.

Stage II Treatment may be one of the following: ECP with or without biological therapy; total skin electron beam radiation therapy (TSEB radiation therapy); topical chemotherapy; local electron beam or x-ray therapy; or interferon-α (biological therapy) alone or in combination with topical therapy.

Stage III Treatment may be one of the following: ECP with or without biological therapy; total skin electron beam radiation therapy (TSEB radiation therapy); topical chemotherapy; local electron beam or x-ray therapy; systemic chemotherapy with or without therapy to the skin; chemotherapy for mycosis fungoides and Sezary syndrome; extracorporeal photochemotherapy; interferon-α (biological therapy) alone or in combination with topical therapy; or retinoids.

Stage IV Treatment may be one of the following: systemic chemotherapy; topical chemotherapy; total skin electron beam radiation therapy (TSEB radiation therapy); ECP with or without biological therapy; local electron beam or x-ray therapy; chemotherapy for mycosis fungoides and Sezary syndrome; extracorporeal photochemotherapy; interferon-α (biological therapy) alone or in combination with topical therapy; monoclonal antibody therapy; or retinoids.

### I. Corneal Transplantation

A surgical procedure to remove the diseased part of the cornea and replace it with a similarly sized and shaped part of a healthy donor cornea.

Indications: corneal transplantations are performed for several reasons, including the following: optical, to improve the optical qualities of the cornea and thus improve vision, *e.g.*, replacing an opaque/scarred cornea due to corneal stromal dystrophy or a cornea with irregular astigmatism due to keratoconus; reconstructive, to reconstruct the anatomic cornea to preserve the eye, *e.g.*, replacing a perforated cornea; and therapeutic, to treat a disease unresponsive to medical management to preserve the eye, *e.g.*, as a therapy for a severe, uncontrolled fungal corneal ulcer, or to alleviate pain, *e.g.*, to relieve the severe foreign-body sensation due to recurrent ruptured bullae in bullous keratopathy.

The most common indications, in descending order, are bullous keratopathy (pseudophakic, Fuchs' endothelial dystrophy, aphakic), keratoconus, repeat graft, keratitis/postkeratitis (viral, bacterial, fungal, *Acanthamoeba,* perforation), and corneal stromal dystrophies.

Donor Tissue Selection: tissue matching is not routinely performed or necessary for the majority of corneal transplants. Corneal tissue from donors with the following conditions is not used for transplantation: death from unknown causes, Creutzfeldt-Jakob disease, subacute sclerosing panencephalitis, progressive multifocal leukoencephalitis, congenital rubella, active encephalitis, active septicemia, active endocarditis, active syphilis, viral hepatitis or seropositivity, rabies, HIV seropositivity or high risk for HIV infection, leukemias, active disseminated lymphomas, prior anterior segment surgery or disease, and most intraocular malignancies. The donor's blood is tested for HIV-1, HIV-2, hepatitis B, and hepatitis C. Tissue from donors with positive serology is not used.

Surgical Technique: corneal transplants may be performed using general or local anesthetic plus IV sedation. To prepare the tissue for transplantation from the donor cornea, the surgeon punches out a corneal button from the central part of the donor cornea using a trephine. To create the recipient bed to receive the donor corneal button, the surgeon removes the central 60 to 80% of the host cornea using a trephine and scissors. The donor corneal button, which is trephined slightly larger than the recipient bed, is then sutured in place.

Postoperative Management postoperative topical antibiotics are used for several weeks and topical corticosteroids for several months. In some patients, the corneal astigmatism may be reduced in the early postoperative period by suture adjustment or selective suture removal. Achievement of full visual potential may take up to 1 year because of changing refraction, slow wound healing, and/or corneal astigmatism. In many patients, earlier and better vision is attained with a rigid contact lens over the corneal transplant. To protect the eye from inadvertent trauma after transplantation, the patient wears shields, glasses, or sunglasses. In addition, patients are advised to avoid bending over completely, lifting heavy objects, straining, or the Valsalva maneuver.

Complications: complications include infection (intraocular and corneal), intraocular bleeding, wound leak, glaucoma, graft rejection, graft failure, high refractive error (especially astigmatism and/or myopia), and recurrence of disease, *i.e.,* corneal stromal dystrophy.

Graft rejection is not uncommon. Patients complain of decreased vision, photosensitivity, ocular ache, and ocular redness. Graft rejection is treated with corticosteroids, which are administered topically (*e.g.*, prednisolone acetate 1% hourly), often with a supplemental periocular injection (*e.g.*, methylprednisolone 40 mg). If the graft rejection is severe or if the graft function is marginal, additional corticosteroids are given orally (*e.g*., prednisone 1 mg/kg/day) and occasionally IV (*e.g.*, methylprednisolone sodium succinate 3 to 5 mg/kg once). In most non-high-risk grafts, the graft rejection episode is easily reversed, and graft function returns fully. The graft may fail if the graft rejection was unusually severe or long-standing or after multiple episodes of graft rejection. Regraft is possible, but the long-term prognosis for a clear regraft is lower than it was for the original graft.

Prognosis: the prognosis for a clear, functioning corneal transplant varies by diagnosis. The chance of long-term transplant success is > 90% for keratoconus, corneal scars, early bullous keratopathy, or corneal stromal dystrophies; 80 to 90% for bullous keratopathy or inactive viral keratitis, 50% for active corneal infection, and 0 to 50% for chemical or radiation injury.

The generally high rate of success of corneal transplantation is attributable to many factors, including the avascularity of the cornea and the fact that the anterior chamber has venous drainage but no lymphatic drainage. These conditions promote low-zone tolerance and an active process termed anterior chamber-associated immune deviation, in which there is suppression of intraocular lymphocytes and delayed-type hypersensitivity to transplanted intraocular antigens. Another important factor is the effectiveness of the immunosuppressive drugs used to treat graft rejection.

### J. Transplantation of Other Organs and Tissues

Skin allografts may be valuable for patients with extensive burns or other causes of massive skin loss. When insufficient donor sites negate the use of autografts alone, strips of autografts and allografts are alternated, covering the entire denuded area to reduce fluid and protein losses and to discourage invasive infection. The allografts are rejected, but these secondarily denuded areas can then be re-covered with autografts taken from healed original donor sites. Allografts also serve as dressings for infected burns or wounds that rapidly become sterile and develop well-vascularized granulations onto which autografts will take readily. Skin cells, expanded in culture before being returned to a burned patient, may also help cover extensive burns, as may newly introduced "artificial skin," which is composed of cultured cells on a synthetic underlayer.

Cartilage transplantation is unique in that chondrocytes are among the few types of mammalian cells that may be allografted without succumbing to the immune response, apparently because the sparse population of cells in hyaline cartilage is protected from cellular attack by the cartilaginous matrix around them. In children, cartilage obtained from cadavers may be used to replace congenital nasal or ear defects. In adults, autografts (usually from rib cartilage) are more commonly used to treat severe injuries. Use of cartilage allografts to resurface articular joints destroyed by arthritis is being attempted.

Bone grafting is widely used, but, except for autografts, no viable donor bone cells survive in the recipient. However, the remaining dead matrix has a bone-inducing capacity that stimulates host osteoblasts to recolonize the matrix and lay down new bone, thus serving as a scaffolding for bridging and stabilizing defects until new bone is formed. Massive resection of malignant bone tumors and reconstruction by implantation of composite bone and cartilage allografts are practical approaches to saving extremities that would otherwise be amputated. Cadaveric allografts are preserved by freezing to decrease immunogenicity of the bone (which is dead at the time of implantation) and glycerolization to maintain chondrocyte viability. No postimplantation immunosuppressive therapy is used. Although these patients develop anti-HLA antibodies, early follow-up reveals no evidence of cartilage degradation.

Small-bowel transplantation is a procedure in development applicable to only a small group of patients with inadequate gut absorptive surface area due to intra-abdominal catastrophes (*e.g.*, volvulus, toxic enterocolitis, trauma). Small-bowel transplants should be further limited to patients who cannot tolerate chronic parenteral nutrition and thus have no other options for survival. Survivals of more than 1 year with intact enteral function are now being achieved after small-bowel transplantation. Issues that need to be addressed are optimal length of the segment to be transplanted, the use of systemic versus portal venous drainage of the graft, the advisability of immediate continuity of the transplant in the recipient's GI tract, and the role of living-related donation for small-bowel allografts. Because of the large amount of gut-associated lymphoid tissue, GVHD is a much greater problem with small-bowel transplants than other vascularized organ allografts.

Treating patients with Parkinson's disease with auto grafts of adrenal medullary tissue stereotactically placed within the CNS has been reported to lead to symptomatic improvement. Allografts of adrenal tissue, especially from fetal donors, have also been proposed. Fetal ventral mesencephalic tissue stereotactically implanted in the putamen of patients with Parkinson's disease has been reported to improve the patient's rigidity and the bradykinesia. However, with the ethical and political debates that exist over the propriety of using human fetal tissue, it is unlikely that a controlled trial large enough to adequately assess fetal neural transplantation will be undertaken. Xenografts of endocrinologically active cells from porcine donors are being tested.

Fetal thymus implants obtained from stillborn infants may restore immunologic responsiveness to children with thymic aplasia and consequent lack of normal development of the lymphoid system. Because the recipient is immunologically unresponsive, immunosuppression is not required; however, GVHD may be severe.

Parathyroid tissue autografts (and even, rarely, allografts) have been used successfully. Parathyroid autotransplantation has been recommended by some groups for treatment of patients with hypercalcemia due to secondary hyperplasia. The technique involves removal of all parathyroid tissue from the neck, with placement of a few small slivers of tissue in a muscle pocket in the forearm, where the tissue can later easily be identified if hypercalcemia recurs. Allografts may be used for patients with iatrogenic hypoparathyroidism whose course with optimal medical management is unsatisfactory. Since immunosuppression is required, this procedure is rarely indicated unless the patient also is receiving a renal allograft, for which suppression will be necessary.

Artificial organ transplantation is one medical field in which the disclosed invention may be used. Artificial organs includes, but are not limited to, organs such as hearts, lungs, livers, and kidneys. *See e.g.,* Arcasoy et al., 340 N. ENGL. J. MED. 1081 (1999); Salvatierra, 345 N. ENGL. J. MED. 1355 (2001). Other artificial organs include, but are not limited to, heart, liver, pancreas, kidney, lung, pancreatic islets, larynx, blood, stem cells, eyes, cornea, muscle, and skin. Artificial organs may comprise, for example, man-made materials including, but not limited to, alloplastic materials.

Moreover, the disclosed invention is also useful for xenotransplantation and other means of organ replacement, including artificial organs, which are increasingly becoming useful for treating human disease. Technologies such as cellular transplantation, including the use of stem cells, organogenesis and xenotransplantation have increasingly become useful for treating human disease. *See* Cascalho et al., 1 NATURE 154 (2001). The prospect of transplanting animal organs, tissue and cells into humans is looking increasingly promising, as progress is made towards overcoming the formidable barriers of cross-species rejection. For instance, pig organs are produced for human transplantation and strategies are developed for making more organs available for transplantation. *See* B. Gridelli et al., 343 N. ENGL. J. MED. 404 (2000).

In addition, in one embodiment, the methods of the present invention may be used in the context of implant surgery, for example, with implant surgery commonly performed in cosmetic or non- cosmetic plastic surgery. Such implants may include dental, fat grafting, for example to the cheeks, lips and buttocks, facial implants, including those to the nose, cheeks, forehead, chin and skull, buttocks implants, breast implants, etc. Other implants include, but are not limited to, corneal ring, cortical, orbital, cochlear, muscle (all muscles, including pectoral, gluteal, abdominal, gastrocnemius, soleus, bicep, tricep), alloplastic joint and bone replacement, bone repair implants (screws, rods, beams, bars, springs), metal plates, spinal, vertebral, hair, botox/collagen/restylane/perlane injections, penile implants, prostate seed implants, breast implants (cosmetic and reconstructive), interuterine devices, hormonal implants, fetal or stem cell implantation, pacemaker, defibrillator, artificial arteries/veins/valves, and artificial organs.

Stem cell research has furthered the transplantation technology. Research on somatic-cell nuclear transfer and studies of stem-cell differentiation could provide valuable information, for instance, about the mechanism of ageing or the cause of cancer. Stem cells derived from this technology might also be a rich source of material for transplantation if specific genes or sets of genes in these pluripotent stem cells could be activated and if the cells could then be coaxed to differentiate. This is not theoretical, because differentiated cell types (vascular endothelium, heart and skeletal muscle, and neurones) have already been obtained by culturing embryonic stem cells from mice. If this technology could be applied to human stem cells, the resulting products might revolutionize medical therapeutics. *See e.g.,* Tsubota et al., 340 N. ENGL. J. MED. 1649 (1999); and Childs et al., 343 N. ENGL. J. MED. 750 (2000).

Stem cells have the ability to divide for indefinite periods in culture and to give rise to specialized cells. Perhaps the most far-reaching potential application of human pluripotent stem cells is the generation of cells and tissue that could be used for so-called "cell therapies." Many diseases and disorders result from disruption of cellular function or destruction of tissues of the body. Today, donated organs and tissues are often used to replace ailing or destroyed tissue. Unfortunately, the number of people suffering from these disorders far outstrips the number of organs available for transplantation. Pluripotent stem cells, stimulated to develop into specialized cells, offer the possibility of a renewable source of replacement cells and tissue to treat a myriad of diseases, conditions, and disabilities including Parkinson's and Alzheimer's diseases, spinal cord injury, stroke, burns, heart disease, diabetes, osteoarthritis and rheumatoid arthritis. There is almost no realm of medicine that might not be touched by this innovation. By way of example, and by no means a limitation, transplant of healthy heart muscle cells could provide new hope for patients with chronic heart disease whose hearts can no longer pump adequately. The hope is to develop heart muscle cells from human pluripotent stem cells and transplant them into the failing heart muscle in order to augment the function of the failing heart. Preliminary work in mice and other animals has demonstrated that healthy heart muscle cells transplanted into the heart successfully repopulate the heart tissue and work together with the host cells. These experiments show that this type of transplantation is feasible. Further, in the many individuals who suffer from Type I diabetes, the production of insulin by specialized pancreatic cells, called islet cells, is disrupted. There is evidence that transplantation of either the entire pancreas or isolated islet cells could mitigate the need for insulin injections. Islet cell lines derived from human pluripotent stem cells could be used for diabetes research and, ultimately, for transplantation. *See* Stem Cells: A Primer, National Institute of Health, May 2000.

Disorders that may be treated with stem cell transplantation include immune deficiency and inherited severe blood cell diseases; for instance, marrow transplantation is now being used to treat diseases such as thalassemia or sickle cell disease in which a mutant gene is inherited. Other inherited disorders such as a group of inherited diseases which have a defect in the monocytes may be treated with stem cell transplantation. Marrow failure is yet another example, as human stem cell transplantation has been used successfully to restore the function of marrow that has been injured by an external noxious agent, such as a chemical or unintended radiation exposure. Bone marrow and stem cell transplantation are among the newest treatment options for lymphoma patients, especially those who have relapsed. *See, e.g.*, Horwitz et al., 344 N. ENGL. J. MED. 881 (2001); and Ezekowitz, 344 N. ENGL. J. MED. 926 (2001).

### K. Graft vs. Host Disease

GVHD is a disease in which immunologically competent donor T cells react against antigens in an immunologically depressed recipient. A major problem in allogeneic transplantation is the prevention and control of GVHD. Symptoms and signs of acute GVHD are fever, exfoliative dermatitis, hepatitis with hyperbilirubinemia, vomiting, diarrhea, and abdominal pain, which may progress to an ileus, and weight loss. Although increased knowledge of the major histocompatibility complex has aided understanding of the etiology of GVHD, patients who are matched at the A, B, C, and DR loci still have a 30 to 60% incidence of GVHD. Surprisingly, a GVHD syndrome has even been reported in patients receiving syngeneic transplants (between identical twins) or autologous transplants (with their own marrow). Although the introduction of cyclosporine in the early 1980s has greatly reduced both the incidence and severity of GVHD, it continues to be the major cause of mortality and severe morbidity after allogeneic BMT.

About 1/3 to 1/2 of BMT recipients develop a more indolent chronic form of GVHD. Although the skin, liver, and gut remain the organs primarily affected, other areas of involvement (*e.g.*, joint, lung) are also noted. Interestingly, bronchiolitis obliterans similar to that seen after lung transplantation can occur. Ultimately, 20 to 40% of the patients die of complications associated with GVHD, the incidence being higher when donor marrow is not from an HLA-identical sibling. In patients without chronic sequelae of GVHD, all immunosuppression may be stopped 6 months after BMT, making late complications rare in these patients, in contrast with the continued need for immunosuppressants and resulting complications in solid organ transplant recipients.

One area of active clinical research aimed at reducing the incidence of GVHD has been the removal of T cells from the donor marrow with monoclonal antibodies, using the rosetting technique, or mechanical separation before reinfusion of the marrow. T-cell depletion has been very effective in decreasing both the incidence and severity of GVHD; however, the incidences of engraftment failure and relapse are increased. A possible explanation is that the cytokines generated in the graft-vs.-host reaction promote stem cell multiplication and maturation necessary for engraftment. Patients who develop GVHD have significantly lower relapse rates, suggesting that T cells responsible for GVHD are probably involved in a graft-vs.-leukemia effect. Other agents used to prevent or treat GVHD include methotrexate, corticosteroids, ATG, and monoclonal antibodies against antigens expressed on mature T cells.

GVHD may also follow blood transfusions in exceptional cases, since even small numbers of donor T cells can induce this reaction. Such situations include intrauterine fetal blood transfusions and transfusions in immunodepressed patients (*e.g.,* BMT recipients, leukemia, lymphoma, neuroblastoma, Hodgkin's and non-Hodgkin's lymphoma). Blood products to be given to patients at risk should be irradiated to prevent development of GVHD.

Following the preparative regimen for BMT, the WBC count can take 2 to 3 wk to recover. During this time, patients are very susceptible to infections. Acyclovir prophylaxis has dramatically decreased the risk of herpes simplex infections during this time. Even after engraftment, patients continue to be immunocompromised and at risk for infections because of the drugs used to treat GVHD. A worrisome late infection is cytomegalovirus interstitial pneumonitis, which generally occurs 40 to 60 days after transplantation. Patients present with tachypnea, dyspnea, hypoxemia, and a chest x-ray with bilateral pulmonary infiltrates. The mortality rate of cytomegalovirus interstitial pneumonitis was 80 to 90%, but treatment with ganciclovir and passive immunity with immunogloublin has decreased the mortality rate to about 25 to 40%. Patients are also at risk of developing pneumocystis pneumonia, but prophylactic use of trimethoprim-sulfamethoxazole has dramatically decreased the incidence of this infection.

### IV. Autoimmune Diseases

### A. Generally

Autoimmune diseases are diseases in which the immune system produces autoantibodies to an endogenous antigen, with consequent injury to tissues. The methods of the present invention relate to treating individuals who are predisposed to an autoimmune disease. Individuals may be identified as being predisposed to a disease by several methods, including, but not limited to, HLA linkage typing, blood or serum-based assays, or identification of genetic variants, *e.g.*, single nucleotide polymorphisms (SNPs).

### 1. The HLA System

HLA are found in varying concentrations on virtually all nucleated cells. The immunologic response to these antigens is the major cause of most graft rejection episodes.

HLA is the designation for antigens that are products of a complex of genes at several closely linked loci collectively called the major histocompatibility complex (MHC), located on chromosome 6. The genes are allelic; *i.e.*, a number of different forms of each gene are found in the population; all alleles are codominant. By Mendelian laws, each person has two alleles for each locus or, possibly, a pair of identical alleles.

The antigens are divided into two classes on the basis of structure and function. The heavy chain of the class I antigens is encoded by the genes at the HLA-A, B, or C loci. Class I molecules are heterodimeric polypeptides consisting of the heavy chain bound to a β₂-microglobulin molecule. These antigens are found on most nucleated cells in the body as well as on platelets and are homologous to serologically detected transplantation antigens in other species. The class II antigens comprise two polypeptide chains, which are both encoded by genes within the HLA-D region. The HLA-D region is divided into subregions, each with genes encoding both the α nd β chains of the different class II molecules (HLA-DR, DQ, and DP). The class II antigens are preferentially expressed on antigen-presenting cells such as B lymphocytes, macrophages, dendritic cells, and some endothelial cells. They are homologous to the immune response (Ir) gene products of other species.

Because the alleles were numbered before their loci were identified, those on loci A and B are not numbered consecutively. Since 1975, the WHO committee for factors of the HLA system has assigned universally accepted designations to individual alleles of each locus (*e.g*., HLA-A1, HLA-B5, HLA-Cw1, HLA-DR1). In the past, provisional alleles were designated with a "w." However, with more recent developments in DNA sequencing of the HLA genes, the "w" has been dropped from most serologic specificities (the C locus maintains the "w" to distinguish it from complement components). Alleles defined by DNA sequence are named so that the gene is identified and each allele is given a unique number that includes the most closely associated serologic specificity (*e.g.,* A*0201, DRB1*0103, DQA1*0102).

In the rejection reaction, class I and class II antigens elicit different responses. T cells responding to classes I and II may be distinguished not only functionally but by differentiation antigens present on the surface of the responding T cells. With the development of monoclonal antibodies (uniformly identical antibodies produced by hybridized cells) reactive with these differentiation antigens, such antigens may be used as markers to monitor T-cell subpopulations in the rejection reaction.

Class I reactive lymphocytes express the CD8 antigens often associated with cytotoxic effector and suppressor cell function. Helper-cell function activity usually is provided by T cells expressing the CD4 antigen that characterizes class II reactive lymphocytes. Thus, while the brunt of immune destruction of the rejection reaction may be directed at class I antigens both via anti-HLA antibodies and cytotoxic effector lymphocytes, lymphocytes that respond to the class II antigens seem to be necessary to facilitate a maximal rejection reaction.

HLA, however, do not exist only to be targets for the allograft response. In the normal immune response, self-HLA molecules bind foreign peptides and present these antigens to the antigen-specific receptors on T cells. Since HLA molecules are highly polymorphic, the allogeneic HLA molecules on the cells of an organ graft are recognized by the T-cell receptor not as self-HLA, but in the same manner as self-HLA plus foreign peptide. Receptor binding of graft HLA alone does not initiate the allograft response. Specific cell types within the body appear to function as antigen-presenting cells and deliver a second signal to the T cell at the time of antigen engagement. In addition, other cell-surface glycoproteins, called integrins, adhere to complementary cell-surface structures to stabilize the binding of the T-cell receptor to the presented antigen. Dendritic cells, a macrophage-like cell population, appear to act optimally in this antigen-presenting role. T-cell activation, following receptor binding of antigens on the presenting cell, is a complex chain of intracellular events leading to the transcription of multiple previously quiescent genes in CD4+ class II antigen-reactive "helper" T cells. Pivotal in the activation process is production of a cytokine, interleukin-2 (IL-2), and expression of the IL-2 receptor on the helper cell surface. IL-2 acts in an autocrine/paracrine fashion to stimulate T-cell proliferation. Activated helper T cells also produce a series of other lymphokines; they promote a cascade of events resulting in the effector mechanisms of graft destruction.

Nontransplantation associations for class I and class II antigens: Evidence is accumulating that the genes encoding for these antigens (and other closely linked genes in the MHC) are important to the general immune function and health of an individual. Several complement components and properdin factor B are governed by genes linked to the MFIC. Also, specific HLA antigens have a statistical association with various presumed autoimmune diseases and lymphoid-cell neoplasms, although the pathogenetic meaning of such associations is unknown. For example, the incidence of psoriasis increases as it is associated with B13 and B17, but decreases with B12. Ankylosing spondylitis and Reiter's syndrome have a pronounced positive correlation with the B27 genotype. DR3 and DR4 seem to be positively associated with type I diabetes mellitus, DR2 with multiple sclerosis, and DR4 with RA. In contrast, persons with malignant lymphomas seem to have a markedly reduced incidence of A11. Perhaps even more intriguing in terms of transplantation is a postulated association of DR6 with an Ir gene controlling the vigor of the rejection to renal allografts.

In a specific embodiment of the present invention, extracorporeal photopheresis may be used to prevent the onset of, delay the onset of, or reduce the effects or potential severity of a disease in individuals predisposed to such diseases, for example, autoimmune diseases. HLA typing is routinely performed in hospitals to assess an individual's susceptibility to certain diseases. For example, HLA DR4 is associated with a predisposition to rheumatoid arthritis. Other HLA linkages that predispose an individual to rheumatoid arthritis include DR1, DR6, and DR10. Once an individual is determined to have the HLA DR4 linkage, ECP may be used to prevent the onset of, delay the onset of, or reduce the effects of rheumatoid arthritis.

Other HLA alleles, also known as MHC alleles, that are associated with autoimmune diseases include B27 (Ankylosing spondylitis); DQA1*0501 and DQB1*0201 (Celiac disease); DRB1*03, DRB1*04, DQB1*0201, DQB1*0302, and DMA*0101 (Type I Diabetes); and Cw6 (Psoriasis). These alleles may also be used to determine whether an individual is predisposed to an autoimmune disease and, thus, whether ECP may be used as a pretreatment.

Moreover, blood or serum-based assays may be used to assess predisposition to a disease. There is, for example, an assay that detects the presence of autonuclear antibodies in serum, which may lead to the onset of lupus. Serum-based assays also exist for predicting autoimmune myocarditis. In addition, serum-based assays may be used to determine insulin levels (diabetes) or liver or heart enzymes for other diseases. T-3 levels may be predictive of Hashimotos thyroiditis. After an individual is determined to be predisposed to a disease using a blood or serum-based assay, the methods of the present invention may be used to prevent, or delay the onset of, or reduce the effects of these diseases.

In a further embodiment of the present invention, individuals may be identified as being predisposed for disease through the identification of genetic variations, including, but not limited to, SNPs. Indeed, it is well-known in the art that biomarkers are measurable biological parameters that may be used to assess an individual's susceptibility to a particular disease, such as cancer. *See, e.g.,* Collins, 19 MOL. ASPECTS MED. 359 (1998). Specifically, in cancer epidemiology, DNA damage, altered bases, chromosomal alterations, mutations in marker genes, and differential expression of a particular protein are all factors that may be analyzed to determine an individual's susceptibility to a particular disease and identify an association between a particular gene or genes and a particular disease. For example, individuals may be identified as being predisposed for breast cancer by comparing a suspected mutant BRCA 2 allele with wild type BRCA 2. *See, e.g.* U.S. Patent Nos. 6,033,857 and 6,124,104. *See also* Pierotti, 41 Q. J. NUCL. MED. 189 (1997). In another example, a SNP in the IL-4 promoter may be predictive of asthma.

### 2. Development of the Autoimmune Response

Although precise details of the autoimmune response are not completely understood, the outcome of antigenic stimulation, whether it be antibody formation, activated T cells, or tolerance, seems to depend on the same factors with autoantigen as with exogenous antigen. Five possible mechanisms for developing an immune response to autoantigens are recognized:
1. Hidden or sequestered antigens (*e.g.,* intracellular substances) may not be recognized as "self"; if released into the circulation they may induce an immune response. This occurs in sympathetic ophthalmia with the traumatic release of an antigen normally sequestered within the eye. Autoantibody alone may not produce disease because it cannot combine with the sequestered antigen. For example, antibodies to sperm and heart muscle antigens are blocked by the basement membrane of the seminiferous tubules and myocardial cell membrane, respectively. Immunologically active T cells may lack such restrictions and would produce injury more effectively.
2. The "self" antigens may become immunogenic because of chemical, physical, or biologic alteration. Certain chemicals couple with body proteins and render them immunogenic (as in contact dermatitis). Drugs can produce several autoimmune reactions. Photosensitivity exemplifies physically induced autoallergy: ultraviolet light alters skin protein, to which the patient becomes allergic. Biologically altered antigens occur in New Zealand mice that develop autoallergic disease resembling SLE when persistently infected with an RNA virus known to combine with host tissues, altering them enough to induce antibody.
3. Foreign antigen may induce an immune response that cross-reacts with normal "self" antigen; *e.g.*, the cross-reaction that occurs between streptococcal M protein and human heart muscle.
4. Autoantibody production may result from a mutation in immunocompetent cells. This may explain the monoclonal autoantibodies seen occasionally in patients with lymphoma.
5. Autoimmune phenomena may be epiphenomena, and the primary pathogenesis the result of an immune response to an obscure antigen (*e.g.,* a virus).

Theoretically, the autoimmune reaction is probably normally held in check by the action of a population of specific suppressor T cells or T-regulatory cells. Any of the above processes could lead to or be associated with a suppressor T-cell defect. A perturbation in the regulation of antibody activity by anti-idiotype antibodies (antibodies to the antigen-combining site of other antibodies) may play a role.

The roles of other complex mechanisms demonstrable experimentally still need clarification. For example, nonantigenic adjuvants (*e.g.*, alum, bacterial endotoxin) enhance the antigenicity of other substances. Freund's complete adjuvant, an emulsion of antigen in mineral oil with heat-killed mycobacteria, is usually required to produce autoimmunity in experimental animals.

Genetic factors play a role. Relatives of patients with autoimmune diseases often show a high incidence of the same type of autoantibodies, and the incidence of autoimmune disease is higher in identical than fraternal twins. Women are affected more often than men. The genetic contribution appears to be one of predisposition. In a predisposed population, a number of environmental factors could provoke disease; *e.g.*, in SLE these might be latent virus infection, drugs, or tissue injury such as occurs with ultraviolet light exposure. This situation would be analogous to the development of hemolytic anemia as a consequence of environmental factors in persons with G6PD deficiency, a predisposing genetically determined biochemical abnormality.

### 3. Pathogenesis

The pathogenetic mechanisms of autoimmune reactions are, in many cases, better understood than the way in which autoantibodies develop. In some autoimmune hemolytic anemias, the RBCs become coated with cytotoxic (type II) autoantibody; the complement system responds to these antibody-coated cells just as it does to similarly coated foreign particles, and the interaction of complement with the antibody complexed to the cell surface antigen leads to RBC phagocytosis or cytolysis.

Autoimmune renal injury can occur as the result of either an antibody-mediated (type II) or IC (type III) reaction. The antibody-mediated reaction occurs in Goodpasture's syndrome, in which lung and renal disease is associated with an anti-basement membrane antibody. The best known example of autoimmune injury associated with soluble antigen-antibody complexes (ICs) is the nephritis associated with Systemic Lupus Erythematosus (SLE). Another example is a form of membranous glomerulonephritis that is associated with an IC containing renal tubular antigen. Although not proven, poststreptococcal glomerulonephritis could be due in part to streptococcus-induced cross-reacting antibodies.

Various autoantibodies are produced in SLE and other systemic (as opposed to organ-specific) autoimmune diseases. Antibodies to formed elements in the blood account for autoimmune hemolytic anemia, thrombocytopenia, and possibly leukopenia; anticoagulant antibodies may cause disordered coagulation problems. Antibodies to nuclear material result in deposition of ICs, not only in glomeruli but also in vascular tissues and in skin at the dermal-epidermal junction. Synovial deposition of aggregated IgG-rheumatoid factor-complement complexes occurs in RA. Rheumatoid factor is usually an IgM (occasionally IgG or IgA) with specificity for a receptor on the constant region of the heavy chain of autologous IgG. The IgG-rheumatoid factor-complement aggregates can also be found within neutrophils, where they cause the release of lysosomal enzymes that contribute to the inflammatory joint reaction. Many plasma cells are present within the joint and may synthesize anti-IgG antibodies. T cells and lymphokines are also found in rheumatoid joints and may contribute to the inflammatory process. The process that sets off the immunologic events is unknown; it could be a bacterial or viral infection. In SLE, the low serum complement level reflects the widespread immunologic reactions taking place; in RA, by contrast, serum complement is normal but intrasynovial complement levels are low.

In pernicious anemia, autoantibodies capable of neutralizing intrinsic factor are found in the GI lumen. Autoantibodies against the microsomal fraction of gastric mucosal cells are even more common. It is postulated that a cell-mediated autoimmune attack against the parietal cells results in the atrophic gastritis, which, in turn, reduces the production of intrinsic factor but still allows absorption of sufficient vitamin B₁₂ to prevent the megaloblastic anemia. If autoantibodies to intrinsic factor should also develop in the GI lumen, however, B₁₂ absorption would cease and pernicious anemia would develop.

Hashimoto's thyroiditis is associated with autoantibodies to thyroglobulin, the microsomes of thyroid epithelial cells, a thyroid cell surface antigen, and a second colloid antigen. Tissue injury and eventual myxedema may be mediated both by the cytotoxicity of the microsomal antibody and by the activity of specifically committed T cells. Low-titered antibodies are also found in patients with primary myxedema, suggesting that it is the end result of unrecognized autoimmune thyroiditis. An autoimmune reaction is also involved in thyrotoxicosis (Graves' disease), and about 10% of patients eventually develop myxedema spontaneously; many more do so after ablative therapy. Other antibodies unique to Graves' disease are called thyroid-stimulating antibodies. They react with thyroid-stimulating hormone (TSH) receptors in the gland and have the same effect as TSH on thyroid cell function.

### B. Rheumatoid Arthritis

Rheumatoid Arthritis is a chronic syndrome characterized by nonspecific, usually symmetric inflammation of the peripheral joints, potentially resulting in progressive destruction of articular and periarticular structures, with or without generalized manifestations.

### 1. Etiology and Pathology

The cause is unknown. A genetic predisposition has been identified and, in white populations, localized to a pentapeptide in the HLA-DR β₁ locus of class II histocompatibility genes. Environmental factors may also play a role. Immunologic changes may be initiated by multiple factors. About 1% of all populations are affected, women two to three times more often than men. Onset may be at any age, most often between 25 and 50 years.

Prominent immunologic abnormalities that may be important in pathogenesis include immune complexes found in joint fluid cells and in vasculitis. Plasma cells produce antibodies (*e.g.*, rheumatoid factor ("RF")) that contribute to these complexes. Lymphocytes that infiltrate the synovial tissue are primarily T helper cells, which can produce pro-inflammatory cytokines. Macrophages and their cytokines (*e.g.,* tumor necrosis factor, granulocyte-macrophage colony-stimulating factor) are also abundant in diseased synovium. Increased adhesion molecules contribute to inflammatory cell emigration and retention in the synovial tissue. Increased macrophage-derived lining cells are prominent along with some lymphocytes and vascular changes in early disease.

In chronically affected joints, the normally delicate synovium develops many villous folds and thickens because of increased numbers and size of synovial lining cells and colonization by lymphocytes and plasma cells. The lining cells produce various materials, including collagenase and stromelysin, which can contribute to cartilage destruction; interleukin-1, which stimulates lymphocyte proliferation; and prostaglandins. The infiltrating cells, initially perivenular but later forming lymphoid follicles with germinal centers, synthesize interleukin-2, other cytokines, RF, and other immunoglobulins. Fibrin deposition, fibrosis, and necrosis also are present. Hyperplastic synovial tissue (pannus) may erode cartilage, subchondral bone, articular capsule, and ligaments. PMNs are not prominent in the synovium but often predominate in the synovial fluid.

Rheumatoid nodules occur in up to 30% of patients, usually subcutaneously at sites of chronic irritation (*e.g.,* the extensor surface of the forearm). They are nonspecific necrobiotic granulomas consisting of a central necrotic area surrounded by palisaded mononuclear cells with their long axes radiating from the center, all enveloped by lymphocytes and plasma cells. Vasculitis may be found in skin, nerves, or visceral organs in severe cases of RA but is clinically significant in only a few cases.

### 2. Symptoms and Signs

Onset is usually insidious, with progressive joint involvement, but may be abrupt, with simultaneous inflammation in multiple joints. Tenderness in nearly all inflamed joints is the most sensitive physical finding. Synovial thickening, the most specific physical finding, eventually occurs in most involved joints. Symmetric involvement of small hand joints (especially proximal interphalangeal and metacarpophalangeal), foot joints (metatarsophalangeal), wrists, elbows, and ankles is typical, but initial manifestations may occur in any joint.

Stiffness lasting > 30 min on arising in the morning or after prolonged inactivity is common; early afternoon fatigue and malaise also occur. Deformities, particularly flexion contractures, may develop rapidly; ulnar deviation of the fingers with slippage of the extensor tendons off the metacarpophalangeal joints is a typical late result. Carpal tunnel syndrome can result from wrist synovitis. Ruptured popliteal cysts can mimic deep vein thrombosis.

Subcutaneous rheumatoid nodules are not usually an early manifestation. Visceral nodules, vasculitis causing leg ulcers or mononeuritis multiplex, pleural or pericardial effusions, lymphadenopathy, Felty's syndrome, Sjögren's syndrome, and episcleritis are other extra-articular manifestations. Fever may be present and is usually low-grade, except in adult-onset Still's disease, a seronegative RA-like polyarthritis with prominent systemic features.

### 3. Laboratory Findings

Blood tests are helpful. A normochromic (or slightly hypochromic)-normocytic anemia, typical of other chronic diseases, occurs in 80% of cases; the Hb is usually > 10 g/dL but may rarely be as low as 8 g/dL. Superimposed iron deficiency or other causes of anemia should be sought if Hb is < 10 g/dL. Neutropenia occurs in 1 to 2% of cases, often with splenomegaly (Felty's syndrome). Mild polyclonal hypergammaglobulinemia and thrombocytosis may be present.

The ESR is elevated in 90% of cases. Antibodies to altered δ-globulin, so-called rheumatoid factors (RFs), as detected by agglutination tests (*e.g.,* the latex fixation test uses human IgG adsorbed to particulate latex) that show IgM RF, occur in about 70% of cases. Although RFs are not specific for RA and are found in many diseases (*e.g.*, granulomatous diseases, chronic infections, hepatitis, sarcoidosis, subacute bacterial endocarditis), a high RF titer helps confirm the diagnosis. In most laboratories, a latex fixation tube dilution titer of 1:160 is considered the lowest value favoring a diagnosis of RA. RF titers are also often measured by nephelometry (< 20 IU/mL is considered negative). A very high RF titer suggests a worse prognosis and is often associated with progressive disease, nodules, vasculitis, and pulmonary involvement. The titer may be influenced by treatment and often falls as inflammatory joint activity decreases.

The synovial fluid, abnormal during active joint inflammation, is cloudy but sterile, with reduced viscosity and usually 3,000 to 50,000 WBCs/µL. Of these cells, PMNs typically predominate, but > 50% may be lymphocytes and other mononuclear cells. WBC cytoplasmic inclusions may be seen on a wet smear but are also present in other inflammatory effusions. Synovial fluid complement is often < 30% of the serum level. Crystals are absent.

On x-ray, only soft tissue swelling is seen in the first months of disease. Subsequently, periarticular osteoporosis, joint space (articular cartilage) narrowing, and marginal erosions may be present. The rate of deterioration, seen on x-ray and clinically, is highly variable, but erosions as a sign of bony damage may occur within the first year.

### 4. Diagnosis

The American College of Rheumatology has developed simplified criteria for the classification of RA. Primarily intended as a communication aid for those in clinical research, these criteria can also help guide clinical diagnosis.

Almost any other disease that causes arthritis must still be considered. Some patients with crystal-induced arthritis meet these new criteria; synovial fluid examination often helps exclude these cases. However, two diseases causing arthritis can very occasionally coexist. When diagnosis is in doubt, unexplained subcutaneous nodules may be aspirated or biopsied to differentiate gouty tophi, amyloid, and other causes.

SLE may mimic RA. SLE usually may be distinguished by the characteristic skin lesions on light-exposed areas, temporal-frontal hair loss, oral and nasal mucosal lesions, nonerosive arthritis, joint fluid with often < 2000 WBCs/µL (predominantly mononuclear cells), positive antibodies to double-stranded DNA, renal disease, and low serum complement levels. Positive antinuclear antibodies and some features of SLE may occur along with otherwise typical RA, giving rise to the term "overlap syndrome." Some of these patients may have severe RA; others have associated SLE or other collagen disease. Polyarteritis, progressive systemic sclerosis, dermatomyositis, and polymyositis may have features that resemble RA.

Other systemic diseases may cause symptoms similar to RA. Sarcoidosis, amyloidosis, Whipple's disease, and other systemic diseases may involve joints; tissue biopsy sometimes helps differentiate these conditions. Acute rheumatic fever is differentiated by a migratory pattern of joint involvement and evidence of antecedent streptococcal infection (culture or changing antistreptolysin-O titer). Changing heart murmurs, chorea, and erythema marginatum are much less common in adults than in children. Infectious arthritis usually is monarticular or asymmetric. Diagnosis depends on identification of the causative agent. Infection may be superimposed on a joint affected by RA. Gonococcal arthritis usually presents as a migratory arthritis that involves tendons around the wrist and ankle and finally settles in one or two joints. Lyme disease can occur without the classic history of tick bite and rash; it may be screened for serologically. Knees are most commonly involved. Reiter's syndrome (reactive arthritis) is characterized by evidence of antecedent urethritis or diarrhea; asymmetric involvement of the heel, sacroiliac joints, and large joints of the leg; urethritis; conjunctivitis; iritis; painless buccal ulcers; balanitis circinata; or keratoderma blennorrhagicum on the soles and elsewhere. Serum and joint fluid complement levels are often elevated. Psoriatic arthritis tends to be asymmetric and is not usually associated with RF, but differentiation may be difficult in the absence of characteristic nail or skin lesions. Distal interphalangeal joint involvement and arthritis mutilans may be suggestive.

Ankylosing spondylitis may be differentiated by its predilection for males, spinal and axial distribution of joint involvement, absence of subcutaneous nodules, and negative RF test. Gout may be monarticular or polyarticular, with complete recovery between acute attacks early in the disease. Chronic gout may mimic RA. Typical needlelike or rodlike birefringent monosodium urate crystals with negative elongation are present in the synovial effusion and may be seen by compensated polarized light. Calcium pyrophosphate dihydrate crystal deposition disease may produce monarticular or polyarticular acute or chronic arthritis. However, the presence of weakly birefringent rodlike or rhomboid calcium pyrophosphate dihydrate crystals with positive elongation in joint fluid and x-ray evidence of articular cartilage calcification (chondrocalcinosis) differentiate this condition.

Osteoarthritis often involves the proximal and distal interphalangeal joints, first carpometacarpal and first metatarsophalangeal joints, knee joints, and spine. Symmetry of involvement, prominent joint swelling (mostly caused by bony enlargement) with some signs of inflammation, joint instability, and subchondral cysts on x-ray may be confusing; the absence of significant amounts of RF, rheumatoid nodules, and systemic involvement along with the characteristic osteoarthritis pattern of joint involvement with synovial fluid WBC counts < 1000 to 2000/µL permit differentiation from RA.

### C. Systemic Lupus Erythematosus

Systemic Lupus Erythematosus ("SLE") is a chronic inflammatory connective tissue disorder of unknown cause that can involve joints, kidneys, serous surfaces, and vessel walls and that occurs predominantly in young women but also in children.

Of SLE cases, 90% occur in women. Increased awareness of mild forms of SLE has resulted in a worldwide rise in reported cases. In some countries, the prevalence of SLE rivals that of RA. The sera of most patients contain antinuclear antibodies (ANA), often including anti-DNA antibodies.

### 1. Pathology, Symptoms, and Signs

Clinical findings vary greatly. SLE may begin abruptly with fever, simulating acute infection, or may develop insidiously over months or years with episodes of fever and malaise. Vascular headaches, epilepsy, or psychoses may be initial findings. Manifestations referable to any organ system may appear. Articular symptoms, ranging from intermittent arthralgias to acute polyarthritis, occur in approximately 90% of patients and may exist for years before other manifestations appear. In long-standing disease, capsular insertional erosions at the metacarpophalangeal joints with marked secondary joint deformity may occur without x-ray evidence of obvious marginal erosions (Jaccoud's arthritis). However, most lupus polyarthritis is nondestructive and nondeforming.

Cutaneous lesions include characteristic malar butterfly erythema; discoid lesions; and erythematous, firm, maculopapular lesions of the face, exposed areas of the neck, upper chest, and elbows. Blistering and ulceration are rare, although recurrent ulcers on mucous membranes (particularly the central portion of the hard palate near the junction of the hard and soft palate, the buccal and gum mucosa, and the anterior nasal septum) are common. Generalized or focal alopecia is common during active phases of SLE. Mottled erythema on the sides of the palms with extension onto the fingers, periungual erythema with edema, and macular reddish purple lesions on the volar surfaces of the fingers also may occur. Purpura may develop secondary to thrombocytopenia or necrotizing angiitis of small vessels. Photosensitivity occurs in 40% of patients.

Recurrent pleurisy, with or without effusion, is common. Lupus pneumonitis is rare, although minor pulmonary function abnormalities are common. Life-threatening pulmonary hemorrhage may rarely occur. Pericarditis is often present. More serious rare complications are coronary artery vasculitis or fibrosing myocarditis.

Generalized adenopathy is common, particularly in children, young adults, and blacks. Splenomegaly occurs in 10% of patients. Histologically, the spleen may show periarterial fibrosis (onion skin lesion).

CNS involvement can cause headaches, personality changes, stroke, epilepsy, psychoses, and organic brain syndrome. Cerebral or pulmonary artery thrombosis or embolism, although rare, is associated with anticardiolipin antibodies.

Renal involvement may be benign and asymptomatic or relentessly progressive and fatal. The most common manifestation is proteinuria. The histopathology of the renal lesion varies from a focal, usually benign glomerulitis to a diffuse membranoproliferative glomerulonephritis. Because milder cases of lupus have been increasingly detected, the percentage of patients with clinically significant renal disease has declined.

Acute lupus hemophagocytic syndrome is a rare presentation of SLE, with fever and fulminant pancytopenia, described in Asians (particularly of Chinese descent), among whom SLE has a high incidence. Bone marrow shows proliferation of reactive histiocytes, with phagocytosis of hemopoietic cells (an example of the reactive hemophagocytic syndrome). There is no evidence of underlying infection. Patients respond promptly to corticosteroids.

### 2. Laboratory Findings

The fluorescent test for ANA screens for SLE; positive ANA tests (usually in high titer) occur in > 98% of SLE patients and should lead to more specific tests for anti-double-stranded DNA antibodies (an enzyme-linked immunosorbent assay or the slightly less sensitive but more specific crithidia slide method). High titers of anti-double-stranded DNA antibodies, if present, are highly specific for SLE.

Other ANA and anticytoplasmic antibodies (*e.g.*, Ro [SSA], La [SSB], Sm, RNP, Jo-1) are diagnostically valuable in SLE or in other connective tissue diseases (as described below). Because Ro is predominantly cytoplasmic, anti-Ro antibodies may occasionally be found in ANA-negative SLE patients presenting with chronic cutaneous lupus. Anti-Ro is the causal antibody for neonatal lupus and congenital heart block. Anti-Sm is highly specific for SLE but, as with anti-double-stranded DNA, is not sensitive.

False-positive serologic tests for syphilis occur in 5 to 10% of SLE patients. They may be associated with a positive test for the lupus anticoagulant or a prolonged partial thromboplastin time. Abnormal values in one or more of these assays indicate the presence of antiphospholipid antibodies (*e.g.*, anticardiolipin antibodies), which are associated with arterial or venous thrombosis, spontaneous abortion, late fetal loss, and thrombocytopenia. Anticardiolipin antibodies may be directly assessed by enzyme-linked immunosorbent assay.

Serum complement levels are often depressed in active disease and are usually lowest in patients with active nephritis. ESR is elevated almost uniformly during active disease. C-reactive protein levels may be strikingly low in SLE, even with ESR > 100 mm/h. Leukopenia is the rule, notably lymphopenia in active SLE. Hemolytic anemia may occur. Autoimmune thrombocytopenia may be severe and life threatening. The presentation of SLE is occasionally indistinguishable from idiopathic thrombocytopenic purpura.

Renal damage may become evident at any time, even when other features of SLE are absent. A high or rising level of anti-DNA antibody may predict an increased risk of lupus nephritis. Renal biopsy is usually not necessary for diagnosis but may help evaluate the status of renal disease (*i.e.,* active inflammation vs. postinflammatory scarring) and guide medical therapy. Urinalysis may be repeatedly normal despite early renal involvement confirmed by biopsy; thus, it should be performed at regular intervals while monitoring patients in apparent remission. RBC and granular casts suggest more active nephritis.

### 3. Diagnosis

SLE is obvious when a patient (particularly a young woman) is febrile with an erythematous skin rash, polyarthritis, evidence of renal disease, intermittent pleuritic pain, leukopenia, and hyperglobulinemia with anti-double-stranded DNA antibodies. Early-stage SLE may be difficult to differentiate from other connective tissue disorders and may be mistaken for RA if arthritic symptoms predominate. Mixed connective tissue disease has the clinical features of SLE with overlapping features of systemic sclerosis, rheumatoid-like polyarthritis, and polymyositis or dermatomyositis.

Meticulous evaluation and long-term observation may be required before SLE is diagnosed. Patients with discoid lesions must be evaluated to differentiate discoid lupus erythematosus from SLE. Some drugs (*e.g.,* hydralazine, procainamide, β-blockers) produce positive ANA tests and, occasionally, a lupuslike syndrome associated with antihistone antibodies. These features usually disappear if the drug is withdrawn promptly. The American College of Rheumatology has proposed criteria for the classification (not for diagnosis) of SLE.

### 4. Prognosis

The more severe the disease, the greater the risk of iatrogenic drug-induced complications, which further increase morbidity and mortality. Examples include infection from immunosuppression and coronary artery disease from chronic corticosteroid use. In general, the course of SLE is chronic and relapsing, often with long periods (years) of remission. During the past two decades, the prognosis has improved markedly. Provided the initial acute phase is controlled, the long-term prognosis is usually good. Flares are rare after menopause, although late-onset SLE does occur and may be difficult to diagnose. The 10-year survival in most developed countries is > 95%. This very improved prognosis underlines the paramount importance of early diagnosis of SLE. Sometimes, however, the presentation may be acute and disastrous (*e.g.*, with cerebral thrombosis or late fetal loss).

### 5. Treatment

Management of idiopathic SLE depends on its manifestations and severity. To simplify therapy, SLE should be classified as mild (fever, arthritis, pleurisy, pericarditis, headache, or rash) or severe (life-threatening disease, *e.g.*, hemolytic anemia, thrombocytopenic purpura, massive pleural and pericardial involvement, significant renal damage, acute vasculitis of the extremities or GI tract, florid CNS involvement). The course is unpredictable.

Mild or remittent disease may require little or no therapy. Arthralgias are usually controlled with NSAIDs. Aspirin is useful, especially in patients with the thrombotic tendency associated with anticardiolipin antibodies, but high doses in SLE may cause liver toxicity. Antimalarials help, particularly when joint and skin manifestations are prominent. Regimens vary, but hydroxychloroquine 200 mg po once or twice per day is preferred. An alternative is chloroquine 250 mg/day po or quinacrine (mepacrine) 50 to 100 mg/day po. Combinations of these drugs are sometimes used. Ophthalmologic examination usually is advised at 6-month intervals, although this practice may be excessively cautious because these doses are modest and recent data suggest that hydroxychloroquine has very low retinal toxicity. DHEA 50 to 200 mg/day may decrease the need for other drugs, especially corticosteroids. Larger doses are less well-tolerated for their androgenic effect and are probably not more effective than the lower dose range.

Severe disease requires immediate corticosteroid therapy. A combination of prednisone and immunosuppressive drugs is recommended in active, serious CNS lupus or active reversible lupus nephritis. Starting oral prednisone dosages for specific manifestations are as follows: hemolytic anemia, 60 mg/day; thrombocytopenic purpura, 40 to 60 mg/day (platelet count may not rise for 4 to 6 wk); severe polyserositis, 20 to 60 mg/day (response begins within days); renal damage, 40 to 60 mg/day in combination with immunosuppressive drugs. Improvement does not usually occur for 4 to 12 wk and may not be evident until corticosteroid dosage is reduced. Azathioprine in doses up to 2.5 mg/kg/day or cyclophosphamide 2.5 mg/kg/day is often used as an immunosuppressive drug for renal SLE. There is a strong trend toward intermittent or cyclical "pulsing" with immunosuppressive drugs, such as cyclophosphamide approximately 500 mg IV (together with mesna and fluid loading to protect the bladder), repeated monthly for >= 6 to 12 months depending on the renal response and hematologic tolerance.

Acute vasculitis and severe CNS lupus are treated with the same regimens as for renal damage, above. In situ thrombosis or embolism of cerebral, pulmonary, or placental vessels may require short-term treatment with heparin and longer term management with warfarin. In CNS lupus or other critical crises, methylprednisolone 1000 mg by slow (1-h) IV infusion on 3 successive days often is the initial form of treatment, together with IV cyclophosphamide, as above.

### D. Crohn's disease

Crohn's Disease is a nonspecific chronic transmural inflammatory disease that most commonly affects the distal ileum and colon but may occur in any part of the GI tract.

### 1. Etiology and Epidemiology

The fundamental cause of Crohn's disease is unknown. Evidence suggests that a genetic predisposition leads to an unregulated intestinal immune response to an environmental, dietary, or infectious agent. However, no inciting antigen has been identified. Cigarette smoking seems to contribute to the development or exacerbation of Crohn's disease.

Over the past few decades, incidence of Crohn's disease has increased in Western populations of Northern European and Anglo-Saxon ethnic derivation, third-world populations, blacks, and Latin Americans. The disease occurs about equally in both sexes and is more common among Jews. Approximately one of six patients has at least one first-degree relative with the same disease or, less frequently, with ulcerative colitis. Most cases begin in patients < 30 years, with the peak incidence in those aged 14 to 24 years.

### 2. Pathology

The earliest mucosal lesion of Croln's disease is crypt injury in the form of inflammation (cryptitis) and crypt abscesses, which progress to tiny focal aphthoid ulcers, usually located over nodules of lymphoid tissue. In some cases, these lesions regress; in others, the inflammatory process evolves with influx and proliferation of macrophages and other inflammatory cells, occasionally forming noncaseating granulomas with multinucleated giant cells.

Transmural spread of inflammation leads to lymphedema and bowel wall thickening, which may eventually result in extensive fibrosis. Development of patchy mucosal ulcers and longitudinal and transverse ulcers with intervening mucosal edema frequently creates a characteristic cobblestoned appearance. The attached mesentery is thickened and lymphedematous; mesenteric fat typically extends onto the serosal surface of the bowel. Mesenteric lymph nodes often enlarge. Transmural inflammation, deep ulceration, edema, muscular proliferation, and fibrosis cause deep sinus tracts and fistulas, mesenteric abscesses, and obstruction, which are the major local complications.

Granulomas can occur in lymph nodes, peritoneum, the liver, and all layers of the bowel wall and are occasionally seen at laparotomy or laparoscopy as miliary nodules. Although pathognomonic, granulomas are absent in up to 50% of patients and are therefore not essential to diagnose Crohn's disease. They appear to have no definitive bearing on the clinical course.

Segments of diseased bowel are characteristically sharply demarcated from adjacent normal bowel ("skip areas")--thus the name regional enteritis. Of all cases of Crohn's disease, about 35% involve the ileum (ileitis); about 45% involve the ileum and colon (ileocolitis), with a predilection for the right side of the colon; and about 20% involve the colon alone (granulomatous colitis). Occasionally, the entire small bowel is involved (jejunoileitis), and rarely, the stomach, duodenum, or esophagus. The perianal region is also affected in 1/4 to 1/3 of cases.

### 3. Symptoms. Signs, and Complications

Chronic diarrhea with abdominal pain, fever, anorexia, weight loss, and a right lower quadrant mass or fullness are the most common presenting features. However, many patients are first seen with an acute abdomen that simulates acute appendicitis or intestinal obstruction. About 1/3 of patients have a history of perianal disease, especially fissures and fistulas, which are sometimes the most prominent or even initial complaint. In children, extraintestinal manifestations frequently predominate over GI symptoms. Arthritis, FUO, anemia, or growth retardation may be a presenting symptom, and abdominal pain or diarrhea may be absent.

The most common patterns of Crohn's disease pathology are (1) inflammation characterized by right lower quadrant abdominal pain and tenderness; (2) recurrent partial obstruction caused by intestinal stenosis and leading to severe colic, abdominal distention, constipation, and vomiting; (3) diffuse jejunoileitis, with inflammation and obstruction resulting in malnutrition and chronic debility; and (4) abdominal fistulas and abscesses, usually late developments, often causing fever, painful abdominal masses, and generalized wasting.

Obstruction; development of enteroenteric, enterovesical, retroperitoneal, or enterocutaneous fistulas; and abscess formation are common complications of inflammation. Intestinal bleeding, perforation, and small-bowel cancer develop rarely. When the colon alone is affected, the clinical picture may be indistinguishable from that of ulcerative colitis.

Extraintestinal manifestations are categorized as:
a. Complications that usually parallel the activity of the intestinal disease and possibly represent acute immunologic or microbiologic concomitants of the bowel inflammation: peripheral arthritis, episcleritis, aphthous stomatitis, erythema nodosum, and pyoderma gangrenosum. These manifestations may be reported by > 1/3 of patients hospitalized with inflammatory bowel disease. They are twice as common when colitis is present as when disease is confined to the small bowel. When extraintestinal manifestations occur, they are multiple in about 1/3 of patients.
b. Disorders associated with inflammatory bowel disease but running an independent course: ankylosing spondylitis, sacroiliitis, uveitis, and primary sclerosing cholangitis. The genetic association of these syndromes and of Crohn's disease (and ulcerative colitis) with the HLA antigen B27 is discussed under the extracolonic complications of ulcerative colitis, below.
c. Complications that relate directly to disrupted bowel physiology: kidney stones from disorders of uric acid metabolism, impaired urinary dilution and alkalinization, and excessive dietary oxalate absorption; UTIs, especially with fistulization into the urinary tract; and hydroureter and hydronephrosis from ureteral compression by retroperitoneal extension of the intestinal inflammatory process. Other bowel-related complications include malabsorption, especially in the face of extensive ileal resection or bacterial overgrowth from chronic small-bowel obstruction or fistulization; gallstones, related to impaired ileal reabsorption of bile salts; and amyloidosis, secondary to long-standing inflammatory and suppurative disease. Thromboembolic complications may occur, usually with severe disease activity, as a result of hypercoagulability associated with altered levels of clotting factors and platelet abnormalities.

### 4. Diagnosis

Crohn's disease should be suspected in a patient with the inflammatory or obstructive symptoms described above and in a patient without prominent GI symptoms but with perianal fistulas or abscesses or with otherwise unexplained arthritis, erythema nodosum, fever, anemia, or stunted growth (in a child).

Laboratory findings are nonspecific and may include anemia, leukocytosis, hypoalbuminemia, and increased levels of acute-phase reactants reflected in elevated ESR, C-reactive protein, or orosomucoids. Elevated alkaline phosphatase and δ-glutamyl transpeptidase accompanying colonic disease often reflect primary sclerosing cholangitis.

Definitive diagnosis is usually made by x-ray. Barium enema x-ray may show reflux of barium into the terminal ileum with irregularity, nodularity, stiffness, wall thickening, and a narrowed lumen. A small-bowel series with spot x-rays of the terminal ileum usually most clearly shows the nature and extent of the lesion. An upper GI series without small-bowel follow-through usually misses the diagnosis.

In advanced cases, the string sign may be seen with marked ileal strictures and separation of bowel loops. In earlier cases, x-ray diagnosis may sometimes be difficult, but air double-contrast barium enema and enteroclysis may show superficial aphthous and linear ulcers. In questionable cases, colonoscopy and biopsy may help confirm the diagnosis of Crohn's colitis and allow direct visualization and biopsy of the terminal ileum. Upper GI endoscopy may identify gastroduodenal involvement in Crohn's disease patients with upper GI symptoms. Although CT can detect extramural complications (*e*.*g*., fistulas, abscesses, masses), it is not routinely needed for initial diagnosis. Ultrasound may help delineate gynecologic pathology in women with lower abdominal and pelvic pain.

### 5. Differential Diagnosis

Differentiation from ulcerative colitis may be difficult in the 20% of cases in which Crohn's disease is confined to the colon (Crohn's colitis). The principal differential diagnoses are acute infectious (self-limited) colitis and ulcerative colitis. Acute infectious colitis is best established by stool culture, rectal biopsy, and watchful waiting. Although perinuclear antineutrophil cytoplasmic antibodies are present in 60 to 70% of ulcerative colitis patients and in only 5 to 20% of Crohn's disease patients, and anti-*Saccharomyces cerevisiae* antibodies are relatively specific for Crohn's disease, these tests are not sufficiently refined in routine clinical application as to reliably separate the two diseases.

Crohn's disease of the small bowel (ileitis) requires differentiation from other inflammatory, infectious, and neoplastic disorders in the right lower quadrant. If in the acute presentation a prior history of chronic bowel symptoms has not been elicited, ileitis may be first diagnosed during surgical exploration for suspected acute appendicitis. Periappendiceal abscess may produce more chronic symptoms and thus be more difficult to diagnose clinically.

Pelvic inflammatory disease, ectopic pregnancy, and ovarian cysts and tumors also produce right lower quadrant inflammatory signs and must be ruled out when considering Crohn's disease in women. Cancer of the cecum, ileal carcinoid, lymphosarcoma, systemic vasculitis, radiation enteritis, ileocecal TB, and ameboma may mimic the x-ray findings of Crohn's disease. AIDS-related opportunistic infections (*e.g., Mycobacterium avium-intracellulare,* cytomegalovirus) must also be considered as causes of localized ileitis.

*Yersinia enterocolitica* enteritis must be excluded if an inflamed, edematous terminal ileum and associated mesenteric adenitis are seen during surgery for acute right lower quadrant pain. Although *Yersinia* enteritis is self-limited without chronic intestinal sequelae, the initial clinical picture may be indistinguishable from Crohn's disease, so appropriate serologic and bacteriologic studies are necessary. In questionable cases, a 3-month follow-up x-ray of the terminal ileum is valuable, because *Yersinia* enteritis will usually resolve completely by this time but Crohn's disease will not.

Nongranulomatous ulcerative jejunoileitis has features of both Crohn's disease and sprue, with malabsorption, small-bowel ulceration, and villous atrophy, but it lacks granulomatous pathology, fistulization, and extraintestinal manifestations of Crohn's disease. Eosinophilic gastroenteritis generally has prominent gastric involvement (rare in Crohn's disease) and is often associated with peripheral eosinophilia, which is the clue to diagnosis.

### 6. Prognosis

Although spontaneous remission or medical therapy may result in a prolonged asymptomatic interval, established Crohn's disease is rarely cured but instead is characterized by intermittent exacerbations. In the absence of surgical intervention, the disease never extends into new areas of small bowel beyond its initial distribution at first diagnosis. With judicious medical and, where appropriate, surgical therapy, most patients with Crohn's disease function well and adapt successfully. Disease-related mortality is very low and continues to decrease.

GI cancer, including cancer of the colon and small bowel, is the leading cause of Crohn's disease-related death. Patients with long-standing Crohn's disease of the small bowel are at increased risk of small-bowel cancer, with bowel in continuity as well as in bypassed loops. Furthermore, patients with Crohn's disease of the colon have a long-term risk of colorectal cancer equal to that of ulcerative colitis, given the same extent and duration of disease.

Approximately 70% of Crohn's disease patients ultimately require surgery. Furthermore, Crohn's disease is likely to recur even after resection of all clinically apparent disease.

Immunomodulating drugs, particularly the antimetabolites azathioprine and 6-mercaptopurine, are effective as long-term therapy for Crohn's disease. Azathioprine 2.0 to 3.5 mg/kg/day or 6-mercaptopurine 1.5 to 2.5 mg/kg/day po significantly improves overall clinical status, decreases corticosteroid requirements, heals fistulas, and maintains remission for many years. However, these drugs often do not produce clinical benefits for 3 to 6 months, and side effects of allergy, pancreatitis, and leukopenia must be watched for.

Methotrexate 25 mg IM or sc once/wk benefits some patients with severe corticosteroid-refractory disease, even those who have failed to respond to azathioprine or 6-mercaptopurine. High-dose cyclosporine has demonstrated benefits in inflammatory and fistulous disease, but its long-term use is contraindicated by multiple toxicities. Infliximab, a monoclonal antibody that inhibits tumor necrosis factor, may be given IV for moderate to severe Crohn's disease (especially fistulous disease) refractory to other treatments; long-term efficacy and side effects remain to be determined. Other potential immunoregulatory treatments include interleukin-1 blockers, antibody to interleukin-12, anti-CD4 antibodies, adhesion molecule inhibitors, and down-regulatory cytokines. These many experimental treatment approaches attest to the inadequacy of current drug therapy for Crohn's disease.

Some patients with intestinal obstruction or fistulas have improved with elemental diets or hyperalimentation, at least over a short term, and children often achieve increased rates of growth. Thus, these measures may serve as preoperative or adjunctive therapy and may even be valuable as primary therapy.

Surgery is usually necessary for recurrent intestinal obstruction or intractable fistulas or abscesses. Resection of the grossly involved bowel may reduce symptoms indefinitely but does not cure the disease. Sulfasalazine has not been shown to prevent postoperative recurrence, but mesalamine >= 2.0 g/day may be effective. The recurrence rate, defined by endoscopic lesions at the anastomotic site, is > 70% at 1 year and > 85% at 3 years; defined by clinical symptoms, it is about 25 to 30% at 3 years and 40 to 50% at 5 years. Ultimately, further surgery is required in nearly 50% of cases. However, recurrence rates appear to be reduced by early postoperative prophylaxis with mesalamine, metronidazole, or possibly 6-mercaptopurine. Moreover, when surgery has been performed for specific complications or failure of medical therapy, most patients experience an improved quality of life.

### E. Ulcerative Colitis

Ulcerative colitis is a chronic, inflammatory, and ulcerative disease arising in the colonic mucosa, characterized most often by bloody diarrhea.

### 1. Etiology and Epidemiology

The cause of ulcerative colitis is unknown. Evidence suggests that a genetic predisposition leads to an unregulated intestinal immune response to an environmental, dietary, or infectious agent. However, no inciting antigen has been identified. The evidence for a specific microbial etiology for ulcerative colitis is even less convincing than for Crohn's disease, and the familial tendency is less pronounced. Unlike in Crohn's disease, current cigarette smoking appears to decrease risk. Like Crohn's disease, ulcerative colitis may afflict people at any age, but the age-onset curve shows a bimodal distribution, with a major peak at ages 15 to 30 and a second smaller peak at ages 50 to 70; however, this later peak may include some cases of ischemic colitis.

### 2. Pathology

Pathologic changes begin with degeneration of the reticulin fibers beneath the mucosal epithelium, occlusion of the subepithelial capillaries, and progressive infiltration of the lamina propria with plasma cells, eosinophils, lymphocytes, mast cells, and PMNs. Crypt abscesses, epithelial necrosis, and mucosal ulceration ultimately develop. The disease usually begins in the rectosigmoid and may extend proximally, eventually involving the entire colon, or it may involve most of the large bowel at once.

Ulcerative proctitis, which is localized to the rectum, is a very common and more benign form of ulcerative colitis. It is often refractory to treatment and undergoes late proximal spread in about 20 to 30% of cases.

### 3. Symptoms and Signs

Bloody diarrhea of varied intensity and duration is interspersed with asymptomatic intervals. Usually an attack begins insidiously, with increased urgency to defecate, mild lower abdominal cramps, and blood and mucus in the stools. However, an attack may be acute and fulminant, with sudden violent diarrhea, high fever, signs of peritonitis, and profound toxemia. Some cases develop following a documented infection (*e.g.*, amebiasis, bacillary dysentery).

When ulceration is confined to the rectosigmoid, the stool may be normal or hard and dry, but rectal discharges of mucus loaded with RBCs and WBCs accompany or occur between bowel movements. Systemic symptoms are mild or absent. If ulceration extends proximally, stools become looser and the patient may have > 10 bowel movements/day, often with severe cramps and distressing rectal tenesmus, without respite at night. The stools may be watery, may contain mucus, and frequently consist almost entirely of blood and pus. Malaise, fever, anemia, anorexia, weight loss, leukocytosis, hypoalbuminemia, and elevated ESR may be present with extensive active ulcerative colitis.

### 4. Complications

Bleeding is the most common local complication. Another particularly severe complication, toxic colitis, occurs when transmural extension of ulceration results in localized ileus and peritonitis. As toxic colitis progresses, the colon loses muscular tone and begins to dilate within hours or days. Plain x-rays of the abdomen show intraluminal gas accumulated over a long, continuous, paralyzed segment of colon--a result of lost muscle tone.

Toxic megacolon (or toxic dilation) exists when the diameter of the transverse colon exceeds 6 cm. The severely ill patient has a fever to 40° C (104° F), leukocytosis, abdominal pain, and rebound tenderness. This condition usually occurs spontaneously in the course of especially severe colitis, but some cases may be precipitated by overzealous use of narcotic or anticholinergic antidiarrheal drugs. Treatment must be given in the early stages, preferably before full-blown megacolon occurs, to avert dangerous complications (*e.g.*, perforation, generalized peritonitis, septicemia). With prompt, effective treatment, the mortality rate may be held at < 4% but may be > 40% if perforation occurs.

Major perirectal complications, such as those in granulomatous colitis (*e.g.*, fistulas, abscesses), do not occur.

The incidence of colon cancer is increased when the entire colon is involved and the disease lasts for > 10 years, independent of disease activity. After 10 years, the cancer risk in extensive colitis appears to be about 0.5 to 1%/year. Although cancer incidence is highest in cases of universal ulcerative colitis, the risk is significantly increased with any extent of ulcerative colitis above the sigmoid. There is probably no higher absolute cancer risk among patients with childhood-onset colitis, independent of the longer duration of disease. The long-term survival after diagnosis of colitis-related cancer is about 50%, a figure comparable to that for colorectal cancer in the general population.

Regular colonoscopic surveillance, preferably during remission, is advised for patients whose disease duration (>= 8 to 10 years) and extent (beyond rectosigmoidal) place them at high risk of developing colon cancer. Endoscopic biopsies should be taken throughout the colon and reviewed by an experienced pathologist. Any grade of definite, confirmed dysplasia is a strong indication for colectomy because the likelihood of concomitant or imminent colorectal cancer may be as high as 80%. In such cases, corroboratory pathologic interpretation is important to distinguish between definite neoplastic dysplasia and reactive or regenerative atypia secondary to inflammation. However, delaying colectomy in favor of repeated follow-up surveillance is unwise if dysplasia is unequivocal. Pseudopolyps have no prognostic significance but may be difficult to distinguish from neoplastic polyps; thus, any suspicious polyp should undergo excision biopsy.

Extracolonic problems include peripheral arthritis, ankylosing spondylitis, sacroiliitis, anterior uveitis, erythema nodosum, pyoderma gangrenosum, episcleritis, and in children, retarded growth and development. Peripheral arthritis, skin complications, and episcleritis often fluctuate with the colitis, whereas spondylitis, sacroiliitis, and uveitis usually follow a course independent of the bowel disease. Most patients with spinal or sacroiliac involvement also have evidence of uveitis, and vice versa. These latter conditions may precede the colitis by many years and tend to occur more commonly in persons with the HLA-B27 antigen.

Minor changes in liver function tests are common, but clinically apparent liver disease occurs in only 3 to 5% of patients. Liver disease may manifest as fatty liver or more seriously as autoimmune hepatitis, primary sclerosing cholangitis, or cirrhosis. Primary sclerosing cholangitis (PSC) occurs in 5% of ulcerative colitis patients, most commonly in those who were young when the colitis began. PSC may precede symptomatic ulcerative colitis by many years and is more reliably diagnosed by endoscopic retrograde cholangiography than by liver biopsy. Some investigators believe that signs of subclinical ulcerative colitis, if systematically sought, could be found in all patients with PSC. A late complication of ulcerative colitis-associated PSC is cancer of the biliary tract, which may appear even 20 years after colectomy. More than 50% of PSC and cholangiocarcinoma cases in Western countries occur in patients with Crohn's disease or ulcerative colitis.

### 5. Diagnosis

The history and stool examination permit a presumptive diagnosis of ulcerative colitis that should always be confirmed by sigmoidoscopy, which provides a direct, immediate indication of disease activity. Total colonoscopy is not usually necessary before treatment and may be hazardous in active stages because of the risk of perforation. In early cases, the mucous membrane is finely granular and friable, with loss of the normal vascular pattern and often with scattered hemorrhagic areas; minimal trauma (friability) causes bleeding in multiple pinpoint spots. The mucosa soon breaks down into a red, spongy surface dotted with many tiny blood- and pus-oozing ulcers. As the mucosa becomes progressively involved, the inflammation and hemorrhage extend into the bowel muscle. Large mucosal ulcers with copious purulent exudate characterize severe disease. Islands of relatively normal or hyperplastic inflammatory mucosa (pseudopolyps) project above areas of ulcerated mucosa. Biopsies may be nonspecific and sometimes cannot exclude acute infectious (self-limited) colitis; however, features that suggest chronicity (*e.g.*, distorted crypt architecture, crypt atrophy, a chronic inflammatory infiltrate) support the diagnosis of ulcerative colitis. Even during asymptomatic intervals, the sigmoidoscopic appearance is rarely normal; some degree of friability or granularity almost always persists. There is loss of the nomial vascular pattern, and biopsy shows evidence of chronic inflammation.

Plain x-rays of the abdomen sometimes help to judge the severity and proximal extent of the colitis by showing loss of haustration, mucosal edema, and absence of formed stool in the diseased bowel. Barium enema, like colonoscopy, is not usually necessary before treatment and may be hazardous in active stages because of risk of perforation. Later in the course of disease, however, the entire colon should be evaluated to determine the extent of involvement. Total colonoscopy is the most sensitive and widely used method, although barium enema may be informative. Barium studies show loss of haustration, mucosal edema, minute serrations, or gross ulcerations in severe cases. A shortened, rigid colon with an atrophic or pseudopolypoid mucosa is often seen after several years' duration.

Colonoscopy with biopsy is mandatory to evaluate the nature of a stricture. Biopsy may also help distinguish ulcerative colitis from Crohn's disease if the inflammation is highly focal or if a granuloma is seen.

### 6. Differential Diagnosis

It is of utmost importance to exclude an infectious cause of acute colitis before treatment, especially during the first attack. Stool cultures for *Salmonella, Shigella,* and *Campylobacter* must be obtained, and *Entamoeba histolytica* should be excluded by examination of fresh, still warm stool specimens or of colonic exudate aspirated at sigmoidoscopy. Mucosal biopsies may yield additional etiologic information. When amebiasis is suspected because of epidemiologic or travel history, serologic titers should be obtained in addition to biopsies.

History of prior antibiotic use should prompt stool assay for *Clostridium difficile* toxin. A detailed sexual history should be obtained, particularly in male homosexuals, to rule out specific sexually transmitted diseases, such as gonorrhea, herpesvirus, and chlamydia. Opportunistic infections (*e.g.,* cytomegalovirus, *Mycobacterium avium-intracellulare*) must also be considered in immunosuppressed patients. In women using birth control pills, contraceptive-induced colitis is possible; it usually resolves spontaneously after hormone therapy is stopped. In elderly patients, especially those with a history of atherosclerotic heart disease, ischemic colitis should be considered. X-ray findings of thumbprinting and segmental distribution would further suggest this diagnosis. Colon cancer seldom produces fever or purulent rectal discharge but must be excluded as a cause of bloody diarrhea. Severe perianal disease, rectal sparing, absence of bleeding, and asymmetric or segmental involvement of the colon indicate Crohn's rather than ulcerative colitis.

### 7. Prognosis

Usually, ulcerative colitis is chronic with repeated exacerbations and remissions. A rapidly progressive initial attack becomes fulminant in nearly 10% of patients, with complications of massive hemorrhage, perforation, or sepsis and toxemia. Complete recovery after a single attack may occur in another 10%; however, there always remains the possibility of an undetected specific pathogen.

Nearly 1/3 of patients with extensive ulcerative colitis require surgery. Total proctocolectomy is curative: Life expectancy and quality of life are restored to normal, and the risk of colon cancer is eliminated.

Patients with localized ulcerative proctitis have the best prognosis. Severe systemic manifestations, toxic complications, and malignant degeneration are unlikely, and late extension of the disease occurs in only about 20 to 30%. Surgery is rarely required, and life expectancy is normal. The symptoms, however, may prove exceptionally stubborn and refractory. Moreover, because extensive ulcerative colitis may begin in the rectum and spread proximally, localized proctitis should not be definitively diagnosed until it has stayed localized for >= 6 months. Localized disease that later extends is often more severe and more refractory to therapy.

Immunomodulatory drugs are acceptable for some patients with refractory or corticosteroid-dependent ulcerative colitis. Azathioprine and 6-mercaptopurine inhibit T-cell function, and a decline in the activity of both natural killer cells and cytotoxic T cells is correlated with a clinical response. The full benefit of azathioprine 2 to 3.5 mg/kg/day or 6-mercaptopurine 1.5 to 2.5 mg/kg/day may not be seen for 3 to 6 months because these drugs are slow-acting. Complications include pancreatitis (an absolute contraindication to continued use) and reversible neutropenia, which simply requires a lower dose with regular monitoring of WBC counts.

Cyclosporine has a rapid onset and is primarily indicated for acute severe ulcerative colitis unresponsive to high-dose IV corticosteroids. Continuous IV infusion of cyclosporine can induce remission and avert surgery in about 80% of such cases. An additional 6 months of treatment with oral cyclosporine, ultimately shifting to azathioprine or 6-mercaptopurine, may sustain longer-term remissions in 50 to 60% of cases. Because serious and even fatal complications (*e.g.*, renal toxicity, seizures, opportunistic infections) may occur, patients generally are not offered cyclosporine unless the safer curative option of colectomy is infeasible or inappropriate. Candidates for cyclosporine therapy should be referred to centers experienced in its use.

### F. Multiple Sclerosis

Multiple Sclerosis (MS) is a slowly progressive Central Nervous System (CNS) disease characterized by disseminated patches of demyelination in the brain and spinal cord, resulting in multiple and varied neurologic symptoms and signs, usually with remissions and exacerbations.

### 1. Etiology and Incidence

The cause is unknown, but an immunologic abnormality is suspected. One postulated cause is infection by a latent virus (possibly by a human herpesvirus or retrovirus), in which viral activation and expression trigger a secondary immune response. An increased family incidence and association with certain HLA allotypes suggest genetic susceptibility. Environment may be a factor: MS is more common in temperate climates (1/2000) than in the tropics (1/10,000). It has been linked to the geographic area in which a patient's first 15 years are spent; relocation after age 15 does not alter the risk. Age at onset is typically 20 to 40 years, and women are affected somewhat more often than men.

### 2. Pathology

Plaques of demyelination, with destruction of oligodendroglia and perivascular inflammation, are disseminated throughout the CNS, primarily in the white matter, with a predilection for the lateral and posterior columns (especially in the cervical and dorsal regions), the optic nerves, and periventricular areas. Tracts in the midbrain, pons, and cerebellum are also affected as is gray matter in the cerebrum and spinal cord. Cell bodies and axons are usually preserved, especially in recent lesions. Later, axons may be destroyed, especially in the long tracts, and a fibrous gliosis makes the tracts appear sclerotic. Recent and old lesions may coexist. Chemical changes in lipid and protein constituents of myelin occur in and around the plaques.

### 3. Symptoms and Signs

The disease is characterized by various symptoms and signs of CNS dysfunction, with remissions and recurring exacerbations. The most common presenting symptoms are paresthesias in one or more extremities, in the trunk, or on one side of the face; weakness or clumsiness of a leg or hand; or visual disturbances, *e.g.,* partial blindness and pain in one eye (retrobulbar optic neuritis), dimness of vision, or scotomas. Other common early symptoms are ocular palsy resulting in double vision (diplopia), transient weakness of one or more extremities, slight stiffness or unusual fatigability of a limb, minor gait disturbances, difficulty with bladder control, vertigo, and mild emotional disturbances; all indicate scattered CNS involvement and often occur months or years before the disease is recognized. Excess heat (*e.g.,* warm weather, a hot bath, a fever) may accentuate symptoms and signs.

Mental: Apathy, lack of judgment, or inattention may occur. Emotional lability is common and may suggest an incorrect initial impression of hysteria. Euphoria occurs in some patients; a reactive depression, in others. Sudden weeping or forced laughter (concomitants of pseudobulbar palsy) indicates that corticobulbar pathways of emotional control are affected. Convulsive seizures seldom occur. Severe changes (*e.g.*, mania, dementia) can occur late in the disease. Scanning speech (slow enunciation with a tendency to hesitate at the beginning of a word or syllable) is common in advanced disease. Aphasia is rare.

Cranial nerves: In addition to optic neuritis, one or more of the following ocular signs usually occur at some time: partial optic nerve atrophy with temporal pallor, changes in visual fields (central scotoma or general narrowing of the fields), or transient ophthalmoplegia with diplopia (due to involvement of the brain stem tracts connecting the 3rd, 4th, and 6th nerve nuclei). In optic neuritis, papilledema accompanied by impaired vision can occur, and the affected pupil does not constrict as completely in response to applied light as does the other pupil; however, other pupillary changes, Argyll Robertson pupils, and total blindness are rare. Nystagmus, a common finding, may be due to cerebellar or vestibular nucleus damage. Other evidence of cranial nerve involvement is uncommon and, when present, is usually due to brain stem injury in the area of the cranial nerve nuclei. Deafness is rare, but vertigo is not. Unilateral facial numbness or pain (resembling trigeminal neuralgia) occurs occasionally, as does hemifacial palsy or spasm.

Motor: Deep reflexes (*e.g.,* knee and ankle jerks) are generally increased; Babinski's sign and clonus are often present. Superficial reflexes, particularly upper and lower abdominal, are diminished or absent. Often, the patient complains of unilateral symptoms, but examination elicits signs of bilateral corticospinal tract involvement. Intention tremor due to cerebellar lesions is common, and continued purposeful effort accentuates it. The motion is ataxic: shaky, irregular, tremulous, and ineffective. Static tremor may occur; it is especially obvious when the head is unsupported. Muscular weakness and spasticity from corticospinal damage produce a stiff, imbalanced gait; later, a combination of spasticity and cerebellar ataxia may become totally disabling. Cerebral lesions may result in hemiplegia, sometimes the presenting symptom. Painful flexor spasms in response to sensory stimuli (*e.g.*, bedclothes) may occur in late stages.

One pattern of disease includes acute optic neuritis, sometimes bilateral, with demyelination of the cervical or thoracic spinal cord (optic neuromyelitis), producing visual loss and paraparesis. Charcot's triad (nystagmus, intention tremor, and scanning speech) is a common cerebellar manifestation in advanced disease. Mild dysarthria may result from cerebellar damage, disturbance of cortical control, or injury to the bulbar nuclei.

Sensory: Complete loss of any form of cutaneous sensation is rare, but paresthesias, numbness, and blunting of sensation (*e.g.,* reduced pain or temperature sense, disturbances of vibratory or position sense) may occur and are often localized, *e.g.*, to the hands or legs. Objective changes are fleeting and are often elicited only with thorough testing. A range of painful sensory disturbances (*e.g.*, burning, electrical, or paroxysmal pain) can occur, especially with spinal cord demyelination.

Autonomic: Urinary urgency or hesitancy, partial retention of urine, or slight incontinence and constipation are common when the spinal cord is affected, as are erectile dysfunction in men and genital anesthesia in women. Urinary and fecal incontinence may occur in advanced disease.

### 4. Course

The course is highly varied, unpredictable, and, in most patients, remittent. Life span is probably not shortened except in the most severe cases. At first, months or years of remission may separate episodes, especially when the disease begins with retrobulbar optic neuritis. Remissions can last > 10 years. However, some patients have frequent attacks and are rapidly incapacitated; for a few, particularly for male patients with onset in middle age, the course may be rapidly progressive. Exposure to excess heat from fever or the environment sometimes worsens symptoms.

### 5. Diagnosis

Diagnosis is indirect, by deduction from clinical and laboratory features. Typical cases can usually be diagnosed confidently on clinical grounds. The diagnosis may be suspected after a first attack. Later, a history of remissions and exacerbations and clinical evidence of CNS lesions disseminated in more than one area are highly suggestive. Other possibilities must be considered.

MRI, the most sensitive diagnostic imaging technique, may show plaques. It may also detect treatable nondemyelinating lesions at the junction of the spinal cord and medulla (*e.g.*, subarachnoid cyst, foramen magnum tumors) that occasionally cause a variable and fluctuating spectrum of motor and sensory symptoms, mimicking MS. Gadolinium-contrast enhancement can distinguish areas of active inflammation from older brain plaques. MS lesions may also be visible on contrast-enhanced CT scans; sensitivity may be increased by giving twice the iodine dose and delaying scanning (double-dose delayed CT scan).

CSF is abnormal in the majority of patients. IgG may be > 13%, and lymphocytes and protein content may be slightly increased, but these findings are not pathognomonic. Oligoclonal bands, which indicate IgG synthesis within the blood-brain barrier, may be detected by agarose electrophoresis of CSF in up to 90% of patients with MS, but absence of these bands does not rule out MS. IgG levels correlate with disease severity. Myelin basic protein may be elevated during active demyelination.

Evoked potentials are recorded electrical responses to stimulation of a sensory system. Pattern-shift visual, brain stem auditory, and somatosensory evoked potentials may be abnormally delayed early in the disease, because demyelination slows the conduction of electrical impulses in these sensory pathways.

Immunomodulatory therapy with interieron-β reduces the frequency of relapses in MS and may help delay eventual disability. Glatiramer acetate may have similar benefits for early, mild MS. IV gamma globulins given monthly may help control relapsing MS refractory to conventional therapies. Immunosuppressive drugs (methotrexate, azathioprine, cyclophosphamide, cladribine) for more severe progressive forms are not uniformly beneficial and have significant toxic risks.

### G. Psoriasis

Psoriasis is a common chronic, recurrent disease characterized by dry, well-circumscribed, silvery, scaling papules and plaques of various sizes. Psoriasis varies in severity from one or two lesions to widespread dermatosis, sometimes associated with disabling arthritis or exfoliation. The cause is unknown, but the thick scaling has traditionally been attributed to increased epidermal cell proliferation and concomitant dermal inflammation. The response of psoriasis to the immunosuppressive drug cyclosporine suggests that the primary pathogenetic factor may be immunologic. About 2 to 4% of whites and far fewer blacks are affected. Onset is usually between ages 10 and 40, but no age is exempt. A family history of psoriasis is common. Except for the psychologic stigma of an unsightly skin disease, general health is unaffected unless psoriatic arthritis, erythrodermic psoriasis, or pustular psoriasis develops.

### 1. Symptoms and Signs

Onset is usually gradual. The typical course is one of chronic remissions and recurrences (or occasionally acute exacerbations) that vary in frequency and duration. Factors precipitating psoriatic flares include local trauma (in the Koebner's phenomenon, lesions appear at sites of trauma) and, occasionally, irritation (variants of Koebner's phenomenon), severe sunburn, viremia, allergic drug reactions, topical and systemic drugs (*e.g.*, chloroquine antimalarial therapy, lithium, β-blockers, interferon-α), and withdrawal of systemic corticosteroids. Some patients (especially children) may have psoriatic eruptions after an acute group A β-hemolytic streptococcal URI.

Psoriasis characteristically involves the scalp (including the postauricular regions), the extensor surface of the extremities (particularly elbows and knees), the sacral area, buttocks, and penis. The nails, eyebrows, axillae, umbilicus, or anogenital region may also be affected. Occasionally the disease is generalized.

Typical lesions are sharply demarcated, variously pruritic, ovoid or circinate erythematous papules or plaques covered with overlapping thick silvery micaceous or slightly opalescent shiny scales. Papules sometimes extend and coalesce to produce large plaques in annular and gyrate patterns, but this phenomenon is more common in cutaneous T-cell lymphoma. The lesions heal without scarring, and hair growth is usually unaltered. Nail involvement occurs in 30 to 50% of patients and may clinically resemble a fungal infection, with stippling, pitting, fraying, discoloration or separation of the distal and lateral margins of the nail plate (onycholysis), and thickening, with hyperkeratotic debris under the nail plate.

Psoriatic arthritis often resembles RA and may be equally crippling. Rheumatoid factor is not present in the serum.

Erythrodermic psoriasis (exfoliative psoriatic dermatitis) may be refractory to therapy. The entire cutaneous surface is red and covered with fine scales; typical psoriatic lesions may be obscured or absent. It may lead to general debility and a need for hospitalization.

Pustular psoriasis is characterized by sterile pustules and may be generalized (von Zumbusch type) or localized to the palms and soles (Barber's psoriasis); typical psoriatic lesions may be absent.

### 2. Diagnosis

Psoriasis may be confused with seborrheic dermatitis, squamous cell carcinoma in situ (Bowen's disease, especially when on the trunk), secondary syphilis, dermatophyte infections, cutaneous lupus erythematosus, eczema, lichen planus, pityriasis rosea, or localized dermatitis caused by scratching (lichen simplex chronicus). However, diagnosis by inspection is rarely difficult; *e.g.*, well-defined, dry, heaped-up psoriatic lesions with large silvery scales are usually distinguishable from the diffuse, greasy, yellowish scaling of seborrheic dermatitis.

Although biopsy findings of typical lesions are generally characteristic, atypical lesions have atypical features making biopsy less helpful; some other skin diseases may have psoriasiform histologic features that may make microscopic diagnosis difficult or equivocal.

### H. Other Autoimmune diseases

Other autoimmune diseases applicable to the methods of the present invention include, but are not limited to, alopecia areata, ankylosing spondylitis, antiphospholipid syndrome, autoimmune Addison's disease, autoimmune hemolytic anemia, autoimmune hepatitis, Behcet's disease, bullous pemphigoid, cardiomyopathy, celiac sprue-deimatitis, chronic fatigue immune dysfunction syndrome (CFIDS), chronic inflammatory demyelinating polyneuropathy, Churg-Strauss syndrome, cicatricial pemphigoid, CREST syndrome, cold agglutinin disease, Crohn's disease, discoid lupus, essential mixed cryoglobulinemia, fibromyalgia-fibromyositis, Graves' disease, Guillain-Barré, Hashimoto's thyroiditis, idiopathic pulmonary fibrosis, idiopathic thrombocytopenia purpura (ITP), IgA nephropathy, insulin dependent diabetes, juvenile arthritis, lichen planus, ménière's disease, mixed connective tissue disease, multiple sclerosis, myasthenia gravis, pemphigus vulgaris, pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, Raynaud's phenomenon, Reiter's syndrome, rheumatic fever, rheumatoid arthritis, sarcoidosis, scleroderma, Sjögren's syndrome, stiff-man syndrome, systematic lupus erythematosus, Takayasu arteritis, temporal arteritis/giant cell arteritis, ulcerative colitis, uveitis, vasculitis, vitiligo, and Wegener's granulomatosis. *See generally* THE MERCK MANUAL (Mark H. Beers & Robert Berkow eds., 17th ed. 1999); *see also generally* THE MERCK MANUAL (Mark H. Beers & Robert Berkow eds., 17th ed. 1999), *available at* http://www.merck.com/pubs/mmanual.

### V. Atopic Disease

Atopic diseases is a generic name for allergic diseases which develop in individuals that are very sensitive to extrinsic allergens. Patients having a predisposition to this disease readily exhibit severe hypersensitivities to alimentary antigens and inhalants. The patients also easily experience abnormalities in autonomic nerves, endocrines and immunological systems, and may manifest atopic disease symptoms in response to external challenges such as fever, coldness, humidity, injury, infection, or by intrinsic stress such as a mental strain. Atopic diseases include, but are not limited to, atopic dermatitis, extrinsic bronchial asthma, urticaria, allergic rhinitis, allergic enterogastritis and the like.

The allergic diseases that may be prevented or treated by the therapeutic agent of the present invention include allergic diseases mediated by IgE antibody, such as bronchial asthma, hay fever, angioneurotic edema, urticaria, serous tympanitis, atopic dermatitis, pollinosis, allergic rhinitis, allergic gastroenteritis, food allergy, drug allergy, and the like.

The immunoglobulin E (IgE)-mediated immune reactions (type I allergies) dominate in the form of anaphylaxis, allergic bronchial asthma, allergic rhinitis and conjunctivitis, allergic urticaria, allergic gastroenteritis and atopic dermatitis.

### A. Atopic Dermatitis

Atopic dermatitis is characterized by chronic, pruritic, superficial inflammation of the skin, frequently associated with a personal or family history of allergic disorders (*e.g.*, hay fever, asthma).

### 1. Etiology

Susceptibility is genetic, but the disorder is triggered by various environmental agents and factors. Numerous inhalants and foods produce wheal and flare reactions on scratch or intradermal tests, but these reactions are usually not relevant; elimination does not usually cause remission, except sometimes in young patients. Patients with atopic dermatitis usually have high serum levels of reaginic (IgE) antibodies, peripheral eosinophilia, and high levels of cAMP phosphodiesterase in their WBCs, but the etiologic significance of these findings is unknown.

### 2. Symptoms, Signs, and Course

Atopic dermatitis may begin in the first few months of life, with red, weeping, crusted lesions on the face, scalp, diaper area, and extremities. In older children or adults, it may be more localized and chronic, typically appearing as erythema and lichenification in the antecubital and popliteal fossae and on the eyelids; neck, and wrists. The course is unpredictable. Although the dermatitis often improves by age 3 or 4 yr, exacerbations are common during childhood, adolescence, or adulthood.

Pruritus is constant; subsequent scratching and rubbing lead to an itch-scratch-rashitch cycle. The dermatitis may become generalized (see below). Secondary bacterial infections and regional lymphadenitis are common. Frequent use of proprietary or prescribed drugs exposes the patient to many topical allergens, and contact dermatitis may aggravate and complicate atopic dermatitis, as may the generally dry skin that is common in these patients. Intolerance to primary environmental irritants is common, and emotional stress, ambient temperature or humidity changes, bacterial skin infections, fragrances, fabric softeners, and wool garments commonly cause exacerbations.

### 3. Complications

Patients with long-standing atopic dermatitis may develop cataracts while in their 20s or 30s. Cataracts may be a feature of atopy or may result from extensive systemic and topical corticosteroid use. Herpes simplex may induce a generalized painful vesicular eruption and sometimes a grave febrile illness (eczema herpeticum) in atopic patients.

House dust mites in bedding, upholstered furniture, and carpeting may significantly exacerbate atopic dermatitis.

### 4. Diagnosis

Diagnosis is based on the distribution and duration of lesions and often on a family history of atopic disorders and the presence of lichenification. Because atopic dermatitis is often hard to differentiate from seborrheic dermatitis in infants or from contact dermatitis at any age, the physician should examine the patient several times before making a definitive diagnosis. The physician must be careful not to attribute all subsequent skin problems to an atopic diathesis.

### 5. Treatment

Precipitating agents and complex topical drugs should be avoided if possible. Corticosteroid creams or ointments applied three times daily are most effective. Because topical corticosteroids are expensive, supplemental use of white petrolatum, hydrogenated vegetable oil (as for cooking), or hydrophilic petrolatum (unless the patient is allergic to lanolin) may be advisable. These emollients, applied between corticosteroid applications, also help hydrate the skin, which is important. Prolonged, widespread use of high-potency corticosteroid creams or ointments should be avoided in infants because adrenal suppression may ensue.

Older adults may benefit from treatment with ultraviolet radiation B, psoralen plus high-intensity ultraviolet A (PWA--see under Psoriasis in Ch. 117), or narrow band ultraviolet A without psoralen. Because of its potential long-term side effects, however, PUVA is rarely indicated for children or young adults.

Bathing should be minimized if it exacerbates symptoms; soap should not be used on dermatitic areas because it may be drying and irritating. Oils help lubricate the skin, and corticosteroid or emollient ointments should be applied within 3 min of bathing, before the skin is dried, to enhance effectiveness.

Antihistamines may provide some relief but are often sedating and anticholinergic. Doxepin, a dibenzoxepin tricyclic compound, is a very active antihistamine that also has a useful psychotherapeutic effect in pruritic patients. The starting dose is 25 to 50 mg po at bedtime. Doxepin cream 5% may be applied qid, but percutaneous absorption may cause systemic symptoms. Hydroxyzine hydrochloride 25 mg tid or qid (for children, 2 mg/kg/day in divided doses q 6 h)may also be useful. Diphenhydramine 25 to 50 mg may be given at bedtime, when pruritus is usually worst.

Fingernails should be kept short to minimize excoriations and secondary infections. For secondary infections, an oral penicillinase-resistant penicillin or a cephalosporin qid is advised.

Oral corticosteroids should be considered a last resort but, if given, are best used in 1-to 2-wk courses. Stunted growth, osteoporosis, and other side effects occur with prolonged use of systemic corticosteroids, and rebound exacerbations on stopping therapy are frequent. Altemate-day use of corticosteroids (*e.g.*, for adults, prednisone 20 to 40 mg every other morning) may help reduce side effects. The initial dose should be continued for several weeks, then slowly decreased while the patient starts using topical drugs.

For unusually widespread, recalcitrant, or disabling cases, experimental treatments, such as oral emulsified cyclosporine 1.5 to 2.5 mg/kg bid in adults, have proven useful. Tacrolimus is a topical immunosuppressive ointment without systemic effects. It may be useful in children and adults with severe atopic dermatitis. Newly developed phosphodiesterase-4 inhibitors may become important therapy.

If atopic dermatitis resists home treatment, hospitalization, which provides closer psychologic and dermatologic attention and a change in environment, often accelerates improvement.

### B. Extrinsic Bronchial Asthma

Bronchial asthma is a lung disorder characterized by: (1) recurring episodes of difficult breathing and wheezing on expiration and inspiration due to constriction of the bronchi, (2) airway inflammation with viscous mucoid secretions, and (3) coughing. Bronchial asthma is divided into allergic or extrinsic asthma (immunoglobulin E-mediated), and non-allergic or intrinsic asthma, caused by infections, chemical irritants, emotional stresses, and vigorous exercise. Asthmatics may suffer a combination of the two. The obstruction of airways in asthma is due to the following factors: (1) increased mucus secretion (2) spasm of the smooth muscle of the airways (3) cellular, especially eosinophilic, infiltration of airways walls (4) edema of airways mucosa (5) injury and desquamation of the airways epithelium.

Extrinsic asthma is an allergic condition and may be detected by an increased in of serum IgE (an allergic antibody) in the body. This type of asthma is generally triggered by a respiratory infection (cough, cold, bronchitis, etc.), by exercise (esp. in cold air), by tobacco or by other air pollutants, or by an allergy to a particular food or drug.

### 1. Symptoms

Symptoms of asthmatic attacks vary greatly in frequency and severity. Some are symptom-free, with periodic, mild, brief episodes. Other asthmatics have continual mild coughing and wheezing, interrupted by severe flare-ups of symptoms following exposure to known allergens such as food, molds, pollen, grasses, animal dander, or chemicals and other pollutants. Acute attacks usually begin suddenly with violent outbursts of wheezing, coughing, and shortness of breath. The attack may begin slowly with increasing signs of respiratory distress. Typical symptoms include: sudden, difficult breathing (dyspnea), wheezing, rapid shallow breathing (tachypnea), and a sense of suffocation; coughing, pressure and painless tightness in the chest; there may or may not be a heavy production of thick, clear mucus. More severe symptoms include: inability to speak without gasping, severely constricted neck muscles; rapid pulse; severe anxiety; profuse sweating; bluish lips; mental confusion; exhaustion.

### C. Urticaria

Urticaria is an inflammatory disease characterized by erythematous, itchy edematous and whealing lesions of the skin or mucous membranes. Individual wheals may be as small as 1-2 mm in diameter, but they can reach several centimeters. Acute urticaria has been defined as episodes lasting for less than 12 weeks particularly 6-12 weeks, chronic urticaria has been defined as episodes lasting beyond 12 weeks.

Different types of urticaria are described such as, but not limited to, acute idiopathic, chronic idiopathic, IgE-mediated, pseudo-allergic, serum-sickness, contact, hereditary angioedema, acquired C I inhibitor deficiency and physical as well as urticaria vasculitis.

The onset of urticaria attack may be defined as a new flare up of urticaria in a patient, who had already experienced urticaria. Rash or flare up means that an urticaria lesion is already present. The onset of primary urticaria may be defined as the first urticaria attack during a patient's life or an attack at a time when the patient did not otherwise show any presence of urticaria; in this latter case, no urticaria lesions are present at the time of onset.

### D. Allergic Rhinitis

IgE-mediated rhinitis, characterized by seasonal or perennial sneezing, rhinorrhea, nasal congestion, pruritus, and, often, conjunctivitis and pharyngitis.

### 1. Hay Fever (Pollinosis)

The acute seasonal form of allergic rhinitis. Hay fever is generally induced by wind-borne pollens. The spring type is due to tree pollens (*e.g.*, oak, elm, maple, alder, birch, juniper, olive); the summer type, to grass pollens (*e.g.*, Bermuda, timothy, sweet vernal, orchard, Johnson) and to weed pollens (*e.g.*, Russian thistle, English plantain); and the fall type, to weed pollens (*e.g.*, ragweed). Occasionally, hay fever is caused primarily by airborne fungal spores. Important geographic regional differences occur.

### a. Symptoms and Signs

The nose, roof of the mouth, pharynx, and eyes begin to itch gradually or abruptly after the pollen season begins. Lacrimation, sneezing, and clear, watery nasal discharge accompany or soon follow the pruritus. Frontal headaches and irritability may occur. More rarely, anorexia, depression, and insomnia may occur. The conjunctiva is injected, and the nasal mucous membranes are swollen and bluish red. Coughing and asthmatic wheezing may develop as the season progresses.

### b. Diagnosis

The history indicates the nature of the allergic process and often the pollens responsible. Diagnosis is supported by the physical findings and eosinophils in the nasal secretions. Skin tests are useful to confirm or identify the responsible pollens.

### c. Treatment

Symptoms may be diminished by avoiding the allergen (see above). Oral antihistamines often provide relief; if the usual ones are too sedating, a nonsedating one may be used (see Antihistamines, above). Topical treatment may be used (see below). Sympathomimetics are often given with antihistamines. Phenylpropanolamine, phenylephrine, or pseudoephedrine are available in many antihistamine-decongestant preparations. Because oral sympathomimetics can raise the BP, patients with a tendency toward hypertension should not use them without periodic monitoring.

If oral antihistamines are unsatisfactory, then 4% cromolyn may be given by nasal spray (delivered by a finger-activated pump). The usual dosage is one spray (5.2 mg) tid to qid. It may be more effective to prevent rather than to relieve acute symptoms. Since cromolyn costs more and only relieves nasal symptoms, other drugs are usually tried first. Azelastine, an antihistamine nasal spray, is effective and causes fewer side effects than oral antihistamines.

When nasal symptoms are not relieved by antihistamines, intranasal glucocorticoid spray usually is effective. Two sprays bid to qid are used initially (see Table 148-2). When symptoms have been relieved, dosage is reduced as tolerated. When used as indicated, these drugs have few side effects. Severe intractable symptoms may require a short course of systemic corticosteroids (prednisone 30 mg/day po with gradual reduction in dosage over 1 wk to zero or to 10 mg on alternate days).

Allergen immunotherapy (desensitization) treatment (see above) is advised if the allergen cannot be avoided, drug treatment is poorly tolerated, or systemic glucocorticoids are needed during the season. If the patient is allergic to pollens, therapy should begin soon after the pollen season ends to prepare for the next season.

### 2. Perennial Rhinitis

Nonseasonal rhinitis, which may or may not be allergic, sometimes complicated by sinusitis, nasal polyps, or sensitivity to aspirin and other NSAIDs.

### a. Symptoms, Signs, and Diagnosis

In contrast to hay fever, symptoms of perennial rhinitis vary in severity (often unpredictably) throughout the year. Extranasal symptoms (*e.g.*, conjunctivitis) are uncommon, but chronic nasal obstruction is often prominent and may extend to the eustachian tube. The resultant hearing difficulty is particularly common in children. The diagnosis is supported by a positive history of atopic disease, the characteristic bluish red mucosa, numerous eosinophils in the nasal secretions, and positive skin tests (particularly to house dust mites, cockroaches, animal danders, or fungi). Some patients have complicating sinus infections and nasal polyps.

### b. Differential diagnosis

Some patients with negative skin tests and numerous eosinophils in their nasal secretions suffer from chronic rhinitis, sinusitis, and polyps, called eosinophilic nonallergic rhinitis or nonallergic rhinitis with eosinophilia. These patients are not atopic, but often have sensitivity to aspirin and other NSAIDs; a subset of patients suffer only from chronic rhinitis.

Some patients suffer from vasomotor rhinitis, which is characterized by mild but annoying chronic continuous nasal obstruction or rhinorrhea and no demonstrable allergy, polyps, infection, eosinophilia, or drug sensitivity (see Ch. 86). An additional group of patients suffer rhinitis from the overuse of topical (α-adrenergic) decongestants (rhinitis medicamentosa).

### c. Treatment

Treatment is similar to that for hay fever if specific allergens are identified, except that systemic glucocorticoids, even though effective, should be avoided because of the need for prolonged use. Surgery (antrotomy and irrigation of sinuses, polypectomy, submucous resection) or cryotherapy is sometimes tried after allergies have been controlled or ruled out. No good data exist to prove that surgery is effective for perennial rhinitis per se. Patients with eosinophilic nonallergic rhinitis usually respond best to a topical glucocorticoid. For patients with vasomotor rhinitis, the only treatment is reassurance, antihistamine and oral vasoconstrictor drugs, and advice to avoid topical decongestants, which produce after-congestion and, when used continuously for a week or more, may aggravate or perpetuate chronic rhinitis (rhinitis medicamentosa). Some patients may benefit from frequent use of saline irrigation, topical ipratropium bromide, or nasal sprays.

### 3. Allergic Conjunctivitis

Allergic inflammation of the conjunctiva. Allergic conjunctivitis of an acute or chronic catarrhal form is usually part of a larger allergic syndrome (*e.g*., hay fever), but it may occur alone through direct contact with airborne substances (*e.g*., pollen, fungal spores, dusts, animal danders). (See also Vernal Keratoconjunctivitis in Ch. 95.)

### a. Symptoms, Signs, and Diagnosis

Prominent itching may be accompanied by excessive lacrimation. The conjunctiva is edematous and hyperemic. The cause is often suggested by the patient's history and may be confirmed by skin testing.

### b. Treatment

An identified or suspected causative allergen should be avoided. Frequent use of a bland eyewash (*e.g.,* buffered 0.65% saline) may reduce the irritation. Contact lenses should not be worn. Oral antihistamines are usually helpful. Topical antihistamines are available (antazoline 0.5% or pheniramine 0.3%) but only combined with the vasoconstrictors naphazoline 0.025% to 0.05% or phenylephrine 0.125% as ophthalmic solutions. Long-term use of vasoconstrictors can cause the same rebound phenomena in the eye as occur in the nose. Topical antihistamine or the preservative in the preparation may be sensitizing, and most patients respond equally well or better to an oral antihistamine plus a topical vasoconstrictor alone rather than to the topical combination (see also Seasonal Allergic Conjunctivitis in Ch. 95). Cromolyn (4% ophthalmic solution) may be helpful, especially to prevent the development of symptoms when allergen exposure is anticipated (see Allergic Rhinitis, above). A corticosteroid ophthalmic suspension (*e.g.*, medrysone 1% or fluorometholone 0.1% applied qid) may be used in severe cases as a last resort and in consultation with an ophthalmologist. Intraocular pressure should be checked before and regularly during such treatment, which should be ended as soon as possible.

Without further elaboration, one skilled in the art can, using the preceding description, utilize the present invention to the fullest extent. Various modifications and variations of the described methods of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims. References are fully and specifically incorporated herein by reference.

### VI. Methods for Determining Whether a Subject is Predisposed to a Disease

Predisposition may be based on a number of observations including, but not limited to, the presence of a mutation in a gene, which mutation has been associated with the development of a particular disease. The presence of particular disease markers such as a particular protein, antibody, etc. may also be used to identify and/or confirm that a subject is predisposed to a particular disease. A subject may also be determined to be predisposed to a particular disease based, for example, on a family history of a particular disease, sex, weight, age, diet, and other environmental factors. A subject may be predisposed to a particular disease by virtue of, for example, merely being determined to be at risk of developing such disease, having an increased likelihood of developing such disease, susceptible to developing such disease, or more likely than not to develop a disease, etc., as compared to a "wildtype" or "normal" subject or otherwise, a subject not predisposed to a disease.

Genetic diagnosis can be used to determine the susceptibility of individuals to different ailments. This can allow doctors to prescribe pretreatment using ECP or apoptotic cells that may prevent the onset of a disease, delay the onset of a disease, or reduce the potential severity of a disease, rather than treating the disease after it develops. Moreover, testing is useful because of the awareness it generates in individuals predisposed to a certain disease. First, individuals can alter their lifestyle to reduce the risk of developing the disease. It is well-recognized medically that environmental factors also play an important role in the development of many diseases. Second, knowledge of being susceptible to a disease is likely to motivate individuals to take diagnostic tests more often so that the disease, if developed, can be detected earlier and be treated more effectively.

The predisposition of a subject to a disease, particularly autoimmune and atopic diseases, may be determined, for example, by screening for genetic markers, serological markers, immunological markers, or polymorphisms including, for example, single nucleotide polymorphisms ("SNPs"). *See, e.g.,* U.S. Patent Application No. 60/366,574, entirely expressly incorporated herein by reference, which provides methodologies for annotating SNPs onto candidate genes based on appropriate sequence comparison software or algorithms such as a BLAST search of SNP databases. Other polymorphisms and methodologies for identifying such polymorphisms are known to those of ordinary skill in the art. In addition, a subject's predisposition to a particular disease may be determined by analyzing gene expression profiles and/or protein expression profiles. *See, e.g.,* U.S. Patent Application No. 10/101,501, entirely expressly incorporated herein by reference, which relates to expression profiles, algorithms to generate expression profiles, microarrays comprising nucleic acid sequences representing expression profiles, and methods of using expression profiles and microarrays. The methods and disease markers useful for determining a subject's predisposition to a particular disease are known to those of ordinary skill in the art. The discussion herein is provided by way of example and is not meant to be limiting in any way whatsoever.

### A. Disease Markers

Table 1 provides a list of disease markers including genetic markers, serological markers, etc., that are associated with particular autoimmune and atopic diseases.

**Table 1. Disease Markers Associated with Autoimmune and Atopic Diseases**

| **Disease** | **Marker (genes/antibodies/snp's)** | **Reference** |
|---|---|---|
| Alopecia areata | • Associated genes: HLA, DBQ, DR, IL-1, AIRE mutation, MX1 • MMP-9+ and CD1a+ cells | • McDonagh et al., 27(5) CLIN EXP DERMATOL 405-9 (2002) • Heffler et al., 147(2) BR J DERMATOL 222-9 (2002) |
| Ankylosin spondylitis | • HLA-B27 • TNF-Alpha, IL-10 | • Khan et al., 16(4) BEST PRACT RES CLIN RHEUMATOL 675-90 (2002) • Seiper et al., 61 Suppl 3 ANN RHEUM Dis III8-III18 (2002) |
| Antiphospholipid syndrome | • anticardiolipin (aCL) antibody, lupus anticoagulant (LA) • phospholipid-binding cofactors (ss2GPI) | • Chi 76 Suppl 2 INT J HEMATOL 47-51 (2002) • Rand ANNU REV MED (2002) |
| Autoimmune Addison's Disease | • haplotypes HLA-A1, - B8 and DR3 • enzymes P45oc21, P45osec and P45oc17 | • Martorell et al., 60(7) NETH J MED 269-75 (2002) |
| Autoimmune hemolytic anemia | • Mitogen-stimulated direct antiglobulin test (MS-DAT) • red cell alloantibodies | • Barcellini et al., 71(3) AM J HEMATOL 177-83 (2002) • Haspl 55(4-5) ACTA MED CROATICA 149-52 (2001) |
| Autoimmune hepatitis | • DRB1*0301 and DRB1*0401 • DRbeta71 position of the HLA class II molecule | • Czaja et al., 47(10) DIG DIS SCI 2139-50 (2002) |
| Behcet's disease | • Fas ligand (FasL) expression • CD1 monoclonal antibody | • Wakisaka 129(2) INT ARCH ALLERGY IMMUNOL175-80 (2002 ) • Poulter et al., 78(2)CLIN EXP IMMUNOL 189-95 (1989 ) |
| Bullous pemphigoid | • IL2, IL-4, IL-5, IFN-gamma, TGF-beta | • Giomi et al., 30(2) DERMATOL SCIJ 116-28 (2002) |
| Cardiomyopathy | • betal-adrenergic receptor; inhibitory G-protein G(i); G-protein receptor kinase | • Wallukat et al., 27(7)HERZ 683-90 (2002 ) |
| Celiac sprue-dermatitis | • Endomysial antibodies; tissue-transglutaminase antibodies • HLA DQ2 | • Vogelsang et al., 40(7)Z GASTROENTEROL I-VII(2002) • Holopainen et al., 48(5)GUT 696-701 (2001) |
| Chronic fatigue immune dysfunction syndrome (CFIDS) | • TNF-alpha; IL-6 • IgG1 and IgG3 deficiency | • Mullington et al., 933 ANN N Y ACAD SCI 201-10 (2001) • Patarca 933 ANN N YACAD ScI 185-200 (2001) |
| Chronic inflammatory demylenating polyneuropathy | • HNPP deletion • CXCL9, CXCL10, CCL3 | • Kom-Lubetzki et al., 113(3) AM J MED GENET 275-8 (2002) • Mahad et al., 73(3) J NEUROL NEUROSURG PSYCHIATRY 320-3 (2002) |
| Churg-Strauss syndrome | •eosinophil-derived neurotoxin (EDN); neutrophil elastase (NE); IL-5 | • Drage et al., 47(2) J AM ACAD DERMATOL 209-16 (2002) |
| Cicatrical pemphigoid | • Autoantigens of BPag2 and laminin 5 | • Kirtschig 49(11) HAUTARZT 818-25 (1998) |
| CREST syndrome | • Antinuclear antibodies recognizing chromosomal centromere proteins | •Meyer 153(3) ANN MED INTERNE (PARIS) 183-8 (2002) |
| Cold agglutinin disease | • anti-red blood cell autoantibody | • Havouis et al., 32(4) EUR J IMMUNOL 1147-56 (2002) |
| Crohn's disease | • NOD2 3020InsC Frameshift Mutation • 99mTechnetium-labelled monoclonal anti-granulocyte antibodies | • D'Amato 29(11) J RHEUMATOL 2470-1 (2002) • Bruno et al., 91(10)ACTA PAEDIATR 1050-5(2002) |
| Discoid lupus | • epidermal surface molecules: ICAM-1, HLA-DR and 27E10 | • Kuhn et al., 146(5) BR J DERMATOL 801-9 (2002 ) |
| Essential mixed cryoglobulinemia | • IgG, anti-IgG rheumatoid factor • hepatitis C virus (HCV) infection | • Fabrizi et al., 22(4)SEMIN NEPHROL 309-18 (2002) • Ferri et al., 55(1) J CLIN PATHOL 4-13 (2002) |
| Fibromyalgia-fibromyositis | • | • |
| Grave's disease | • S-100 protein, epithelial membrane antigen (EMA) • (CA) repeats CAR/CAL and RepIN20 occuring in the human SEL1L gene | • Mitselou et al., 22(3)ANTICANCER RES 1777-80 (2002) • Chiaramonte et al., 232(1-2) MOL CELL BIOCHEM 159-61 (2002) |
| Guillian-Barre | • CSF IgG | • McFarlin et al.,307(19-20) N ENG J MED3071183-8, 1246-1250(1982) • Schliep et al., 218 J Neurol 77-96 (1978) |
| Hashimoto's thyroditis | • IL-6 | • Weetman et al., 127(2)J ENDOCRINOL 357-61(1990) |
| Idiopathic pulmonary fibrosis | • truncated isoforms of the p63 gene (deltaN-p63 proteins) • extracellular signal-regulated kinase (ERK), c-jun N-terminal kinase (JNK), and p38 kinase (p38 MAPK) | • Chilosi et al., 82(10)LAB INVEST 1335-45(2002) • Yoshida et al., 198(3J Pathol 388-96 (2002) |
| Idiopathic thrombocytopnic purpura (ITP) | • GPIIb/IIIa and GPIb/IX | • Olsson et al., 107(3-4) THROMB RES 135-9 (2002) |
| IgA nephropathy | • TGF-betal and Smad 2 • IL-6 | • Ruan et al., 31(4)ZHONGHUA BING LI XUEZA ZHI 314-7 (2002) • Harada et al., 92(4)Nephron 824-6 (2002) |
| Insulin dependent diabetes | • oxidized LDL-anti-oxidized LDL complexes | • Atchley et al., 45(11) DIABETOLOGIA 1562-71 (2002) |
| Juvenile arthritis | • three different HLA loci: HLA-A, -DR/DQ and -DP • IL-1 alpha, IL-1Ra, IL-6, IL-10, MIF, IFN1 | • Forre et al., 31(3)SCAND J RHEUMATOL 123-8 (2002) |
| Lichen planus | • CD1 monoclonal antibody | • Poulter et al., 78(2)CLIN EXP IMMUNOL 189-95 (1989) |
| Meniere's disease | • Cl(-) under beta(2)-adrenergic control | • Milhaud et al., 283(6) AM J PHYSIOL CELL PHYSIOL C1752-C1760 (2002) |
| Mixed connective tissue disease | • RNP antibodies | • Tan et al. 25 ARTH RHEUM 1271-1277 (1982) • Sharp 25 ARTH RHEUM767-70 (1982) |
| Multiple Sclerosis | • CSF IgG • IL-17 | • McFarlin et al.,307(19-20) N ENG J MED 3071183-8, 1246-1250 (1982) • Schliep et al., 218 J Neurol 77-96 (1978) • Aggarwal et al., J BIOL CHEM (2002) |
| Myasthenia gravis | • AChR binding antibody radioimmunoassay | • Drachman 505 ANN N Y ACAD SCI 90-105 (1987 ) |
| Pemphigus vulgaris | • IgG autoantibodies against desmoglein3 | • Amagai et al., 51(3)Keio J Med 133-9 (2002) |
| Pernicious anemia | • PAI-2 | • Varro et al., 123(1)GASTROENTEROLOGY 271-80 (2002) |
| Polyarteritis nodosa | • p-ANCA; anti-MPO | • van Bommel et al., 13(6) EUR J INTERN MED392 (2002) |
| Polychondritis | • collagens, matrilin-1 and cartilage oligomeric matrix protein | • Hansson et al., 4(5)ARTHRITIS RES 296-301(2002) |
| Polyglandular syndromes | • adrenal cortex and/or steroid 21-hydroxylase autoantibodies | • Betterle et al., 23(3) ENDOCR REV 327-64 (2002) |
| Polymyalgia rhuematica | • polymorphisms of HLA-DRB1 | • Jacobsen et al., 29(10) J RHEUMATOL 2148-53(2002) |
| Polymyositis and Dermatomyositis | • serum surfactant protein D (SP-D) | • Ihn et al., 41(11)RHEUMATOLOGY (Oxford) 1268-72 (2002) |
| Primary agammaglobulinemia | • lack of secondary follicles and follicular dendritic cells | • Einstein et al., 22(5) J CLIN IMMUNOL 297-305 (2002) |
| Primary biliary cirrhosis | • Intestinal trefoil factor (ITF) • HLA-A*0201-restricted epitope PDC-E2 165 to 174 | • Kimura et al., 36(5) HEPATOLOGY 1227-35 (2002) • Matsumura et al., 36(5)HEPATOLOGY 1125-34(2002) |
| Psoriasis | • 12R-LOX • NK markers and NK-T cell markers • IL-17 | • Schneider et al., 68-69 PROSTAGLANDINS OTHER LIPID MEDIAT 291-301 (2002) • Cameron et al., 294(8)ARCH DERMATOL RES363-9 (2002) • Aggarwal et al., J BIOL CHEM (2002) |
| Raynaud's phenomenon | • IL-1 gene family halotypes | • Hulkkonen et al.,41(10) RHEUMATOLOGY(OXFORD) 1206-8 (2002) |
| Reiter's syndrome | • human leukocyte antigen-B27 | • Lane et al., 65(6) AMFAM PHYSICIAN 1073-80 (2002) |
| Rheumatic fever | • C-reactive protein (CRP) | • Golbasi et al., 4(5) EurJ Heart Fail 593-5 (2002) |
| Rheumatic arthritis | • Anti-cyclic citrullinated peptide antibodies (anti- CCP • IL-17 | • Schellekens et al., 43ARTH AND RHEUM 155-63(2000) • Aggarwal et al., J BIOLCHEM (2002) |
| Sarcoidosis | • *Propionibacterium acnes* and *P. granulosum* • polymorphisms of the MCP-1 and MIP-1A genes | •Yamada et al., 198(4) JPATHOL 541-7 (2002) •Takada et al., 41(10)INTERN MED 813-8 (2002) |
| Scleroderma | • polymorphisms of the SPARC gene | • Zhou et al., 46(11)ARTHRITIS RHEUM 2990-9(2002) |
| Sjogren's syndrome | • SSA (Ro) antibodies • SSB (La) antibodies | • Alexander et al. 9 J RHEUMATOL 239-246 (1982) • Lane et al., 65(6) AMFAM PHYSICIAN 1073-80(2002) |
| Stiff-man syndrome | • Autoantibodies to 65 kDa glutamic acid decarboxylase (GAD65) | •Al-Bukhari et al., 130(I) CLIN EXP IMMUNOL 131-9 (2002) |
| Systematic lupus erythematosus | • Sm antibodies • RNP antibodies • SSA (Ro) antibodies | • Tan et al. 25 ARTH RHEUM 1271-1277 (1982) • Sharp 25 ARTH RHEUM767-70 (1982) • Lane et al., 65(6) AM FAM PHYSICIAN 1073-80 (2002) |
| Takayasu arteritis | • Annexin I and II | • Ohkawara et al., 947 ANN N Y ACAD SCI 390-3 (2001) |
| Temporal arteritis/giant cell arteritis | • metalloproteinases, lipid peroxidation, nitric oxide synthase (NOS)-2 • polymorphisms of HLA- DRB1 | • Borkowski et al., 161(1) AM J PATHOL 115-23 (2002) • Jacobsen et al., 29(10) J RHEUMATOL 2148-53 (2002) |
| Ulcerative colitis | • 99mTechnetium-labelled monoclonal anti-granulocyte antibodies | • Bruno et al., 91(10) ACTA PAEDIATR 1050-5 (2002) |
| Uveitis | • HLA-B27 | • Smith 15(3) OPHTHALMOL CLIN NORTH AM 297-307 (2002) |
| Vasculitis | • SSA (Ro) antibodies • SSB (La) antibodies | • Sharp 25 ARTH RHEUM 767-70 (1982) • Alexander et al. 9 J RHEUMATOL 239-246 (1982) |
| Vitiligo | • hydrogen peroxide (H2O2) in epidermis | • Boisseau-Garsaud et al., 41(10) INT J DERMATOL 640-2 (2002) |
| Wegener's granulomatosis | • antineutrophil cytoplasmic antibodies (ANCA) directed against proteinase 3 (PR3) and myeloperoxidase (MPO) | • Csernok et al., 41(11) Rheumatology (Oxford) 1313-7 (2002) • Lane et al., 65(6) AM FAM PHYSICIAN 1073-80 (2002) |
| Atopic dermatitis | • Biphasic cytokine expression • Macrophage-derived chemokine (MDC, CCL22) • Ki-67 antigen • type 2 cytokines | • Meagher et al., 43(4)AUSTRALAS J DERMATOL 247-54 (2002) • Yamashita et al., 22 (2)CRIT REV IMMUNOL 105-14 (2002) • Sapuntsova et al., 133(5) BULL EXP BIOL MED 488-90 (2002) • Miescher et al., 23(6) MOL ASPECTS MED 413 (2002) |
| Extrinsic bronchial asthma | • Integrins alpha4/betal (VLA4) and ICAM3 • Perforin • Type 2 cytokines | • Popper et al., 440(2)VIRCHOWS ARCH 172-80 (2002) • Arnold et al., 54(10) PNEUMOLOGIE 468-73 (2000) • Miescher et al., 23(6) MOL ASPECTS MED 413 (2002) |
| Urticaria | • Type 2 cytokines • IL-10 | • Miescher et al., 23(6)MOL ASPECTS MED 413 (2002) • Irinyi et al., 82(4) ACTA DERM VENEREOL 249-53 (2002) |
| Allergic rhinitis | • IL-12 • ICAM 1 and LFA 1 • Chemokine receptor expression | • Lu et al., 16(8) LIN CHUANG ER BI YAN HOU KE ZA ZHI 423-5 (2002) • Song et al., 16(8) LIN CHUANG ER BI YAN HOU KE ZA ZHI 387-9 (2002) • Campbell et al., 14(11) INT IMMUNOL 1255-62 (2002) |
| Allergic enterogastritis | • Yuri/nested/22F/R | • Nakamura et al., 73(4) KANSENSHOGAKU ZASSHI 356-60 (1999) |

### B. Protein Expression Profiles

Proteomics is the large-scale study of expression, function and interactions of proteins. Recent advances in the field spawned miniaturized proteomics technologies capable of parallel detection of thousands of different antigens using submicroliter quantities of biological fluids. More particularly, proteomics technologies exist that enable characterization of autoantibody responses.

Autoantibodies have diagnostic utility for several autoimmune diseases. Such diseases include myasthenia gravis (antiacetylcholine receptor antibody), Grave's disease (antithyroid hormone receptor antibody), and SLE (combination of antinuclear antibodies, plus anti-DNA or anti-Sm antibodies). Furthermore, in T-cell-mediated IDDM, the presence of combinations of autoantibodies against at least two islet antigens, including insulin, glutamic acid decarboxylase, and IA-2, are diagnostic for or predictive of future development of IDDM. Pietropaolo et al., 4 CURR. DIR. AUTOIMMUN. 252-282 (2001). The presence of autoantibodies against a single islet antigen has minimal clinical value. The clinical utility of autoantibodies in IDDM suggests that autoantibody profiles may have diagnostic utility for other T-cell-mediated diseases, such as RA and multiple sclerosis.

Intermolecular and intramolecular epitope spreading of the autoreactive B-cell response is associated with progression to overt clinical disease in human and murine SLE and in IDDM. Craft et al., 40 ARTHRITIS RHEUM. 1374-1382 (1997); James et al., 164 IMMUNOL. REV. 185-200 (1998); Pietropaolo et al., 4 CURR. DIR. AUTOIMMUN. 252-282 (2001). Proteomics technologies are ideally suited to monitoring epitope spreading. Epitope spreading of the autoantibody response may represent a common harbinger of more severe and progressive autoimmunity, providing the clinician with valuable prognostic information to guide the use of nonspecific disease-modifying therapies.

Examples of miniaturized proteomics technologies useful for autoantibody profiling include, for example, arrays of addressable beads (LabMAP™ system of Luminex, Inc. (Austin, Texas)); arrays of addressable tags (eTAG™ assay of Aclara, Inc. (Mountain View, California)); arrays of addressable nanoparticles (SurroMed, Inc. (Mountain View, California)) (Nicewarner-Pena et al., 294 SCIENCE 137-141 (2001)); microfluidics; arrays of living cells (Ziauddin et al., 411 NATURE 107-110 (2001); Uetz et al., 403 NATURE 623-627 (2000)); arrays on planar surfaces (Ge et al., 28 NUCLEIC ACIDS RES. e3ii-vii (2000); Meheus et al., 17 CLIN. Exp. RHEUMATOL. 205-214 (1999); Büssow et al., 26 NUCLEIC ACIDS RES. 5007-5008 (1998); Lueking et al., 270 ANAL. BIOCHEM. 103-111 (1999); Walter et al., 3 CURR. OPIN. MICROBIOL. 298-302 (2000); Joos et al., 21 ELECTROPHORESIS 2641-2650 (2000); Robinson et al., 8 NAT MED 295-301 (2002); *Emest Orlando Lawrence Berkely National Laboratory* <http://rana.lbl.gov>; *Stanford University School of Medicine* <http://www.Stanford.edu/group/antigenarrays>; *Stanford University School of Medicine* <http://cmgm.Stanford.edu/pbrown>; Haab et al., 2 GENOME BIOL. research0004 (2000)).

In addition, one group of researchers has developed spotted antigen arrays specifically for the analysis of autoantibody responses. Robinson et al., 8 NAT. MED. 295-301 (2002). More specifically, this group analyzed the autoreactive B-cell response in patients with autoimmune diseases including systemic lupus erythematosus (SLE), scleroderma, and mixed connective tissue disease. One example of a specialized proteome array for specific autoimmune diseases is the connective tissue disease array. The array contains 200 distinct proteins, peptides, nucleic acids, and protein complexes targeted in a host of autoimmune diseases including SLE, polymyositis, limited and diffuse scleroderma, primary biliary sclerosis, and Sjögren's disease. Robinson et al., 8 NAT. MED. 295-301 (2002). Specific antigens include Ro, La, histone proteins, Jo-1, heterogeneous nuclear ribonucleoproteins (hnRNPs), small nuclear ribonucleoproteins, Smith ribonucleoproteins (Sm/RNP), topoisomerase I, centromere protein B, thyroglobulin, thyroid peroxidase, RNA polymerase, cardiolipin, pyruvate dehydrogenase, serine-arginine splicing factors, and DNA.

The synovial proteome array may be used to study autoimmune arthritis involving synovial joints, including rheumatoid arthritis (RA) and its animal models. The synovial proteome array contains 650 candidate RA autoantigens including deiminated fibrin, citrulline-modified filaggrin and fibrinogen peptides, vimentin, the endoplasmic chaperone BiP, glucose-6-phosphate isomerase, hnRNP A2/Bl, collagens and overlapping peptides derived from several of these proteins.

The myelin proteome array contains 500 proteins and peptides derived from the myelin sheath, the target of the autoimmune response in multiple sclerosis and in experimental autoimmune encephalomyelitis (EAE). These myelin antigens include myelin basic protein, proteolipid protein, myelin-associated glycoprotein, myelin oligodendrocytic glycoprotein, golli-myelin basic protein, oligodendrocyte-specific protein, cyclic nucleotide phosphodiesterase and overlapping peptides derived from these proteins. The myelin proteome array may be used to characterize the autoantibody response in EAE serum, multiple sclerosis patient serum and cerebral spinal fluid, and to guide selection of antigen-specific therapies in relapsing EAE. Robinson et al., CLIN. IMMUNOL. (2002) in press*.*

The islet cell proteome array contains glutamic acid decarboxylase, IA-2, insulin and additional candidate autoantigens in insulin-dependent diabetes mellitus (IDDM).

### C. Single Nucleotide Polymorphisms

The identification and analysis of a particular gene or protein, or of a single nucleotide polymorphism (SNP), has generally been accomplished by experiments directed specifically towards that gene or protein, or SNP. With the recent advances, however, in the sequencing of the human genome, the challenge is to decipher the expression, function, and regulation of thousands of genes that can contain intragenic SNPs, which cannot be realistically accomplished by analyzing one gene or protein, or SNP at a time. To address this situation, the data mining methodologies of the present invention have been developed and proven to be a valuable tool.

Information is accumulating about the normal variation among human genomes. During the course of evolution, spontaneous mutations appear in the genomes of organisms. Variations in genomic DNA sequences have been estimated as being created continuously at a rate of about 100 new single base changes per individual. Kondrashow, 175 Theor. BIOL. 583-94 (1995); Crow, 12 EXP. CLIN. IMMUNOGENET. 121-28 (1995). These changes in the progenitor nucleotide sequences can confer an evolutionary advantage that likely increases the frequency of the mutation, an evolutionary disadvantage that likely decreases the frequency of the mutation, or the mutation will be neutral. In many cases, equilibrium is established between the progenitor and mutant sequences so that both are present in the population. The presence of both forms of the sequence results in genetic variation or polymorphism. Over time, a significant number of mutations can accumulate within a population such that considerable polymorphism can exist between individuals within the population.

Numerous types of polymorphisms are known to exist. There are several sources of sequence variation, such as when DNA sequences are either inserted or deleted from the genome, for example, by viral insertion. The presence of repeated sequences in the genome can also cause sequence variation and is variously termed short tandem repeats (STRs), variable number tandem repeats (VNTRs), short sequence repeats (SSRs) or microsatellites. These repeats can be dinucleotide, trinucleotide, tetranucleotide, or pentanucleotide repeats. Polymorphism results from variation in the number of repeated sequences found at a particular locus.

Most commonly, sequence differences between individuals involve differences in single nucleotide positions. SNPs account for approximately 90% of human DNA polymorphism. Collins et al., 8 GENOME RES. 1229-31 (1998). SNPs include single base pair positions in genomic DNA at which different sequence alternatives (alleles) exist in a population. In addition, the least frequent allele generally must occur at a frequency of 1% or greater. DNA sequence variants with a reasonably high population frequency are observed approximately every 1,000 nucleotide across the genome, with estimates as high as 1 SNP per 350 base pairs. Wang et al., 280 SCIENCE 1077-82 (1998); Harding et al., 60 AM. J. HUMAN GENET. 772-89 (1997); Taillon-Miller et al., 8 GENOME RES. 748-54 (1998); Cargill et al., 22 NAT. GENET. 231-38 (1999); and Semple et al., 16 BIOINFORM. Disc. NOTE 735-38 (2000). The frequency of SNPs varies with the type and location of the change. In base substitutions, two-thirds of the substitutions involve the C - T and G - A type. This variation in frequency can be related to 5-methylcytosine deamination reactions that occur frequently, particularly at CpG dinucleotides. Regarding location, SNPs occur at a much higher frequency in non-coding regions than in coding regions. Information on over one million variable sequences is already publicly available via the Internet and more such markers are available from commercial providers of genetic information. Kwok and Gu, 5 MED. TODAY 538-53 (1999).

Several definitions of SNPs exist. *See, e.g.,* Brooks, 235 GENE 177-86 (1999). As used herein, the term "single nucleotide polymorphism" or "SNP" includes all single base variants, thus including nucleotide insertions and deletions in addition to single nucleotide substitutions. There are two types of nucleotide substitutions. A transition is the replacement of one purine by another purine or one pyrimidine by another pyrimidine. A transversion is the replacement of a purine for a pyrimidine, or vice versa.

The inheritance patterns of most common diseases are complex, indicating that the diseases are probably caused by mutations in one or more genes and/or through interactions between genes and environment. Many known human DNA sequence variants are known to be associated with particular diseases or to influence an individual's response to a particular drug. *See, e.g.,* Drysdale et al., 12 PROC. NAT. ACAD. SCI. 10483-84 (2000). Because of the high frequency of SNPs within the genome, there is a greater probability that a SNP will be linked to a genetic locus of interest than other types of genetic markers.

Numerous methods exist for detecting SNPs within a nucleotide sequence. A review of many of these methods can be found in Landegren et al., 8 GENOME RES. 769-76 (1998). For example, a SNP in a genomic sample can be detected by preparing a Reduced Complexity Genome (RCG) from the genomic sample, then analyzing the RCG for the presence or absence of a SNP. *See, e.g.,* WO 00/18960. Multiple SNPs in a population of target polynucleotides in parallel can be detected using, for example, the methods of WO 00/50869. Other SNP detection methods include the methods of U.S. Patent Nos. 6,297,018 and 6,322,980. Furthermore, SNPs can be detected by restriction fragment length polymorphism (RFLP) analysis. *See, e.g.,* U.S. Patent Nos. 5,324,631; 5,645,995. RFLP analysis of SNPs, however, is limited to cases where the SNP either creates or destroys a restriction enzyme cleavage site. SNPs can also be detected by direct sequencing of the nucleotide sequence of interest. In addition, numerous assays based on hybridization have also been developed to detect SNPs and mismatch distinction by polymerases and ligases.

SNPs can be a powerful tool for the detection of individuals whose genetic make-up alters their susceptibility and/or predisposition to certain diseases. Genotyping of such markers therefore can be valuable to characterize patient populations. DNA sequence variants with no known functional consequences can also be useful in association and linking analyses. For example, information may be revealed that can then be used to detect individuals at risk for pathological conditions based on the presence of SNPs.

SNPs can be directly or indirectly associated with disease conditions in humans or animals. In a direct association, the alteration in the genetic code caused by the SNP directly results in the disease condition. Sickle cell anemia and cystic fibrosis are examples of direct SNP association with a disease. In an indirect association, the SNP does not directly cause the disease, but may alter the physiological environment such that there is an increased likelihood that the subject is susceptible to develop the disease as compared to an individual without the SNP. Additionally, SNPs can also be associated with disease conditions, without a direct or an indirect association with the disease. In this case, the SNP may be located in close proximity to the defective gene, usually within 5 centimorgans, such that there is a strong association between the presence of the SNP and the disease state.

Disease-associated SNPs can occur in coding and non-coding regions of the genome. When located in a coding region, a SNP can result in the production of a protein that is non-functional or that has decreased functionality. More frequently, SNPs may occur in non-coding regions. If a SNP occurs in a regulatory region, it can affect expression of the protein. For example, the presence of a SNP in a promoter region can alter the expression of a protein. If the protein is involved in protecting the body against development of a pathological condition, this decreased expression can make the individual more susceptible to the condition.

In association studies, the frequency of variants of individual genetic markers are compared between healthy persons and subject populations, anticipating that an observed difference in frequency can be the direct effect of the sequence difference. Also, co-inheritance with nearby unknown genetic variants can have such an effect. Associated markers with no direct effect on disease are referred to as being in linkage disequilibrium with the disease-related changes. Chapman and Thompson, 42 ADV. GENET. 413-37 (2001). These variants may, therefore, provide a guide to the gene that is directly involved in the disease. If the DNA sequence is derived from an individual in families where the particular disease is known to segregate, then the location of the disease-associated genetic changes among the chromosomes can be pinpointed by genetic linkage analysis, using the same types of genetic markers. This methodology has proven valuable for defining the nature of conditions primarily influenced by single or a limited number of genes. *See, e.g.,* Alizadeh et al., 403 NATURE 503-11 (2000).

SNPs are well-suited for identifying genotypes that predispose an individual to develop a disease condition for several reasons. First, SNPs are the most common polymorphisms present in the genome, and are frequently located in or near any locus of interest. Because SNPs located in genes can be expected to directly affect protein structure or expression levels, they not only serve as markers but also as candidates for gene therapy treatments to cure or prevent a disease. SNPs also show greater genetic stability than repeated sequences and thus are less likely to undergo changes that would complicate diagnosis.

In particular diseases, single or small sets of genes have been identified that are typically altered by mutations. The identification of such disease-genes and their associated SNPs provides insights into the causes of common diseases and promotes the development of highly specific diagnostic and therapeutic products. Identifying and characterizing candidate genes and SNPs is critical for defining disease pathways, disease stages, drug effect pathways, and drug metabolic pathways. Sequence variation, as it relates to drug response, can aid in predicting the safety, toxicity, and/or efficacy of drugs. Along these lines, correlating SNPs with drug effects, therapy, and clinical outcome can significantly improve productivity and increase the efficiency of the development or improvement of drugs. Besides advancing drug development, SNPs can further facilitate developments in and improvements of methods and products, such as gene and antisense therapies, molecular diagnostics for predicting drug responses, and molecular diagnostics for selecting drug dosing regimens based upon genotype.

### EXAMPLES

The following examples describe clinical trials that examine the efficacy of using ECP to pretreat patients prior to cardiac transplantation and bone marrow transplantation. These examples, however, do not limit the methods of the present invention. Rather, one skilled in the art may apply the methodologies described to other types of transplantations or to pretreat individuals predisposed to disease.

### Example 1. Evaluation of Extracorporeal Photopheresis with UVADEX® in Conjunction with Center Standard Conditioning Regimen against Center Standard Conditioning Regimen Alone

A randomized, double-blind, controlled study is being performed at 8-12 centers to compare ECP with UVADEX® in conjunction with center standard conditioning regimen to center standard conditioning regimen alone in order to better prevent GVHD in patients about to undergo an allogeneic bone marrow or peripheral blood stem cell transplant. The study is currently in Clinical Phase II.

The current study further assesses the preventive effect of pre-transplant photopheresis on GVHD. In particular, the study seeks to assess the impact of ECP with UVADEX® in conjunction with center standard conditioning regimen as compared to center standard conditioning regimen alone, on the incidence of acute and chronic GVHD in patients about to undergo allogeneic sibling or unrelated transplant for treatment of hematologic malignancies.

### A. Study Design and Sample Size

This trial is a randomized, multi-center, parallel group, controlled study to determine the incidence of acute GVHD in two groups of patients about to undergo allogeneic bone marrow transplant, with one group randomized to receive photopheresis immediately prior to transplantation. Based upon a prior study using a pentostatin-based regimen with photopheresis, the expected incidence of acute GVHD in the photopheresis arm is 10%, while the expected incidence in the control arm is 40%. Patients will be stratified into two groups based on donor match: either a full match (6/6-related donor) or matched unrelated donor-5/6 related donor. It is expected that 40% of patients will fall in the matched donor group but to allow flexibility sites will not be forced to enroll at that rate. Rates of acute GVHD likely differ between the two strata, but as that difference is unknown, the sample-size calculation here assumes them to be the same. Also assuming a 15% peri-transplant mortality rate, a sample size of 83 patients (72 evaluable patients) per arm will be required. This is based on an alpha=0.05 and a power of 80% in two-sided testing using Cochran-Mantel-Haenszel (CMH) test of the odds-ratio. It should be noted that if the true rates of treated and untreated were 10% and 35% respectively, then the CMH test would have greater than 90% power (90% power requires only 65 evaluable patients per treatment arm). However, if the true rates of acute GVHD were 15% and 30%, then 134 evaluable patients per group would be required to achieve 80% power (72 evaluable patients per treatment arm achieves only 49% power in this case). The number of patients in this study, therefore will be 166, or 83 per arm.

### B. Selection of Patients

Patients are eligible if they have a diagnosis of hematologic malignancy for which a treatment option would be an allogeneic 6/6 or 5/6 matched sibling or matched unrelated donor. Patients may be candidates for a standard allogeneic bone marrow or peripheral blood stem cell transplant. Patients must have a suitable HLA-matched sibling related bone marrow donor or a compatible matched unrelated bone marrow donor. Additionally, patients must be physically and psychologically capable of undergoing bone marrow transplantation and its attendant period of strict isolation. Patients must also test negative for antibodies to HIV and present no evidence of any active ongoing infection. Finally, patients or their guardians must sign a statement of informed consent. Patients must be excluded if they show any hypersensitivity or allergy to psoralen (methoxsalen) or to both heparin and citrate products. Patients also must be excluded if their treatment requires donor lymphocyte infusion up to day 100.

### C. Study Treatment

The study treatment will involve ECP including a UVADEX® 20 mcg/mL treatment. The treatment will include two ECP treatments immediately prior to transplantation. The dose of UVADEX® used to inoculate these cells will be calculated based on the treatment volume collected during the plasma/buffy coat collection process, using the following formula: treatment volume (as displayed on the UVAR® XTS screen) in mL * 0.017 = Amount of UVADEX® (in mL) required for administration into the re-circulation bag.

After the cells are inoculated with UVADEX®, the buffy coat/plasma suspension is irradiated with ultraviolet-A light and then re-infused back into the patient. Patients will participate for X days prior to bone marrow transplantation + 365 days. The overall duration of the study is 18 months. Patients who are randomized to the photopheresis arm of the study will receive two photopheresis treatments on two consecutive days preceding transplantation. This may be performed on an outpatient basis. Patients who are randomized to the no photopheresis arm of the study will receive two sham treatments on two consecutive days preceding transplantation. This may also be performed on an outpatient basis.

### D. The Data Endpoints

The primary endpoint data that is sought is the incidence of acute GVHD in the first 100 days after transplantation. Other secondary data endpoints that will be obtained will include the following: incidence of chronic GVHD during 365 days after transplantation, time to engraftment, primary disease relapse, safety and tolerability of photopheresis, survival up to day 365 after transplantation

### E. Analysis of the Data and Results

Rates of acute GVHD will be analyzed between the two treatment groups using the CMH statistic; a 95% two-sided confidence interval on the overall odds-ratio will be formed, along with the Breslow-Day test for homogeneity of the odds-ratios across the two donor strata (full match versus matched unrelated and 5/6 related). Rates of acute GVHD that occur in each center in the two treatment groups and strata will be displayed. Rates of chronic GVHD will be analyzed in the same way for the secondary endpoints as for the primary endpoint. Time to engraftment will be analyzed via a Kaplan-Meier approach. Number and percent of patients with disease relapse (as well as other adverse events) will be summarized and displayed. The primary endpoint analysis will have alpha protected at the 0.05 level. Nominal p values will be used for secondary analysis with no adjustments for multiple comparisons.

A statistically significant decrease in the rate of incidence of GVHD is preferably observed in the treated subjects upon completion of the study over the known incidence of GVHD in nontreated subjects. Moreover, the incidence of GVHD in ECP and standard conditioning regimen treated subjects is preferably observed to be statistically equal to or less than subjects treated with the standard conditioning regimen alone. The study may also look at the progression of organ rejection, or the prevention or delay of organ rejection, or the reduction of the severity of rejection. The differences between incidence of rejection or the progression of the rejection, or the prevention or delay of organ or tissue rejection, or the reduction of the severity of rejection, between the treated subjects and controls are preferably statistically significant and/or observable by clinical or other tests or evaluations. Therefore, the study confirms that ECP treatment of a subject prior to organ transplantation is effective prophylacticly, for example, in preventing, reducing the severity of, or delaying or preventing the onset of organ rejection.

### Example 2: A Clinical Trial Examining the Ability of ECP to Prevent or Reduce Organ Transplant Rejection in Subjects About to Undergo Cardiac Transplantation.

A randomized, double blind, controlled study is being performed at 12 clinical sites to assess the effect of pre-transplant photopheresis on transplant rejection. In particular, the study seeks to assess the impact of ECP with UVADEX® as compared to ECP with UVADEX® in conjunction with center standard conditioning regimen and as compared to center standard conditioning regimen alone, on the incidence of rejection in patients about to undergo allogeneic sibling or unrelated cardiac transplantation.

### A. Patients

A total of 60 eligible adult recipient of primary cardiac transplants at 12 clinical sites are randomly assigned to receive ECP alone (17 patients), standard triple-drug immunosuppressive therapy (cyclosporine, asathioprine, and prednsone; 23 patients) plus ECP or standard triple-drug therapy (20 patients). Patients are stratified and the groups balanced according to age and sex. To be eligible, patients are required to be living within a reasonable commuting distance of the transplantation center and to have adequate peripheral venous access. The study design is approved by all relevant institutional review boards.

### B. Study Design and Sample Size

Patients randomly assigned to receive ECP and ECP plus standard triple-drug therapy are treated with ECP on two consecutive days, one week prior to transplantation. These patients receive treatment by means of the UVAR® XTS Photopheresis System (Therakos, Inc., Exton, PA). Briefly, blood is removed from the patient with a 16-guage antucubital angiocatheter (or an 8-French internal jugular cenous bioptome sheath if access to a peripheral vein can not be obtained) and passed through the device's centrifuge. During the discontinuous leukaphoretic processing, a total of approximately 240 ml of buffy coat and 300 ml of plasma is collected and mixed. The final buffy coat preparation contains approximately 25 to 50 percent of the patient's total peripheral-blood mononuclear cell compartment, with a hematocrit of approximately 5 percent. Two hundred micrograms of liquid methoxsalen (UVADEX®) is injected into the bag of buffy coat. The buffy coat then passes as a 1 mm-thick film through a cassette, where it undergoes a 180-minute exposure to ultraviolet A light, yielding an average exposure of 1 to 2 per square centimeter of leukocyte surface area. After exposure of the cells to the ultraviolet light, the buffy coat is then re-infused into the patient. Each complete procedure lasts approximately four hours.

After transplantation, monitoring is standardized during the six-month period following transplantation for all the patients in the three groups. Routine laboratory studies and measurements of cyclosporine levels in the blood are performed according to a standardized schedule. Endomyocardial biopsies are obtained according to the following schedule: weekly during the first postoperative month; every two weeks during months 2 and 3; and every four weeks during months 4-6, for a total of 11 biopsies.

The primary end point of the study is the number and frequency of acute rejection episodes as defined histologically in a blinded manner at the central pathology laboratory. Secondary end points include the incidence of clinically treated infections, detection of plasma cytomegalovirus (CMV) DNA by polymerase chain reaction (PCR) assay performed by investigators at a central reference laboratory who are unaware of the patient's treatment assignments, and survival without the need for a second transplantation. The study end point are monitored and the patients followed for six months after transplantation; safety and survival follow-up is continued for an additional six months, during which time management at each center reverts to non-standardized institutional protocols.

Two of the groups receive triple-drug immunosuppressive therapy (cyclosporine, azathioprine, and prednisone). Prophylactic use of monoclonal or polyclonal antibodies is not permitted. All the institutions target the rough levels of cyclosporine in whole blood to achieve the equivalent of 250 to 350 ng per milliliter by fluorescence polarization immunoassay (TDX, Abbott, Abbott Park, Ill). Azathioprine treatment begins with a single preoperative dose of 4 mg/kg of body weight, followed by postoperative administration of 0 to 5 mg/kg per day, adjusted to achieve a white-cell count of 4000 to 8000 per cubic milliliter. Corticosteroids were administered beginning with a 500-to-1000 mg dose of intravenous methylpredisolone preoperatively or intraoperatively. Tapered administration of prednisone is then initiated, beginning at 1 mg per kilogram per day, with the goal of reaching a dose of 0.1 mg/kg per day by the fifth month after transplantation.

All the patients receive standardized prophylaxis against infection. For protection against candida infection, patients receive oral mycostatin for the first three months after transplantation. For prevention of *Pneumocystis carinii* pneumonia, patients receive 160 mg of trimethoprim in combination with 800 mg of sulfamethoxazole three times per week, starting in the second week after surgery and continuing throughout the study period. For prophylaxis against CMV infection, all the recipients of CMV-positive organs, whether the recipient was CMV-positive or CMV-negative, receive 10 mg of intravenous ganciclovir per kilogram per day for the first 10 to 14 days after transplantation.

### C. Detection and Treatment of Rejection

Over the six-month period, 11 transvenous endomyocardial biopsies are performed according to a standardized schedule, as outlined above. All biopsy specimens are evaluated by a single pathologist in a blinded manner at the central pathology laboratory according to the standards published by the International Society for Heart and Lung Transplantation. Specific standardized treatment regimens based on the severity of rejection (evaluated in a blinded manner within 24 hours by a pathologist at the local institution) are used. All the patients with grade 3A, 3B, or 4 biopsy specimens are treated. Patients with a grade 2 biopsy specimen in month 1 or two successive grade 2 specimens in months 2 through 6 are treated. Patients with three successive grade 1B specimens are treated. A prolonged episode of rejection (as ascertained by two or more consecutive abnormal specimens) is counted as a single rejection episode if the biopsy grade remained higher than 0 or 1A.

### D. PCR Assay for CMV DNA

For all the patients, serial blood samples (taken every two weeks from weeks 2 to 12 and monthly from months 4 through 6, for a total of nine samples) are analyzed by PCR amplification of plasma CMV DNA. The qualitative PCR procedure used has been described by Wolf and Spector. Plasma samples are maintained at -20 degrees Celsius until processed in a blinded manner at the central laboratory. Ten microliters of plasma diluted 1:10 is passed through three freeze-thaw cycles, dissolved in PCR buffer, and incubated with proteinase K at 60 degrees Celsius for one hour. Samples are then heated at 95 degrees Celsius for 10 minutes, centrifuged at 12000 x g for five minutes, and directly amplified by PCR. For amplification, two sets of primer pairs are constructed for the EcoRI fragment D region of human CMV strain AD169. The template is combined with the appropriate primer pair (50 pmol of each), deoxyribonucleoside triphospates (200 micromolar each; Pharmacia SKB, Piscataway, N.J), and Taq polymerase (2.5 U; Perkin-Elmer Cetus Instruments, Norwalk, Conn.) in a total volume of 100 microliters of PCR buffer and then amplified by 35 cycles of denaturation, primer annealing, and chain extension. After amplification, 10 microliters of the product was denatured at 94 degrees Celsius and immediately hybridized at 55 degrees Celsius with a P32 end-labeled probe. The hybrid heteroduplexes are then resolved by electrophoresis on 6% polyacrylamide gels, and autoradiographs were obtained.

The sensitivity of the procedure is considered to be 6000 replicate copies of the viral DNA. All specimens are tested at least twice in separate PCR procedures, and buffer controls and control samples are run with each reaction. All the positive and negative controls are required to demonstrate the appropriate signal before any result is considered for analysis

### E. Statistical Analysis and Results

Demographic factors and randomization strata (sex and age) between treatment groups are compared using Fisher's exact test. The frequency of rejection episodes in each of the three treatments groups is analyzed by means of a chi-square test for trend, with one degree of freedom. A collapsed version of this analysis compares the number of patients who have one rejection episode or none with the number who had two or more, and other dichotomous variables, with the use of Fisher's exact test. Survival estimates in the form of several functions are generated by the Kaplan-Meier product limit method. The survival of the patients is analyzed with the log-rank test for comparison of the survival curves. Comparison of the infection rates in the three groups is performed with either a Fisher's exact test or a chi-square test for trend, with one degree of freedom. For the detection of CMV DNA by PCR, the percentage of time a patient has a positive test result is calculated. A Wilcoxon rank-sum test is used to compare treatment groups. All P values are two-tailed.

A statistically significant decrease in the rate of incidence of transplant rejection is preferably observed in the treated subjects upon completion of the study over the known incidence of rejection in nontreated subjects. Moreover, the incidence of rejection in ECP treated subjects is preferably observed to be statistically equal to or less than subjects treated with ECP and the center standard conditioning regimen. Further, the incidence of rejection in ECP and standard conditioning regimen treated subjects is preferably observed to be statistically equal or less than the standard conditioning regimen treated subjects alone. The study may also look at the progression of organ rejection, or the prevention or delay of organ rejection, or the reduction of the severity of rejection. The differences between incidence of rejection or the progression of the rejection, or the prevention or delay of organ or tissue rejection, or the reduction of the severity of rejection, between the treated subjects and controls are preferably statistically significant and or observable by clinical or other tests or evaluations. Therefore, the study confirms that ECP treatment of a subject prior to organ transplantation is effective prophylacticly, for example, in preventing, reducing the severity of, or delaying or preventing the onset of organ rejection.

### Example 3: Administration of ECP to Subjects Predisposed to Rheumatoid Arthritis

### A. Patients

Sixty patients are selected to participate in a randomized, double blind, controlled study. Patients are stratified and the groups balanced according to age and sex. To be eligible, patients are determined to be predisposed to rheumatoid arthritis by methods described herein and otherwise known to those of ordinary skill in the art. In addition, patients are required to be living within a reasonable commuting distance of the treatment center and to have adequate peripheral venous access. All relevant institutional review boards approve the study design.

### B. Study Design and Sample Size

Blood samples are taken from all sixty patients prior to treatment, for example, once a month for 6 months, and analyzed for several factors including white blood cell count, erythrocyte sedimentation rate, rheumatoid factor and C-reactive protein, using standard test procedures. The erythrocyte sedimentation rate and C-reactive protein are elevated in most inflammatory conditions including rheumatoid arthritis, and Rheumatoid Factor is elevated in most cases of rheumatoid arthritis as well as in some cases of certain other autoimmune diseases.

### C. ECP Treatment

ECP patients receive treatment by means of the UVAR® XTS Photopheresis System (Therakos, Inc., Exton, PA). Briefly, blood is removed from the patient with a 16-guage antucubital angiocatheter (or an 8-French internal jugular cenous bioptome sheath if access to a peripheral vein can not be obtained) and passed through the device's centrifuge. During the discontinuous leukaphoretic processing, a total of approximately 240 ml of buffy coat and 300 ml of plasma is collected and mixed. The final buffy coat preparation contains approximately 25 to 50 percent of the patient's total peripheral-blood mononuclear cell compartment, with a hematocrit of approximately 5 percent. Two hundred micrograms of liquid methoxsalen (UVADEX®) is injected into the bag of buffy coat. The buffy coat then passes as a 1 mm-thick film through a cassette, where it undergoes a 180-minute exposure to ultraviolet A light, yielding an average exposure of 1 to 2 per square centimeter of leukocyte surface area. After exposure of the cells to the ultraviolet light, the buffy coat is then re-infused into the patient. Each complete procedure lasts approximately four hours.

Patients receiving ECP are treated according to a schedule as described herein and approved by the trial investigators. For example, ECP patients may receive treatment twice a month for six months. Post treatment measurements are conducted once a month for 6 months after the final treatment of each patient. Blood samples are taken and analyzed for white blood cell count, erythrocyte sedimentation rate, rheumatoid factor and C-reactive protein, using standard test procedures.

### D. Statistical Analysis and Results

Demographic factors and randomization strata (sex and age) between treatment groups are compared using an appropriate statistical test, for example, Fisher's exact test. The frequency of decreased white blood cell count, erythrocyte sedimentation rate, rheumatoid factor and C-reactive protein in each of the treatments groups is analyzed by means of a chi-square test for trend, with one degree of freedom. A statistically significant decrease in white blood cell count, erythrocyte sedimentation rate, rheumatoid factor and C-reactive protein is preferably observed in the subjects treated with apoptotic cells upon completion of the study versus untreated subjects.

### Example 4: Pre-Transplant Administration of an Effective Amount of Apoptotic Cells to Subjects Undergoing a Cardiac Transplant

A randomized, double blind, controlled study is being performed at 12 clinical sites to assess the effect of pre-transplant treatment with apoptotic cells on transplant rejection. In particular, the study seeks to assess the impact of treatment with apoptotic cells in conjunction with center standard conditioning regimen and as compared to center standard conditioning regimen alone, on the incidence of rejection in patients undergoing cardiac transplantation.

### A. Patients

A total of 60 eligible adult recipient of primary cardiac transplants at 12 clinical sites are randomly assigned to receive treatment with apoptotic cells alone (17 patients), standard triple-drug immunosuppressive therapy (cyclosporine, asathioprine, and prednsone; 23 patients) plus treatment with apoptotic cells or standard triple-drug therapy (20 patients). Patients are stratified and the groups balanced according to age and sex. To be eligible, patients are required to be living within a reasonable commuting distance of the transplantation center and to have adequate peripheral venous access. The study design is approved by all relevant institutional review boards.

### B. Study Design and Sample Size

Apoptotic cells are prepared from an appropriate mammalian cell line, for example, Jurkat cells (ATCC No.TIB-152). The cells are treated with 50 mM sodium butyrate in RPMI medium, at confluency for one day, and then the sodium butyrate medium is changed. To increase the number of apoptotic cells and bodies, the cells can additionally be irradiated with UV-Iight (*e.g.* 75 mj). Supernatant containing floating cells is removed 24 hours following irradiation. Apoptotic bodies are quantitated by centrifuging the supernatant (1200 rpm, 5 minutes), aspirating the supernatant, washing the resulting cell pellet with PBS and centrifuging again, as above. The pellet containing the apoptotic cells is re-suspended in PBS. The cells stained for quantitation are re-suspended in 1x binding buffer at a concentration of 1x10⁶ cells/ml. About 100ml of the cells are transferred to a 5 ml tube, and 10ml of fluorescein-conjugated annexin V and 10ml propidium iodide reagent are added. The cells are gently vortexed and the cell mixture is incubated for 15 minutes at 25°C in the dark. Following the incubation, 400ml of 1x binding buffer is added to each tube. The sample is analyzed on a flow cytometer over one hour.

Patients randomly assigned to receive treatment with apoptotic cells, and treatment with apoptotic cells plus standard triple-drug therapy, are treated with apoptotic cells on two consecutive days, one week prior to transplantation. These patients receive treatment by means preferred in the present invention, where each treatment unit dosage composition comprises, for example, a liquid suspension of cellular material containing from about 10,000 to about 10,000,000 apoptotic cells per kilogram of patient body weight.

After transplantation, monitoring is standardized during the six-month period following transplantation for all the patients in the three groups. Routine laboratory studies and measurements of cyclosporine levels in the blood are performed according to a standardized schedule. Endomyocardial biopsies are obtained according to the following schedule: weekly during the first postoperative month; every two weeks during months 2 and 3; and every four weeks during months 4-6, for a total of 11 biopsies.

The primary end point of the study is the number and frequency of acute rejection episodes as defined histologically in a blinded manner at the central pathology laboratory. Secondary end points include the incidence of clinically treated infections, detection of plasma cytomegalovirus (CMV) DNA by polymerase chain reaction (PCR) assay performed by investigators at a central reference laboratory who are unaware of the patient's treatment assignments, and survival without the need for a second transplantation. The study end point are monitored and the patients followed for six months after transplantation; safety and survival follow-up is continued for an additional six months, during which time management at each center reverts to non-standardized institutional protocols.

Two of the groups receive triple-drug immunosuppressive therapy (cyclosporine, azathioprine, and prednisone). Prophylactic use of monoclonal or polyclonal antibodies is not permitted. All the institutions target the rough levels of cyclosporine in whole blood to achieve the equivalent of 250 to 350 ng per milliliter by fluorescence polarization immunoassay (TDX, Abbott, Abbott Park, Ill). Azathioprine treatment begins with a single preoperative dose of 4 mg/kg of body weight, followed by postoperative administration of 0 to 5 mg/kg per day, adjusted to achieve a white-cell count of 4000 to 8000 per cubic milliliter. Corticosteroids were administered beginning with a 500-to-1000 mg dose of intravenous methylpredisolone preoperatively or intra-operatively. Tapered administration of prednisone is then initiated, beginning at 1 mg per kilogram per day, with the goal of reaching a dose of 0.1 mg/kg per day by the fifth month after transplantation.

All the patients receive standardized prophylaxis against infection. For protection against candida infection, patients receive oral mycostatin for the first three months after transplantation. For prevention of *Pneumocystis carinii* pneumonia, patients receive 160 mg of trimethoprim in combination with 800 mg of sulfamethoxazole three times per week, starting in the second week after surgery and continuing throughout the study period. For prophylaxis against CMV infection, all the recipients of CMV-positive organs, whether the recipient was CMV-positive or CMV-negative, receive 10 mg of intravenous ganciclovir per kilogram per day for the first 10 to 14 days after transplantation.

### C. Detection and Treatment of Rejection

Over the six-month period, 11 transvenous endomyocardial biopsies are performed according to a standardized schedule, as outlined above. All biopsy specimens are evaluated by a single pathologist in a blinded manner at the central pathology laboratory according to the standards published by the International Society for Heart and Lung Transplantation. Specific standardized treatment regimens based on the severity of rejection (evaluated in a blinded manner within 24 hours by a pathologist at the local institution) are used. All the patients with grade 3A, 3B, or 4 biopsy specimens are treated. Patients with a grade 2 biopsy specimen in month 1 or two successive grade 2 specimens in months 2 through 6 are treated. Patients with three successive grade 1B specimens are treated. A prolonged episode of rejection (as ascertained by two or more consecutive abnormal specimens) is counted as a single rejection episode if the biopsy grade remained higher than 0 or 1A.

### D. PCR Assay for CMV DNA

For all the patients, serial blood samples (taken every two weeks from weeks 2 to 12 and monthly from months 4 through 6, for a total of nine samples) are analyzed by PCR amplification of plasma CMV DNA. The qualitative PCR procedure used has been described by Wolf and Spector. Plasma samples are maintained at -20 degrees Celsius until processed in a blinded manner at the central laboratory. Ten microliters of plasma diluted 1:10 is passed through three freeze-thaw cycles, dissolved in PCR buffer, and incubated with proteinase K at 60 degrees Celsius for one hour. Samples are then heated at 95 degrees Celsius for 10 minutes, centrifuged at 12000 x g for five minutes, and directly amplified by PCR. For amplification, two sets of primer pairs are constructed for the EcoRI fragment D region of human CMV strain AD 169. The template is combined with the appropriate primer pair (50 pmol of each), deoxyribonucleoside triphospates (200 micromolar each; Pharmacia SKB, Piscataway, N.J), and Taq polymerase (2.5 U; Perkin-Elmer Cetus Instruments, Norwalk, Conn.) in a total volume of 100 microliters of PCR buffer and then amplified by 35 cycles of denaturation, primer annealing, and chain extension. After amplification, 10 microliters of the product was denatured at 94 degrees Celsius and immediately hybridized at 55 degrees Celsius with a P32 end-labeled probe. The hybrid heteroduplexes are then resolved by electrophoresis on 6% polyacrylamide gels, and autoradiographs were obtained.

The sensitivity of the procedure is considered to be 6000 replicate copies of the viral DNA. All specimens are tested at least twice in separate PCR procedures, and buffer controls and control samples are run with each reaction. All the positive and negative controls are required to demonstrate the appropriate signal before any result is considered for analysis

### E. Statistical Analysis and Results

Demographic factors and randomization strata (sex and age) between treatment groups are compared using Fisher's exact test. The frequency of rejection episodes in each of the three treatments groups is analyzed by means of a chi-square test for trend, with one degree of freedom. A collapsed version of this analysis compares the number of patients who have one rejection episode or none with the number who had two or more, and other dichotomous variables, with the use of Fisher's exact test. Survival estimates in the form of several functions are generated by the Kaplan-Meier product limit method. The survival of the patients is analyzed with the log-rank test for comparison of the survival curves. Comparison of the infection rates in the three groups is performed with either a Fisher's exact test or a chi-square test for trend, with one degree of freedom. For the detection of CMV DNA by PCR, the percentage of time a patient has a positive test result is calculated. A Wilcoxon rank-sum test is used to compare treatment groups. All P values are two-tailed.

A statistically significant decrease in the rate of incidence of transplant rejection is preferably observed in the treated subjects upon completion of the study over the known incidence of rejection in non-treated subjects. Moreover, the incidence of rejection in subjects treated with apoptotic cells is preferably observed to be statistically equal to or less than subjects treated with apoptotic cells and the center standard conditioning regimen. Further, the incidence of rejection in subjects treated with apoptotic cells and standard conditioning regimen is preferably observed to be statistically equal or less than the standard conditioning regimen treated subjects alone. The study may also look at the progression of organ rejection, or the prevention or delay of organ rejection, or the reduction of the severity of rejection. The differences between incidence of rejection or the progression of the rejection, or the prevention or delay of organ or tissue rejection, or the reduction of the severity of rejection, between the treated subjects and controls are preferably statistically significant and or observable by clinical or other tests or evaluations. Therefore, the study confirms that treatment of a subject with apoptotic cells prior to organ transplantation is effective prophylacticly, for example, in preventing, reducing the severity of, or delaying or preventing the onset of organ rejection.

### Example 5: Administration of an Effective Amount of Apoptotic Cells to Subjects Predisposed to Rheumatoid Arthritis

### A. Patients

Sixty patients are selected to participate in a randomized, double blind, controlled study. Patients are stratified and the groups balanced according to age and sex. To be eligible, patients are determined to be predisposed to rheumatoid arthritis by methods described herein and otherwise known to those of ordinary skill in the art. In addition, patients are required to be living within a reasonable commuting distance of the treatment center. All relevant institutional review boards approve the study design.

### B. Study Design and Sample Size

Blood samples are taken from all sixty patients prior to treatment, for example, once a month for 6 months, and analyzed for several factors including white blood cell count, erythrocyte sedimentation rate, rheumatoid factor and C-reactive protein, using standard test procedures. The erythrocyte sedimentation rate and C-reactive protein are elevated in most inflammatory conditions including rheumatoid arthritis, and Rheumatoid Factor is elevated in most cases of rheumatoid arthritis as well as in some cases of certain other autoimmune diseases.

### C. Detection and Treatment

Apoptotic cells are prepared from an appropriate mammalian cell line, for example, Jurkat cells (ATCC No.TIB-152). The cells are treated with 50 mM sodium butyrate in RPMI medium, at confluency for one day, and then the sodium butyrate medium is changed. To increase the number of apoptotic cells and bodies, the cells can additionally be irradiated with UV-light (*e.g.* 75 mj). Supernatant containing floating cells is removed 24 hours following irradiation.

Apoptotic bodies are quantitated by centrifuging the supernatant (1200 rpm, 5 minutes), aspirating the supernatant, washing the resulting cell pellet with PBS and centrifuging again, as above. The pellet containing the apoptotic cells is re-suspended in PBS. The cells stained for quantitation are re-suspended in 1x binding buffer at a concentration of 1x10⁶ cells/ml. About 100ml of the cells are transferred to a 5 ml tube, and 10ml of fluorescein-conjugated annexin V and 10ml propidium iodide reagent are added. The cells are gently vortexed and the cell mixture is incubated for 15 minutes at 25°C in the dark. Following the incubation, 400ml of 1x binding buffer is added to each tube. The sample is analyzed on a flow cytometer over one hour.

Patients are treated with an injection of suspended apoptotic cells prepared as described above as preferred according to the present invention. Each patient receives, for example, 4 individual treatments over a time span of 16 weeks, where each treatment unit dosage composition comprises, for example, a liquid suspension of cellular material containing from about 10,000 to about 10,000,000 apoptotic cells per kilogram of patient body weight.

Post treatment measurements are conducted once a month for 6 months after the final treatment of each patient. Blood samples are taken and analyzed for white blood cell count, erythrocyte sedimentation rate, rheumatoid factor and C-reactive protein, using standard test procedures.

### D. Statistical Analysis and Results

Demographic factors and randomization strata (sex and age) between treatment groups are compared using an appropriate statistical test, for example, Fisher's exact test. The frequency of decreased white blood cell count, erythrocyte sedimentation rate, rheumatoid factor and C-reactive protein in each of the treatments groups is analyzed by means of a chi-square test for trend, with one degree of freedom. A statistically significant decrease in white blood cell count, erythrocyte sedimentation rate, rheumatoid factor and C-reactive protein is preferably observed in the subjects treated with apoptotic cells upon completion of the study versus untreated subjects.

### Example 6: Efficacy of Administering Apoptotic Cells in a Murine Model for Systemic Lupus Erythematosus

The classical model for SLE-like disease, the MRL/MPJ-Fas^{lpr}, is used to assess the efficacy of using apoptotic cells to prevent SLE. These mice develop SLE-like disease due to mutation in Fas, a receptor that mediates apoptosis and activation of induced cell death of the immune system. Since in SLE patients, as well as in MRL/MpJ-Fas'pr mice, the development of autoantibodies and kidney disease are the most specific pathophysiological parameters, those parameters are valuated in MRL/MPJ-Fas^{lpr} following administration of apoptotic cells.

### A. Apoptosis Protocol

MRL/MpJ-Faslpr and C3H-SnJ mice (normal) are obtained, for example, from Jackson Laboratories, Inc. (Bar Harbor, ME). Thymocytes, and splenoscytes are prepared from 4 to 8 week-old mice using methods known to those of ordinary skill in the art. Apoptotsis of thymocytes or splenocytes is induced by either serum deprivation, 1 uM dexamethasone, or gamma-irradiation (66 rad). The cells were incubated at 370C in 5% C02 for I to 3 hours to allow apoptotic changes to occur. Apoptosis is confirmed by annexin-FITC staining by flow cytometry, DNA fragmentation and propidium iodide staining of fragmented DNA.

### B. Administration of Apoptotic Cells

After incubation, the apoptotic cells are injected into each mouse recipient. The composition comprising sex- and age-matched syngeneic apoptotic cells is injected at about 5x10⁶ cells per mouse and compared to syngeneic, sex- and age-matched mice that are injected with the vehicle (saline). The route of administration is intravenous, via the tail vein, without further manipulation. The injections are performed every week for a total of four to six injections.

Serum samples are obtained immediately prior to immunization and at two-weeks intervals following immunization. The immune response is evaluated by quantifying serum anti-ssDNA and anti-dsDNA by ELISA as known in the art. Sera are diluted 1:100 for the autoantibody screens.

Mice are examined every day for clinical signs of disease and once a month for hematuria or proteinurea. After four months the mice are killed and the kidneys examined histologically and using fluorescent immune staining.

### C. Statistical Analysis and Results

Two groups of age- and sex-matched MRL/IVIpJ-Fas^{lpr} mice are compared. In group 1, 200 ul of saline containing syngeneic apoptotic cells are intravenously injected into each of one of five mice in a weekly interval for five times. In group 2, 200 ul of saline (the vehicle for the first group) are injected into the same number of mice at the same time. IgG anti-ssDNA O.D. levels are consecutively measured in two weeks intervals and are preferably comparable to the level before immunization.

When compared 10 weeks following the beginning of the immunization, mice immunized with vehicle alone preferably have, as expected from mice that developed lupuslike disease, higher IgG anti-ssDNA O.D levels. However, mice injected with syngeneic apoptotic cells preferably have statistically significant reduced levels of autoantibodies.

In order to evaluate the increase in anti-ssDNA, serial bimonthly sera samples are evaluated simultaneously for IgM in mice immunized with saline and in mice immunized with apoptotic cells. The O.D. statistical evaluation preferably shows a statistically significant decrease in mice treated with apoptotic cells versus mice treated with saline. IgG anti-ssDNA levels are preferably statistically significantly decreased following the immunization with syngeneic apoptotic cells for mice immunized with saline versus mice immunized with syngeneic apoptotic thymocytes.

In order to see if autoantibodies even more specific for SLE are decreased, anti-dsDNA is measured in all mice at the age of 6 weeks, before starting to immunize, and at 16-18 weeks of age, upon sacrifice. Anti-dsDNA is preferably significantly reduced in mice immunized with syngeneic apoptotic cells versus mice injected with saline

To further compare if the clinical response follows the serological one, kidney-disease is compared in the two groups. None of the mice have any evidence for proteinuria or hematuria as measured by urine-stick at 6 weeks of age, before the immunization. At 16 weeks, mice immunized with saline preferably have significant elevations in proteinuria and hematuria versus mice injected with apoptotic cells.

At 16 weeks all mice injected with saline alone demonstrate glomerular disease manifested by proteinuria and hematuria. However, mice injected with syngeneic apoptotic cells preferably show marked improvement, more preferably show slight deterioration, and most preferably show no deterioration, consistent with the serological response.

In order to confirm the clinical response, the extent of the disease progression in the kidneys are evaluated by paraffin embedded and immunofluorescent histological studies. The data preferably demonstrates that mice injected with syngeneic apoptotic cells show decreased involvement of disease in the glomeruli, vessels and in the tubuli.

While there has been described what is presently believed to be the preferred embodiments of the present invention, other and further modifications and changes may be made without departing from the spirit of the invention. All further and other modifications and changes are included that come within the scope of the invention as set forth in the claims. The disclosure of all publications cited above is expressly incorporated by reference in their entireties to the same extent as if each were incorporated by reference individually.

### Example 7: Pre-Transplant Administration of an Effective Amount of Apoptotic Cells to Subjects Undergoing a Cardiac Transplant

A randomized, double blind, controlled study is being performed at 12 clinical sites to assess the effect of pre-transplant treatment with apoptotic cells on transplant rejection. In particular, the study seeks to assess the impact of treatment with apoptotic cells in conjunction with center standard conditioning regimen and as compared to center standard conditioning regimen alone, on the incidence of rejection in patients undergoing cardiac transplantation.

### A. Patients

A total of 60 eligible adult recipient of primary cardiac transplants at 12 clinical sites are randomly assigned to receive treatment with apoptotic cells alone (17 patients), standard triple-drug immunosuppressive therapy (cyclosporine, asathioprine, and prednsone; 23 patients) plus treatment with apoptotic cells or standard triple-drug therapy (20 patients). Patients are stratified and the groups balanced according to age and sex. To be eligible, patients are required to be living within a reasonable commuting distance of the transplantation center and to have adequate peripheral venous access. The study design is approved by all relevant institutional review boards.

### B. Study Design and Sample Size

Apoptotic cells are prepared from an appropriate mammalian cell line, for example, Jurkat cells (ATCC No.TIB-152). The cells are treated with 50 mM sodium butyrate in RPMI medium, at confluency for one day, and then the sodium butyrate medium is changed. To increase the number of apoptotic cells and bodies, the cells can additionally be irradiated with UV-light (*e.g.* 75 mj). Supernatant containing floating cells is removed 24 hours following irradiation. Apoptotic bodies are quantitated by centrifuging the supernatant (1200 rpm, 5 minutes), aspirating the supernatant, washing the resulting cell pellet with PBS and centrifuging again, as above. The pellet containing the apoptotic cells is re-suspended in PBS. The cells stained for quantitation are re-suspended in 1x binding buffer at a concentration of 1x10⁶ cells/ml. About 100ml of the cells are transferred to a 5 ml tube, and 10ml of fluorescein-conjugated annexin V and 10ml propidium iodide reagent are added. The cells are gently vortexed and the cell mixture is incubated for 15 minutes at 25°C in the dark. Following the incubation, 400ml of 1x binding buffer is added to each tube. The sample is analyzed on a flow cytometer over one hour.

Patients randomly assigned to receive treatment with apoptotic cells, and treatment with apoptotic cells plus standard triple-drug therapy, are treated with apoptotic cells on two consecutive days, one week prior to transplantation. These patients receive treatment by means preferred in the present invention, where each treatment unit dosage composition comprises, for example, a liquid suspension of cellular material containing from about 10,000 to about 10,000,000 apoptotic cells per kilogram of patient body weight.

After transplantation, monitoring is standardized during the six-month period following transplantation for all the patients in the three groups. Routine laboratory studies and measurements of cyclosporine levels in the blood are performed according to a standardized schedule. Endomyocardial biopsies are obtained according to the following schedule: weekly during the first postoperative month; every two weeks during months 2 and 3; and every four weeks during months 4-6, for a total of 11 biopsies.

The primary end point of the study is the number and frequency of acute rejection episodes as defined histologically in a blinded manner at the central pathology laboratory. Secondary end points include the incidence of clinically treated infections, detection of plasma cytomegalovirus (CMV) DNA by polymerase chain reaction (PCR) assay performed by investigators at a central reference laboratory who are unaware of the patient's treatment assignments, and survival without the need for a second transplantation. The study end point are monitored and the patients followed for six months after transplantation; safety and survival follow-up is continued for an additional six months, during which time management at each center reverts to non-standardized institutional protocols.

Two of the groups receive triple-drug immunosuppressive therapy (cyclosporine, azathioprine, and prednisone). Prophylactic use of monoclonal or polyclonal antibodies is not permitted. All the institutions target the rough levels of cyclosporine in whole blood to achieve the equivalent of 250 to 350 ng per milliliter by fluorescence polarization immunoassay (TDX, Abbott, Abbott Park, Ill). Azathioprine treatment begins with a single preoperative dose of 4 mg/kg of body weight, followed by postoperative administration of 0 to 5 mg/kg per day, adjusted to achieve a white-cell count of 4000 to 8000 per cubic milliliter. Corticosteroids were administered beginning with a 500-to-1000 mg dose of intravenous methylpredisolone preoperatively or intra-operatively. Tapered administration of prednisone is then initiated, beginning at 1 mg per kilogram per day, with the goal of reaching a dose of 0.1 mg/kg per day by the fifth month after transplantation.

All the patients receive standardized prophylaxis against infection. For protection against candida infection, patients receive oral mycostatin for the first three months after transplantation. For prevention of *Pneumocystis carinii* pneumonia, patients receive 160 mg of trimethoprim in combination with 800 mg of sulfamethoxazole three times per week, starting in the second week after surgery and continuing throughout the study period. For prophylaxis against CMV infection, all the recipients of CMV-positive organs, whether the recipient was CMV-positive or CMV-negative, receive 10 mg of intravenous ganciclovir per kilogram per day for the first 10 to 14 days after transplantation.

### C. Detection and Treatment of Rejection

Over the six-month period, 11 transvenous endomyocardial biopsies are performed according to a standardized schedule, as outlined above. All biopsy specimens are evaluated by a single pathologist in a blinded manner at the central pathology laboratory according to the standards published by the International Society for Heart and Lung Transplantation. Specific standardized treatment regimens based on the severity of rejection (evaluated in a blinded manner within 24 hours by a pathologist at the local institution) are used. All the patients with grade 3A, 3B, or 4 biopsy specimens are treated. Patients with a grade 2 biopsy specimen in month 1 or two successive grade 2 specimens in months 2 through 6 are treated. Patients with three successive grade 1B specimens are treated. A prolonged episode of rejection (as ascertained by two or more consecutive abnormal specimens) is counted as a single rejection episode if the biopsy grade remained higher than 0 or 1A.

### D. PCR Assay for CMV DNA

For all the patients, serial blood samples (taken every two weeks from weeks 2 to 12 and monthly from months 4 through 6, for a total of nine samples) are analyzed by PCR amplification of plasma CMV DNA. The qualitative PCR procedure used has been described by Wolf and Spector. Plasma samples are maintained at -20 degrees Celsius until processed in a blinded manner at the central laboratory. Ten microliters of plasma diluted 1:10 is passed through three freeze-thaw cycles, dissolved in PCR buffer, and incubated with proteinase K at 60 degrees Celsius for one hour. Samples are then heated at 95 degrees Celsius for 10 minutes, centrifuged at 12000 x g for five minutes, and directly amplified by PCR. For amplification, two sets of primer pairs are constructed for the EcoRI fragment D region of human CMV strain AD169. The template is combined with the appropriate primer pair (50 pmol of each), deoxyribonucleoside triphospates (200 micromolar each; Pharmacia SKB, Piscataway, N.J), and Taq polymerase (2.5 U; Perkin-Elmer Cetus Instruments, Norwalk, Conn.) in a total volume of 100 microliters of PCR buffer and then amplified by 35 cycles of denaturation, primer annealing, and chain extension. After amplification, 10 microliters of the product was denatured at 94 degrees Celsius and immediately hybridized at 55 degrees Celsius with a P32 end-labeled probe. The hybrid heteroduplexes are then resolved by electrophoresis on 6% polyacrylamide gels, and autoradiographs were obtained.

The sensitivity of the procedure is considered to be 6000 replicate copies of the viral DNA. All specimens are tested at least twice in separate PCR procedures, and buffer controls and control samples are run with each reaction. All the positive and negative controls are required to demonstrate the appropriate signal before any result is considered for analysis

### E. Statistical Analysis and Results

Demographic factors and randomization strata (sex and age) between treatment groups are compared using Fisher's exact test. The frequency of rejection episodes in each of the three treatments groups is analyzed by means of a clu-square test for trend, with one degree of freedom. A collapsed version of this analysis compares the number of patients who have one rejection episode or none with the number who had two or more, and other dichotomous variables, with the use of Fisher's exact test. Survival estimates in the form of several functions are generated by the Kaplan-Meier product limit method. The survival of the patients is analyzed with the log-rank test for comparison of the survival curves. Comparison of the infection rates in the three groups is performed with either a Fisher's exact test or a chi-square test for trend, with one degree of freedom. For the detection of CMV DNA by PCR, the percentage of time a patient has a positive test result is calculated. A Wilcoxon rank-sum test is used to compare treatment groups. All P values are two-tailed.

A statistically significant decrease in the rate of incidence of transplant rejection is preferably observed in the treated subjects upon completion of the study over the known incidence of rejection in non-treated subjects. Moreover, the incidence of rejection in subjects treated with apoptotic cells is preferably observed to be statistically equal to or less than subjects treated with apoptotic cells and the center standard conditioning regimen. Further, the incidence of rejection in subjects treated with apoptotic cells and standard conditioning regimen is preferably observed to be statistically equal or less than the standard conditioning regimen treated subjects alone. The study may also look at the progression of organ rejection, or the prevention or delay of organ rejection, or the reduction of the severity of rejection. The differences between incidence of rejection or the progression of the rejection, or the prevention or delay of organ or tissue rejection, or the reduction of the severity of rejection, between the treated subjects and controls are preferably statistically significant and or observable by clinical or other tests or evaluations. Therefore, the study confirms that treatment of a subject with apoptotic cells prior to organ transplantation is effective prophylacticly, for example, in preventing, reducing the severity of, or delaying or preventing the onset of organ rejection.

## Claims

1. Use of an effective amount of apoptotic cells in the manufacture of a medicament for the treatment or prevention of an atopic disease.

2. The use of claim 1, wherein said atopic disease is detected in a subject by identification of a disease marker for said atopic disease.

3. The use of claim 2, wherein said disease marker is selected from the group consisting of genetic marker, serological marker, immunological marker, gene expression profile, protein expression profile, and polymorphism.

4. The use of claim 1, wherein said predisposition is determined by the identification of a disease marker for said atopic disease in said subject.

5. The use of claim 4, wherein said disease marker is selected from the group consisting of genetic marker, serological marker, immunological marker, gene expression profile, protein expression profile, and polymorphism.

6. The use of claim 1, wherein said medicament is to be administered according to a dosage schedule selected from the group consisting of weekly, monthly, twice a month, three times a month, every other month, every three months, every six months, every nine months, and yearly.

7. The use of claim 1, wherein said apoptotic cells are in a liquid suspension.

8. The use of claim 7, wherein said apoptotic cells comprise from about 10% to about 90% of the total number of cells in said liquid suspension.

9. The use of claim 8, wherein said apoptotic cells comprise from about 30% to about 70% of the total number of cells in said liquid suspension.

10. The use of claim 1, wherein said apoptotic cells comprise apoptotic cells derived from a cultured cell line.

11. The use of claim 10, wherein said apoptotic cells comprise cultured cell line cells subjected to extracorporeal treatment.

12. The use of claim 11, wherein said extracorporeal treatment is selected from the group consisting of antibodies, chemotherapeutic agents, radiation, extracorporeal photopheresis, ultrasound, proteins, and oxidizing agents.

13. The use of claim 1, wherein said apoptotic cells comprise blood cells compatible with those of the subject to which the medicament is to be administered.

14. The use of claim 13, wherein said apoptotic cells comprise blood cells subjected to extracorporeal treatment.

15. The use of claim 14, wherein said extracorporeal treatment is selected from the group consisting of antibodies, chemotherapeutic agents, radiation, extracorporeal photopheresis, ultrasound, proteins, and oxidizing agents.

16. The use of claim 13, wherein said blood cells comprise the subject's blood cells.

17. The use of claim 13, wherein said blood cells comprise white blood cells that are compatible with the subject to which the medicament is to be administered.

18. The use of claim 17, wherein said blood cells comprise the subject's blood cells.

19. The use of claim 17, wherein said blood cells comprise T-cells.

20. The use of claim 19, wherein said blood cells comprise the subject's blood cells.

21. The use of claim 1, wherein said effective amount of apoptotic cells comprises a dosage of apoptotic cells comprising from about 10,000 to about 10,000,000 apoptotic cells per kilogram body weight of the subject to which the medicament is to be administered.

22. The use of claim 21, wherein said dosage contains from about 500,000 to about 5,000,000 apoptotic cells per kilogram body weight of said subject.

23. The use of claim 22, wherein said dosage contains from about 1,500,000 to about 4,000,000 apoptotic cells per kilogram body weight of said subject.

24. Use of blood in the manufacture of a medicament for treatment or prevention of an atopic disease, or an autoimmune reaction manifested by a T cell response, wherein said blood comprises blood from a subject predisposed to said disease having a photoactivatable compound and wherein said blood having a photoactivatable compound is treated with light of a wavelength that activates said photoactivatable compound.

25. The use of claim 24, wherein said medicament is to be administered after the identification of a disease marker for said atopic disease, or autoimmune reaction in said subject.

26. The use of claim 25, wherein said disease marker is selected from the group consisting of genetic marker, serological marker, immunological marker, gene expression profile, protein expression profile, and polymorphism.

27. The use of claim 24, wherein said medicament is to be administered to said subject according to a schedule selected from the group consisting of weekly, monthly, twice a month, three times a month, every other month, every three months, every six months, every nine months, and yearly.

28. The use of claim 24, wherein said photoactivatable compound comprises a psoralen or psoralen derivative.

29. The use of claim 28, wherein said psoralen or psoralen derivative comprises 8-methoxypsoralen.

30. The use of claim 28, wherein said psoralen or psoralen derivative comprises amotosalen.

31. The use of claim 24, wherein said atopic disease is selected from the group consisting of atopic dermatitis, extrinsic bronchial asthma, urticaria, allergic rhinitis, and allergic enterogastritis.
